# FASCICULE DE BREVET EUROPEEN

(11) **EP 3 541 920 B1**
(45) Date de publication et mention de la délivrance du brevet: **01.07.2020**
(21) Numéro de dépôt: 17798233.7
(22) Date de dépôt: 16.11.2017
(51) Int. Cl.: C12M 1/107, C12M 3/00, C12M 1/00, C12M 1/02

(54) **SYSTEME ET PROCEDE DE PRODUCTION DE BIOGAZ A PARTIR DE BIOMASSE SOLIDE**
SYSTEM UND VERFAHREN ZUR HERSTELLUNG VON BIOGAS AUS FESTER BIOMASSE
SYSTEM AND PROCESS FOR PRODUCING BIOGAS FROM SOLID BIOMASS

(30) Priorité: 17.11.2016 FR 1661167
(43) Date de publication de la demande: 25.09.2019
(73) Titulaire: YANNCO, 92800 Puteaux (FR)
(72) Inventeur: MERCIER, Yann, 92800 Puteaux (FR)
(74) Mandataire: Casalonga
(86) Numéro de dépôt international: PCT/EP2017/079496
(87) Numéro de publication internationale: WO 2018/091600

(56) Documents cités:
- EP-A2- 0 023 176
- WO-A1-2015/033068
- BE-A- 427 335
- FR-A- 1 006 957
- FR-A1- 2 500 256
- US-A- 5 958 756

## Description

La présente invention concerne un système de production de biogaz comportant au moins une unité centrale de stockage de digestat liquide et de digestion complémentaire, au moins une unité centrale de stockage de biogaz, une pluralité de digesteurs anaérobies, un réseau d'alimentation de digestat liquide, un réseau d'évacuation de digestat liquide et un réseau d'évacuation de biogaz ; l'un au moins desdits digesteurs anaérobies étant amovible et apte à être connecté et déconnecté desdits réseaux.

L'invention concerne également un procédé de production de biogaz comportant successivement une étape de chargement en biomasse solide d'au moins un digesteur anaérobie dans un lieu différent du lieu d'implantation dudit système, une étape de transport dudit digesteur jusqu'au lieu dudit système et des étapes successives, telles que définies ci-après, visant à produire du biogaz.

Enfin, l'invention porte plus particulièrement sur un digesteur anaérobie amovible comportant un ou plusieurs moyens configurés pour permettre sa connexion et sa déconnexion aux réseaux précédemment définis.

Au sens de la présente invention, on entend par biomasse solide, des matières organiques d'origine végétales, animales, bactériologiques ou fongiques, contenant un taux de matière sèche supérieur à 12%, de préférence supérieur à 15%, qui permettent la production de biogaz.

La biomasse solide peut être fibreuse, notamment pailleuse, et/ou hétérogène, et/ou contenir des éléments indésirables, notamment des cailloux, du gravier, du sable, des barbelés, des ficelles, des pièces métalliques provenant d'outils agricoles ou encore des sacs plastiques, des éléments d'emballage et de conditionnement, ou encore des déchets urbains, éventuellement mal triés.

La méthanisation (encore appelée digestion anaérobie) est un procédé biologique naturel qui met en œuvre la dégradation des matières organiques par plusieurs types de micro-organismes, en particulier des bactéries, dans des conditions contrôlées et ceci en l'absence d'oxygène.

Un tel procédé conduit, d'une part, à la production de biogaz, qui correspond à un mélange gazeux composé majoritairement de méthane et de dioxyde de carbone, et, d'autre part, à la production d'un produit humide riche en matières organiques partiellement stabilisées appelé digestat, qui peut être ensuite utilisé comme amendement organique.

Le biogaz présente l'avantage d'être un gaz convertible en énergie renouvelable et peut par exemple servir dans la production d'électricité et/ou de chaleur ou dans la production de carburant. Le biogaz peut également être injecté dans un réseau de gaz naturel après épuration, c'est-à-dire après que le méthane présent dans le biogaz a été séparé du CO₂ et des autres gaz présents dans le biogaz

Le digestat peut subir un traitement de séparation de phases liquide/solide dont la fraction solide (appelé digestat solide) est plus riche en matières organiques et en éléments phosphatés et la fraction liquide (appelé digestat liquide) est plus riche en azote ammoniacal et en potassium. Les digestats liquides et solides peuvent être stockés, et/ou traités et/ou épandus séparément.

Ainsi le procédé biologique de méthanisation permet de valoriser des matières organiques, en produisant une énergie renouvelable, et de diminuer les émissions de gaz à effet de serre, en captant le méthane issu de la dégradation de ces matières, et en substituant l'emploi d'énergies fossiles et/ou d'engrais chimiques.

Les matières organiques, susceptibles de produire du biogaz par le biais d'une méthanisation, peuvent être des déchets provenant par exemple du secteur agricole et/ou agro-industriel et/ou être d'origine urbaine. En particulier, le fumier, provenant de l'exploitation agricole d'élevages, est une matière organique, issue des déjections animales mélangées à la paille des litières, qui constitue une source majeure et particulièrement intéressante dans la production de biogaz. A cet égard, la méthanisation dans le secteur agricole à partir de matières organiques, en particulier de pailles et/ou de fumier, représente une activité qui se développe de plus en plus à l'heure actuelle.

La méthanisation est couramment mise en œuvre à l'intérieur d'un réacteur appelé digesteur correspondant le plus souvent à une cuve, ayant une forme le plus souvent essentiellement cylindrique, mais parfois parallélépipédique, verticale ou horizontale, qui est destinée à contenir les matières organiques à traiter, en particulier du fumier et/ou des pailles et/ou des déchets d'origine industrielle et/ou d'origine urbaine, afin de produire du biogaz et du digestat.

Les différents procédés de méthanisation peuvent être répartis en deux grandes familles en fonction de la nature des déchets à dégrader, à savoir les procédés de méthanisation en voie liquide et les procédés de méthanisation en voie sèche.

En premier lieu, les procédés de méthanisation en voie liquide sont généralement mis en œuvre pour traiter des déchets dont le mélange a un taux moyen de matière sèche inférieur à 15%, correspondant par exemple à des mélanges de boues liquides ou de graisses ou des mélanges principalement composés de lisier. De tels procédés peuvent être aussi utilisés pour traiter des matières organiques solides qui ont été mélangées avec de l'eau ou du digestat liquide.

Les procédés de méthanisation en voie liquide consistent notamment à acheminer de manière généralement continue les matières organiques à traiter, le plus souvent au moyen de systèmes de pompage ou de vis sans fin à l'intérieur d'un digesteur qui est généralement hermétiquement fermé par l'intermédiaire d'un toit ou d'une membrane souple. Les matières organiques à traiter sont continuellement brassées à l'intérieur du digesteur, souvent par un ou plusieurs agitateurs, pouvant être à hélices ou à pâles, ou parfois par des injections de gaz, notamment de biogaz, afin d'éviter les phénomènes de décantation de la biomasse au fond du digesteur et/ou les phénomènes de flottation et de croûtage de la biomasse en surface du liquide dans le digesteur, et de favoriser la formation de biogaz par le contact entre la biomasse et les micro-organismes. Le digesteur peut éventuellement être chauffé dans des températures comprises entre 20 et 60°C, en particulier dans des températures comprises entre 35 et 55°C. Les matières organiques séjournent en moyenne plusieurs semaines dans le digesteur, typiquement pendant une période pouvant aller de 20 à 80 jours.

Le biogaz, produit au cours de cette réaction de méthanisation, est généralement stocké à pression atmosphérique dans une membrane souple étanche fixée au-dessus du digesteur. La membrane se présente alors sous la forme d'un dôme contenant un ciel gazeux au-dessus de la paroi verticale du digesteur. Plus rarement, le biogaz peut être stocké dans un gazomètre, qui est souvent une poche de stockage souple située à côté du digesteur.

Les procédés de méthanisation en voie liquide présentent notamment l'inconvénient que les biomasses à dégrader peuvent être difficiles à manipuler et à préparer pour leur acheminement vers le digesteur et nécessitent une dépense d'énergie importante pour être brassés de manière continue à l'intérieur du digesteur.

Dans les procédés de méthanisation en voie liquide qui incluent l'introduction de matières organiques solides, les matières organiques solides sont souvent broyées avant d'être incorporées dans la cuve de digestion afin de faciliter l'acheminement des matières organiques au sein du digesteur ainsi que leur brassage. Parfois, les matières organiques solides font l'objet d'un processus de tri préalable afin d'en retirer le plus possible d'objets indésirables.

Ces types de procédés de méthanisation en voie liquide continue présentent souvent des difficultés au niveau de l'alimentation de la biomasse car il est souvent nécessaire de traiter préalablement les biomasses avant leur introduction dans le digesteur, notamment en broyant la partie solide de la biomasse et en extrayant les matières indésirables. De plus, les systèmes de brassage au sein du digesteur peuvent être endommagés du fait de la nature des matières organiques ou des matières indésirables.

En second lieu, les procédés de méthanisation, dit par voie sèche, sont mis en œuvre pour traiter des déchets solides dont le mélange a un taux moyen de matière sèche généralement supérieur à 15%, notamment allant de 15% à 40%, correspondant par exemple à du fumier, de la paille, des déchets industriels ou urbains, ou des biomasses hétérogènes.

Un premier type de procédé de méthanisation par voie sèche consiste à acheminer en continu, notamment au moyen d'une trémie et d'un système de vis sans fin, ou plus rarement d'un système de pompage puissant, les matières organiques solides à traiter à l'intérieur d'un digesteur, le plus souvent disposé de manière horizontale. Les matières organiques solides à traiter sont brassées, le plus souvent lentement, à l'intérieur du digesteur qui peut être également chauffé dans des températures comprises entre 20 et 60°C, en particulier dans des températures comprises entre 35 et 55°C.

De plus, les matières organiques solides sont souvent broyées avant d'être incorporées dans la cuve de digestion afin de faciliter l'acheminement des matières organiques au sein du digesteur ainsi que leur brassage. Parfois les matières organiques solides font aussi l'objet d'un processus de tri préalable afin d'en retirer le plus possible d'objets indésirables.

Ce type de procédé de méthanisation en voie sèche continue présente souvent des difficultés au niveau de l'alimentation de la biomasse car il est souvent nécessaire de traiter préalablement les déchets avant leur introduction dans le digesteur, notamment en les broyant et en extrayant les matières indésirables. De plus, le système de brassage au sein du digesteur peut être endommagé du fait de la nature des matières organiques ou des matières indésirables.

Un second type de procédé de méthanisation en voie sèche est un procédé discontinu, ou en bâchées. Il consiste à acheminer, souvent au moyen d'engins mécaniques de chargement/déchargement, par exemple de type chargeur à godets, les matières organiques solides à l'intérieur de cellules de méthanisation qui sont fermées une fois remplies. Ces cellules sont généralement des sortes de garages équipés de portes que l'on referme une fois le chargement réalisé. Ce type de procédé est souvent dénommé procédé en garage. Les matières organiques sont ensuite aspergées ou douchées avec du digestat liquide, chargé en bactéries, afin de réaliser une percolation conduisant à la production de biogaz. Une fois la réaction terminée, les portes de chaque cellule sont ouvertes afin de récupérer le digestat solide, généralement au moyen des mêmes engins mécaniques que pour le chargement. Un tel procédé met généralement en œuvre plusieurs cellules de méthanisation ou digesteurs, alimentées en bâchées l'une après l'autre. Dans ce type de procédé, les cellules sont généralement destinées à fonctionner majoritairement en même temps même si elles ont été alimentées à des moments différents. Le biogaz, produit au cours de la réaction de méthanisation, est le plus souvent stocké séparément dans un gazomètre, souvent une poche de stockage souple située à côté des digesteurs.

Ce type de procédé en garage pose notamment le problème de la gestion du biogaz. Au début de la réaction de méthanisation, le biogaz produit dans chaque cellule est souvent mis en mélange avec l'air présent dans le garage avant fermeture de la porte du garage et après le chargement de la biomasse à traiter, ce qui a pour conséquence que, pendant le temps nécessaire au remplacement de l'air par du biogaz, un mélange air-biogaz est produit par la cellule. De même, en fin de réaction, le biogaz présent dans le garage doit être remplacé par de l'air avant ouverture de la porte du garage, ce qui a pour conséquence que, pendant le temps nécessaire au remplacement du biogaz par de l'air, un mélange air-biogaz est également produit par la cellule. Ces mélanges d'air et de biogaz produits en début et en fin de réaction de méthanisation ont pour conséquence que le biogaz produit par les procédés de méthanisation en garage est souvent mélangé avec de l'air.

Cette présence d'air dans le biogaz produit par les procédés en garage rend très difficile la valorisation du biogaz par injection du bio-méthane dans un réseau de gaz naturel. En effet, pour pouvoir être injecté dans un réseau de gaz naturel, le biogaz doit être épuré, c'est-à-dire que le méthane présent dans le biogaz doit être séparé du CO₂ et des autres gaz présents dans le biogaz. Or lorsque le biogaz contient de l'air, il contient en particulier de l'azote gazeux (N₂) qui peut s'avérer techniquement difficile à séparer du méthane.

De plus, dans un garage, la réaction de méthanisation n'est pas convenablement optimisée car la biomasse solide, introduite dans les cellules, n'est pas mélangée intimement avec le digestat liquide et souvent des chemins préférentiels de circulation du digestat liquide dans la biomasse solide se forment, empêchant une bonne diffusion du digestat liquide au sein la biomasse solide à traiter, et donc empêchant un contact intime entre les bactéries et la biomasse, réduisant sensiblement le taux de dégradation de la biomasse solide.

Plus généralement, un autre inconvénient majeur liés aux procédés de méthanisation en voie liquide et en voie sèche est l'émission de mauvaises odeurs pouvant survenir sur le lieu de production de biogaz notamment lors du stockage et du traitement préalable des biomasses solides, ainsi que lors des opérations de chargement et de déchargement de biomasse dans les unités de méthanisation.

Par exemple, les fumiers, les lisiers, et généralement tous les déchets issus des déjections animales, mais aussi les déchets organiques issus des activités industrielles, comme par exemple les déchets d'abattoirs, ainsi que les déchets organiques issus des municipalités ou de la grande distribution sont le plus souvent des déchets susceptibles d'émettre des odeurs fortes et désagréables au cours de leur stockage et des prétraitements dont ils font l'objet avant leur introduction dans l'unité de méthanisation.

Dans une moindre mesure, les opérations de traitement du digestat, c'est-à-dire après digestion anaérobie des matières organiques, peuvent également conduire à l'émission d'odeurs désagréables, en particulier au cours des opérations de séparation de phase, notamment en raison de l'évaporation de l'ammoniac contenu dans le digestat.

De fait, les émissions d'odeurs sont un enjeu majeur dans l'acceptation par le voisinage des projets de méthanisation, qu'ils soient réalisés par l'intermédiaire d'un procédé en voie sèche ou d'un procédé en voie liquide. Il s'agit le plus souvent de l'enjeu principal. En effet, les moyens mis en œuvre pour minimiser et contrôler les odeurs générées au cours des différentes étapes de méthanisation sur un territoire sont très onéreux en investissement et en exploitation : bâtiments de confinement des installations de prétraitements, systèmes d'aspiration de l'air souillé dans de grands volumes, systèmes d'épuration d'air par voie physico-chimiques, systèmes d'épuration d'air par voie biologiques, audits réalisés par des organismes agréés, réunions périodiques avec des associations de riverains afin d'évaluer et contrôler le niveau des mauvaises odeurs, etc.

Ainsi les matières organiques à traiter soulèvent souvent des difficultés au cours des différents procédés de méthanisation car, d'une part, elles sont génératrices d'odeurs lors des opérations de stockage, de chargement et de déchargement, et d'autre part, ces biomasses présentent généralement l'inconvénient de comporter de nombreux éléments indésirables et/ou d'être difficiles à manipuler et/ou à broyer, ce qui rend difficiles les opérations de préparation de la biomasse (tri, broyage, pompage, transferts, etc.).

En effet, la paille, ainsi que le fumier, ou encore les biomasses hétérogènes, peuvent comporter des cailloux, du gravier, du sable, des barbelés, des ficelles, des pièces métalliques provenant d'outils agricoles ou encore des sacs plastiques, des éléments d'emballage ou de conditionnement. De tels éléments sont souvent difficiles à séparer de la biomasse à traiter et peuvent donc endommager les installations composant l'unité de méthanisation. A titre d'exemple, ces éléments indésirables peuvent endommager les pompes, les vis sans fin, les agitateurs et les broyeurs, conduisant ainsi au remplacement partiel ou total des pièces d'usure et/ou des pièces principales selon des fréquences élevées, engendrant des coûts de maintenance élevés.

De plus, la paille et les produits pailleux, tels que les fumiers, la menu-paille, les cannes de maïs, etc., sont délicats à manipuler car les produits pailleux sont abrasifs, ce qui peut abîmer, dégrader ou user les équipements employés au cours des différentes étapes des procédés de méthanisation (étapes de broyage, de triage, de pompage, de pressage et/ou de brassage).

De tels dégâts sont donc susceptibles d'entraîner une diminution de la production de biogaz, voire un arrêt de celle-ci, et de générer des manques à gagner et/ou une augmentation des coûts de maintenance et d'exploitation de l'installation.

En outre, une fois introduits, la paille et autres produits pailleux sont souvent difficiles à brasser dans le digesteur, car ils ont tendance à flotter et/ou à décanter et/ou à s'emmêler autour des agitateurs, ce qui engendre des dépenses d'énergie importantes, notamment des consommations électriques élevées. De telles dépenses énergétiques impactent négativement sur la rentabilité des installations.

Ainsi le fumier, la paille et les produits pailleux sont des matières organiques qui peuvent s'avérer difficiles à valoriser en vue d'une méthanisation.

Afin de remédier à certains de ces inconvénients, la demande de brevet FR 2 990 951 décrit notamment un procédé de préparation d'un substrat de méthanisation à partir de biomasse fibreuse solide, telle que le fumier et la paille, comprenant une étape d'immersion de la biomasse dans un liquide, en particulier de l'eau ou du digestat liquide, une étape d'hydrolyse et une étape de séparation de la matière sèche en suspension du liquide par pressage de manière à préparer un substrat de méthanisation. En particulier, ce procédé comprend une étape de broyage de la biomasse avant ou après immersion, par exemple mise en œuvre à l'aide d'une pompe broyeuse. Ce procédé a pour de but de retirer les éléments indésirables de la biomasse fibreuse solide par décantation et, par conséquent, d'optimiser la préparation du substrat de méthanisation avant de réaliser son acheminement dans le digesteur. Un système de production de biogaz avec une pluralité de digesteurs est décrit dans le document US 5958756 A.

Cependant, la biomasse fibreuse solide reste encore trop souvent difficile à manipuler au cours de ce procédé. En effet, la paille s'avère compliquée à compresser après immersion, ce qui peut engendrer des problèmes de serrage, de bouchage et de blocage au niveau des pompes et des presses et/ou des moyens de séparation.

Un tel procédé présente également l'inconvénient d'engendrer des dépenses d'énergie importantes afin de maintenir correctement la paille en suspension dans le liquide au cours de l'étape de dilution. En effet, on observe que la paille a tendance à s'emmêler, à s'agglomérer, à flotter et à ne pas se mélanger aisément dans le liquide.

De plus, ce procédé ne résout pas convenablement le problème survenant lors du brassage de la biomasse dans le digesteur. En effet, la paille reste encore trop difficile à brasser dans le digesteur car cette dernière flotte en surface et s'emmêle, ce qui conduit à un phénomène d'accumulation et de croûtage qui empêche notamment la bonne circulation du biogaz au sein de la cuve, ce qui complique sa récupération, et qui peut aboutir à la prise en masse complète de la biomasse solide présente dans le digesteur et la paralysie de celui-ci.

Ainsi les différentes unités de méthanisation mises en œuvre à l'heure actuelle présentent un certain nombre d'inconvénients quand elles doivent traiter en totalité ou en partie des biomasses solides, et en particulier des biomasses solides hétérogènes.

Au vu de ce qui précède, l'invention a notamment pour but de valoriser plus efficacement la biomasse solide par méthanisation tout en minimisant les dépenses d'énergie, en réduisant les dépenses de personnel d'exploitation, en rendant non nécessaire l'achat et l'installation de certains équipements de préparation et d'agitation de la biomasse solide, en diminuant les risques d'endommagement des installations composant l'unité de méthanisation et en diminuant les coûts de maintenance et de préparation de la biomasse solide.

L'invention a aussi pour but de valoriser la biomasse par méthanisation en minimisant, voire en supprimant totalement la production de mauvaises odeurs, notamment sur le lieu de production du biogaz.

La présente invention a donc notamment pour objet un système de production de biogaz comprenant :
- au moins une unité centrale de stockage de digestat liquide et de digestion complémentaire, constituée d'un ou plusieurs ouvrages, apte à contenir principalement du digestat liquide,
- au moins une unité centrale de stockage de biogaz, constitué d'un ou plusieurs ouvrages, apte à contenir principalement du biogaz,
- une pluralité de digesteurs anaérobies dont certains sont des digesteurs de biomasse solide, chacun desdits digesteurs de biomasse solide comprenant :
   - une cuve apte à contenir de la biomasse solide, comportant au moins une zone d'admission de la biomasse solide, située sur la partie supérieure de la cuve, sur laquelle un toit est apte à s'ouvrir et à se refermer, et comportant en outre au moins une zone d'évacuation de la biomasse solide digérée résiduelle, certaines desdites zones d'admission et d'évacuation de biomasse solide pouvant être communes, et
   - au moins une ouverture d'alimentation en digestat liquide, située sur la partie inférieure du digesteur anaérobie, reliée à au moins un circuit d'alimentation de digestat liquide, apte à permettre l'introduction de digestat liquide dans ledit digesteur de biomasse solide, et
   - au moins une ouverture d'évacuation de digestat liquide, située sur la partie supérieure du digesteur anaérobie, reliée à au moins un circuit d'évacuation de digestat liquide, apte à permettre l'évacuation de digestat liquide dudit digesteur de biomasse solide,
   - au moins une ouverture d'évacuation de biogaz, située sur la partie supérieure du digesteur anaérobie, reliée à au moins un circuit d'évacuation de biogaz, apte à permettre l'évacuation de biogaz depuis ledit digesteur de biomasse solide, et
   - au moins une ouverture de vidange de digestat liquide, située sur la partie inférieure du digesteur anaérobie, apte à permettre la vidange de digestat liquide dudit digesteur de biomasse solide, et
   - au moins une ouverture d'introduction et/ou d'évacuation d'air, située sur la partie supérieure du digesteur anaérobie, apte à permettre l'introduction et/ou l'évacuation d'air dans ledit digesteur de biomasse solide,
      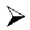 certaines ouvertures parmi lesdites ouvertures pouvant être communes, à l'exception des ouvertures d'alimentation et d'évacuation de digestat liquide qui sont distinctes, et
- au moins un réseau d'alimentation de digestat liquide, incluant une pluralité de circuits d'alimentation de digestat liquide, permettant de relier certaines ouvertures d'alimentation de digestat liquide de certains digesteurs de biomasse solide à l'unité centrale de stockage de digestat liquide et de digestion complémentaire, apte à permettre l'alimentation de digestat liquide, par voie directe ou indirecte, dans lesdits digesteurs depuis l'unité centrale de stockage de digestat liquide et de digestion complémentaire, et
- au moins un réseau d'évacuation de digestat liquide, incluant une pluralité de circuits d'évacuation de digestat liquide, permettant de relier certaines ouvertures d'évacuation de digestat liquide de certains digesteurs de biomasse solide à l'unité centrale de stockage de digestat liquide et de digestion complémentaire, apte à permettre l'évacuation de digestat liquide, par voie directe ou indirecte, depuis lesdits digesteurs vers l'unité centrale de stockage de digestat liquide et de digestion complémentaire, et
- au moins un réseau d'évacuation de biogaz, incluant une pluralité de circuits d'évacuation de biogaz, permettant de relier certaines ouvertures d'évacuation de biogaz de certains digesteurs de biomasse solide à l'unité centrale de stockage de biogaz, apte à permettre l'évacuation de biogaz, par voie directe ou indirecte, depuis lesdits digesteurs vers l'unité centrale de stockage de biogaz,
   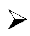 certaines parties desdits réseaux pouvant être communes, et
- certains digesteurs parmi desdits digesteurs de biomasse solide sont des digesteurs amovibles et comportent des moyens configurés pour permettre, d'une part, la connexion de chacun desdits digesteur amovibles à au moins un circuit d'alimentation de digestat liquide et à au moins un circuit d'évacuation de digestat liquide et à au moins un circuit d'évacuation de biogaz et, d'autre part, la déconnection de chacun desdits digesteurs desdits circuits, et
- au moins un moyen de circulation de digestat liquide, de préférence une pompe, située sur le réseau d'alimentation de digestat liquide et/ou sur le réseau d'évacuation de digestat liquide, apte à permettre la circulation du digestat liquide à travers certains digesteurs de biomasse solide, et
- au moins un dispositif de chauffage de digestat liquide.

Le système de production de biogaz selon l'invention présente les avantages, d'une part, de valoriser plus efficacement la biomasse solide tout en réduisant les investissements en divers équipements, les dépenses énergétiques, de personnel et de maintenance des équipements, l'usure des matériels et les risques d'endommagement, et, d'autre part, de minimiser la production de mauvaises odeurs, notamment sur le lieu d'implantation du système de production du biogaz.

Le système de production de biogaz comprend une pluralité de digesteurs anaérobie de biomasse solide.

Par digesteur anaérobie de biomasse solide, on entend un digesteur apte à contenir de la biomasse solide et dans lequel une réaction de méthanisation est apte à se produire dans des conditions anaérobies.

De préférence, le ou les digesteurs anaérobies de biomasse solide sont conçus pour contenir de la biomasse solide.

Parmi les digesteurs anaérobies de biomasse solide, l'un au moins desdits digesteurs est amovible.

### Digesteurs anaérobie amovible

Par digesteur anaérobie amovible, on entend, au sens de la présente invention, un digesteur apte à être, d'une part, connecté à au moins un circuit d'alimentation de digestat liquide, à au moins un circuit d'évacuation de digestat liquide et à au moins un circuit d'évacuation de biogaz et, d'autre part, à être déconnecté desdits circuits.

De plus, un digesteur anaérobie amovible correspond à un digesteur apte à être transporté, notamment par camion ou par tracteur, dans des lieux différents du lieu d'installation du système de production de biogaz, notamment pour être chargé, à travers la zone d'admission située au niveau de sa partie supérieure, en biomasse solide, et notamment être déchargé, une fois la méthanisation terminée, à travers la zone d'évacuation de la biomasse solide digérée résiduelle.

En d'autres termes, au sens de la présente invention, un digesteur amovible correspond à un digesteur de méthanisation qui peut être chargé en biomasse solide dans un lieu distinct du lieu de production de biogaz, transporté vers le lieu de production de biogaz c'est-à-dire le lieu d'implantation du système de production selon l'invention, et déchargé en biomasse solide résiduelle, une fois la méthanisation terminée, dans un lieu différent du lieu de production de biogaz.

Ainsi la pluralité de digesteurs anaérobies tels que définis ci-avant comprend au moins un digesteur amovible apte à être connecté à au moins un circuit d'alimentation de digestat liquide, à au moins un circuit d'évacuation de digestat liquide et à au moins un circuit d'évacuation de biogaz et, d'autre part, à être déconnecté de chacun desdits circuits, pour être transporté dans un lieu distinct du lieu de production du biogaz afin d'être chargé en biomasse solide et/ou déchargé en biomasse solide digérée résiduelle.

De cette façon, les mauvaises odeurs susceptibles de survenir sur le site d'implantation du système de production de biogaz sont efficacement minimisées voire totalement supprimées dans la mesure où le digesteur anaérobie amovible ne nécessite à aucun moment d'être ouvert sur le lieu de production du biogaz.

Par ailleurs, les coûts des moyens mis en œuvre pour minimiser et/ou contrôler les odeurs générées sur le lieu d'implantation du système de production de biogaz peuvent être minimisés voire totalement supprimés.

### Digesteur anaérobie immobile ou inamovible

De même, la pluralité de digesteurs anaérobies peut éventuellement comprendre au moins un digesteur anaérobie immobile ou inamovible.

Par digesteur anaérobie immobile ou inamovible, on entend un digesteur qui ne peut pas être successivement connecté à un circuit d'alimentation de digestat liquide et à un circuit d'évacuation de digestat liquide et/ou à un circuit d'évacuation de biogaz et successivement déconnecté de chacun desdits circuits. De plus, un tel digesteur ne peut pas être transporté dans un lieu distinct du lieu de production du biogaz afin d'être chargé en biomasse solide et/ou déchargé en biomasse solide digérée résiduelle.

En d'autres termes, un tel digesteur anaérobie est fixe.

Le digesteur anaérobie immobile comprend une cuve apte à contenir de la biomasse solide, comportant au moins une zone d'admission de la biomasse solide, située sur la partie supérieure de la cuve, sur laquelle un toit est apte à s'ouvrir et à se refermer, et comportant en outre au moins une zone d'évacuation de la biomasse solide digérée résiduelle, certaines desdites zones d'admission et d'évacuation de biomasse solide pouvant être communes. De préférence, la zone d'admission et d'évacuation de la biomasse solide est commune.

Le digesteur anaérobie immobile comporte en outre :
- au moins une ouverture d'alimentation en digestat liquide, située sur la partie inférieure du digesteur anaérobie, reliée à au moins un circuit d'alimentation de digestat liquide, apte à permettre l'introduction de digestat liquide dans ledit digesteur,
- au moins une ouverture d'évacuation de digestat liquide, située sur la partie supérieure du digesteur anaérobie, reliée à au moins un circuit d'évacuation de digestat liquide, apte à permettre l'évacuation de digestat liquide dudit digesteur anaérobie,

- au moins une ouverture d'évacuation de biogaz, située sur la partie supérieure du digesteur anaérobie, reliée à au moins un circuit d'évacuation de biogaz, apte à permettre l'évacuation de biogaz depuis ledit digesteur,
- au moins une ouverture de vidange de digestat liquide, située sur la partie inférieure du digesteur anaérobie, apte(s) à permettre la vidange de digestat liquide dudit digesteur, et
- au moins une ouverture d'introduction et/ou d'évacuation apte(s) à permettre l'introduction et/ou l'évacuation d'air dans ledit digesteur,
certaines parmi lesdites ouvertures pouvant être communes à l'exception des ouvertures d'alimentation et d'évacuation de digestat liquide qui sont distinctes.

De préférence, la pluralité de digesteurs anaérobies tels que définis ci-avant comprend au moins un digesteur amovible et au moins un digesteur anaérobie immobile.

### Réseau d'alimentation en digestat liquide

Au sens de la présente invention, un réseau d'alimentation en digestat liquide comprend une pluralité de circuits d'alimentation de digestat liquide aptes à relier au moins une ouverture d'alimentation en digestat liquide d'un ou plusieurs digesteurs anaérobies de biomasse solide à l'unité centrale de stockage de digestat liquide et de digestion complémentaire ce qui permet d'alimenter, par voie directe ou indirecte, en digestat liquide le ou les digesteurs anaérobies de biomasse solide.

Par réseau d'alimentation de digestat liquide apte à permettre l'alimentation de digestat liquide par voie directe dans certains digesteurs de biomasse solide depuis une unité centrale de stockage de digestat liquide et de digestion complémentaire, on entend au sens de l'invention que lesdits digesteurs sont alimentés directement par du digestat liquide en provenance de ladite unité centrale de stockage de digestat liquide et de digestion complémentaire. Il s'agit d'une liaison en parallèle.

En particulier, pour chaque digesteur relié en parallèle à ladite unité centrale de stockage de digestat liquide et de digestion complémentaire, l'alimentation en digestat liquide se fait directement depuis ladite unité centrale de stockage de digestat liquide et de digestion complémentaire, sans transiter par un autre digesteur anaérobie.

Par réseau d'alimentation de digestat liquide apte à permettre l'alimentation de digestat liquide par voie indirecte dans certains digesteurs depuis une unité centrale de stockage de digestat liquide et de digestion complémentaire, on entend que certains digesteurs de biomasse solide sont alimentés par du digestat liquide en provenance de ladite unité centrale de stockage de digestat liquide et de digestion complémentaire à travers un ou plusieurs autres digesteurs précédents. Il s'agit d'une liaison en série.

En particulier, pour chaque digesteur de biomasse solide relié en série à ladite unité centrale de stockage de digestat liquide et de digestion complémentaire, l'alimentation en digestat liquide se fait de façon indirecte depuis ladite unité centrale de stockage de digestat liquide et de digestion complémentaire, et le digestat liquide transite par un ou plusieurs autres digesteurs anaérobies situés directement ou non aval avant d'alimenter ledit digesteur anaérobie.

Conformément à ce mode de réalisation, l'alimentation, par voie indirecte, en digestat liquide d'un digesteur anaérobie provient du digesteur anaérobie précédent, lequel digesteur peut être à son tour soit relié directement à ladite unité centrale de digestion complémentaire et de stockage du digestat liquide, soit relié à un autre digesteur anaérobie, et ainsi de suite.

De préférence, le ou les réseau(x) d'alimentation de digestat liquide comprennent des liaisons en parallèle c'est-à-dire que le réseau d'alimentation de digestat liquide est apte à permettre l'alimentation de digestat liquide par voie directe.

De préférence, le réseau d'alimentation en digestat liquide comprend une pluralité de circuits d'alimentation de digestat liquide reliant certains digesteurs anaérobies amovibles (ou plusieurs digesteurs anaérobies amovibles) à l'unité centrale de stockage de digestat liquide et de digestion complémentaire.

Plus précisément, le réseau d'alimentation en digestat liquide comprend une pluralité de circuits d'alimentation de digestat liquide reliant au moins une ouverture d'alimentation en digestat liquide de certains digesteurs anaérobies amovibles (ou plusieurs digesteurs anaérobies amovibles) à l'unité centrale de stockage de digestat liquide et de digestion complémentaire.

Conformément à ce mode de réalisation, le réseau d'alimentation en digestat liquide permet de relier au moins une ouverture d'alimentation en digestat liquide de certains digesteurs anaérobies amovibles à l'unité centrale de stockage de digestat liquide et de digestion complémentaire par voie directe.

Selon un mode de réalisation, le réseau d'alimentation en digestat liquide comprend une pluralité de circuits d'alimentation de digestat liquide reliant au moins une ouverture d'alimentation en digestat liquide d'au moins un digesteur anaérobie amovible et d'au moins un digesteur anaérobie immobile à l'unité centrale de stockage de digestat liquide et de digestion complémentaire.

### Réseau d'évacuation en digestat liquide

Au sens de la présente invention, un réseau d'évacuation en digestat liquide comprend une pluralité de circuits d'évacuation de digestat liquide aptes à relier au moins une ouverture d'évacuation en digestat liquide d'un ou plusieurs digesteurs anaérobies de biomasse solide à l'unité centrale de stockage de digestat liquide et de digestion complémentaire ce qui permet d'évacuer, par voie directe ou indirecte, le digestat liquide depuis le ou les digesteurs anaérobies de biomasse solide vers ladite unité centrale.

Par réseau d'évacuation de digestat liquide, apte à permettre l'évacuation de digestat liquide par voie directe de certains digesteurs de biomasse solide vers une unité centrale de stockage de digestat liquide et de digestion complémentaire, on entend au sens de l'invention que du digestat liquide contenu dans lesdits digesteurs est évacué directement vers ladite unité centrale de stockage de digestat liquide et de digestion complémentaire. Il s'agit d'une liaison en parallèle.

En particulier, depuis chaque digesteur relié en parallèle à ladite unité centrale de stockage de digestat liquide et de digestion complémentaire, l'évacuation de digestat liquide se fait directement vers ladite unité centrale de stockage de digestat liquide et de digestion complémentaire, sans transiter par un autre digesteur anaérobie.

Par réseau d'évacuation de digestat liquide, apte à permettre l'évacuation de digestat liquide par voie indirecte de certains digesteurs de biomasse solide vers une unité centrale de stockage de digestat liquide et de digestion complémentaire, on entend au sens de l'invention que du digestat liquide contenu dans lesdits digesteurs est évacué vers ladite unité centrale de stockage de digestat liquide et de digestion complémentaire à travers un ou plusieurs autres digesteurs. Il s'agit d'une liaison en série.

En particulier, pour chaque digesteur de biomasse solide relié en série à ladite unité centrale de stockage de digestat liquide et de digestion complémentaire, l'évacuation de digestat liquide se fait de façon indirecte vers ladite unité centrale de stockage de digestat liquide et de digestion complémentaire, et le digestat liquide transite par un ou plusieurs autres digesteurs anaérobies avant d'alimenter ladite unité centrale de stockage de digestat liquide et de digestion complémentaire.

Conformément à ce mode de réalisation, l'évacuation, par voie indirecte, de digestat liquide d'un digesteur anaérobie se fait vers le digesteur anaérobie suivant, lequel digesteur peut être à son tour soit être relié directement à l'unité centrale de digestion complémentaire et de stockage du digestat liquide, soit être relié à un autre digesteur anaérobie, et ainsi de suite.

De préférence, le ou les réseau(x) d'évacuation de digestat liquide comprennent des liaisons en parallèle c'es-à-dire que le réseau d'évacuation de digestat liquide est apte à permettre l'évacuation de digestat liquide par voie directe.

De préférence, le réseau d'évacuation en digestat liquide comprend une pluralité de circuits d'évacuation de digestat liquide reliant certains digesteurs anaérobies amovibles (ou plusieurs digesteurs anaérobies amovibles) à l'unité centrale de stockage de digestat liquide et de digestion complémentaire.

Plus précisément, le réseau d'évacuation en digestat liquide comprend une pluralité de circuits d'évacuation de digestat liquide reliant au moins une ouverture d'évacuation en digestat liquide de certains digesteurs anaérobies amovibles (ou plusieurs digesteurs anaérobies amovibles) à l'unité centrale de stockage de digestat liquide et de digestion complémentaire.

Conformément à ce mode de réalisation, le réseau d'évacuation en digestat liquide permet de relier au moins une ouverture d'évacuation en digestat liquide de certains digesteurs anaérobies amovibles à l'unité centrale de stockage de digestat liquide et de digestion complémentaire par voie directe.

Selon un mode de réalisation, le réseau d'évacuation en digestat liquide comprend une pluralité de circuits d'évacuation de digestat liquide reliant au moins une ouverture d'évacuation en digestat liquide d'au moins un digesteur anaérobie amovible et d'au moins un digesteur anaérobie immobile à l'unité centrale de stockage de digestat liquide et de digestion complémentaire.

### Réseau d'évacuation en biogaz

Au sens de la présente invention, un réseau d'évacuation de biogaz comprend une pluralité de circuits d'évacuation de biogaz aptes à relier au moins une ouverture d'évacuation en biogaz d'un ou plusieurs digesteurs anaérobies de biomasse solide à l'unité centrale de stockage de biogaz ce qui permet d'évacuer, par voie directe ou indirecte, le biogaz depuis le ou les digesteurs anaérobies de biomasse solide vers ladite unité centrale.

Par réseau d'évacuation de biogaz apte à permettre l'évacuation de biogaz par voie directe de certains digesteurs de biomasse solide vers une unité centrale de stockage de biogaz, on entend au sens de l'invention que du biogaz contenu dans lesdits digesteurs est évacué directement vers ladite unité centrale de stockage de biogaz et de digestion complémentaire. Il s'agit d'une liaison en parallèle.

En particulier, depuis chaque digesteur relié en parallèle à ladite unité centrale de stockage de biogaz, l'évacuation de biogaz se fait directement vers ladite unité centrale de stockage de biogaz, sans transiter par un autre digesteur anaérobie.

Par réseau d'évacuation de biogaz apte à permettre l'évacuation de biogaz par voie indirecte de certains digesteurs de biomasse solide vers une unité centrale de stockage de biogaz, on entend au sens de l'invention que du biogaz contenu dans lesdits digesteurs est évacué vers ladite unité centrale de stockage de biogaz à travers un ou plusieurs autres digesteurs. Il s'agit d'une liaison en série.

Conformément à ce mode de réalisation, pour chaque digesteur de biomasse solide relié en série à ladite unité centrale de stockage de biogaz, l'évacuation

En particulier, pour chaque digesteur de biomasse solide relié en série à ladite unité centrale de stockage de biogaz, l'évacuation de biogaz se fait de façon indirecte vers ladite unité centrale de stockage de biogaz, et le biogaz transite par un ou plusieurs autres digesteurs anaérobies avant d'alimenter ladite unité centrale de stockage de biogaz.

Conformément à ce mode de réalisation, l'évacuation, par voie indirecte, de biogaz d'un digesteur anaérobie se fait vers le digesteur anaérobie suivant, lequel digesteur peut être à son tour soit reliée directement à l'unité centrale de digestion complémentaire et de stockage du biogaz, soit reliée à un autre digesteur anaérobie, et ainsi de suite.

De préférence, le ou les réseau(x) d'évacuation de biogaz comprennent des liaisons en parallèle c'est-à-dire que le réseau d'évacuation de biogaz est apte à permettre l'évacuation de biogaz par voie directe.

De préférence, le réseau d'évacuation en biogaz comprend une pluralité de circuits d'évacuation de biogaz reliant certains digesteurs anaérobies amovibles (ou plusieurs digesteurs anaérobies amovibles) à l'unité centrale de stockage de biogaz.

Plus précisément, le réseau d'évacuation en biogaz comprend une pluralité de circuits d'évacuation de biogaz reliant au moins une ouverture d'évacuation en biogaz de certains digesteurs anaérobies amovibles (ou plusieurs digesteurs anaérobies amovibles) à l'unité centrale de stockage de biogaz.

Conformément à ce mode de réalisation, le réseau d'évacuation en biogaz permet de relier au moins une ouverture d'évacuation en biogaz de certains digesteurs anaérobies amovibles à l'unité centrale de stockage de biogaz en évacuant du biogaz par voie directe.

Selon un mode de réalisation, le réseau d'évacuation en biogaz comprend une pluralité de circuits d'évacuation de biogaz reliant au moins une ouverture d'évacuation en biogaz d'au moins un digesteur anaérobie amovible et d'au moins un digesteur anaérobie immobile à l'unité centrale de stockage de biogaz.

### Parties communes aux réseaux

Selon certains modes de réalisation, le réseau d'alimentation en digestat liquide, le réseau d'évacuation en digestat liquide et le réseau d'évacuation en biogaz présentent des parties communes, notamment un ou plusieurs circuits communs.

De préférence, le réseau d'évacuation en digestat liquide et le réseau d'évacuation en biogaz présentent des parties communes, notamment un ou plusieurs circuits en communs, c'est-à-dire un ou plusieurs circuits d'évacuation de digestat liquide et de biogaz.

### Dispositif de chauffage de digestat liquide

Le système de production de biogaz comprend en outre au moins un dispositif de chauffage de digestat liquide.

De préférence, l'unité centrale de stockage du digestat liquide et de digestion complémentaire peut être équipée d'au moins un dispositif de chauffage du digestat liquide qu'elle contient.

De cette façon, le digestat liquide qui est destiné à alimenter le ou les digesteur(s) anaérobie(s), de préférence le ou les digesteur(s) amovible(s), est de préférence maintenu à une température comprise entre 30 et 55°C, et avantageusement entre 35 et 40°C, régime mésophile, ou à une température comprise entre 45 et 55°C, régime thermophile.

L'alimentation en digestat liquide chaud, continue ou séquentielle, du ou des digesteur(s) anaérobies par l'unité centrale de digestion complémentaire et de stockage du digestat liquide, permet le maintien dans le ou les digesteur(s) anaérobies d'une température de réaction élevée sensiblement identique à la température du digestat liquide dans l'unité centrale de digestion et de stockage du digestat liquide, ce qui favorisera la réaction de digestion anaérobie dans chacun des digesteurs anaérobies, de préférence en mode mésophile ou thermophile.

### Unité centrale de stockage de digestat liquide et de digestion complémentaire

L'unité centrale de stockage de digestat liquide et de digestion complémentaire est unité apte à stocker du digestat liquide dans laquelle une digestion complémentaire peut avoir lieu.

L'unité centrale de stockage de digestat liquide et de digestion complémentaire a aussi de préférence une fonction supplémentaire de production de biogaz.

En effet, durant l'étape de digestion, la recirculation du digestat liquide provenant du ou des digesteurs anaérobies de biomasse solide vers l'unité centrale de stockage du digestat liquide et digestion complémentaire, après diffusion du digestat liquide dans la biomasse solide, peut provoquer des entrainements avec le digestat liquide de matières fines solides biodégradables non encore digérées et/ou des entrainements de substances dissoutes biodégradables non encore digérées, comme par exemple des acides gras volatiles n'ayant pas eu le temps d'être transformés en biogaz dans le digesteur anaérobie.

Ainsi, la méthanisation résiduelle de ces matières se produit dans l'unité centrale de digestion complémentaire et de stockage du digestat liquide.

De préférence, l'unité centrale de stockage du digestat liquide et de digestion complémentaire ne contient pas de biomasse solide ou dans des quantités très faibles.

### Moyen de circulation de digestat liquide

De préférence, le moyen de circulation de digestat liquide est une pompe située sur le réseau d'alimentation de digestat liquide.

### Système de production de biogaz

Chaque digesteur anaérobie de biomasse solide du système comprend une cuve apte à contenir de la biomasse solide, laquelle cuve comporte une ou plusieurs zone(s) d'admission de la biomasse solide. Ladite ou lesdites zone(s) d'admission, est (sont) située(s) en particulier sur au moins une partie de la surface supérieure de la cuve sur laquelle le toit s'ouvre et se referme, permettant l'incorporation de la biomasse solide à l'intérieur de la cuve.

La zone d'admission permet donc d'introduire directement la biomasse solide brute (i.e. non traitée) dans le digesteur sans avoir à effectuer une opération de séparation des éléments indésirables ou une dilution préalable dans un liquide ou un broyage, ce qui permet de diminuer les investissements dans les équipements de préparation de la biomasse solide, de diminuer les dépenses énergétiques, de maintenance et de personnel et les risques d'endommagement par rapport à des unités de méthanisation classiques.

En particulier, les digesteurs anaérobies du système selon l'invention permettent de s'affranchir d'un dispositif visant à séparer les éléments indésirables de la biomasse solide, d'un dispositif de broyage et d'un dispositif d'agitation de la biomasse dans le digesteur.

Après introduction de la biomasse solide, et fermeture du toit, la cuve des digesteurs est remplie avec du digestat liquide en provenance, directe ou indirecte, d'au moins une unité centrale de stockage de digestat liquide et de digestion complémentaire apte à stocker du digestat liquide. Après remplissage de la cuve, d'abord par de la biomasse solide, puis par du digestat liquide, la méthanisation se déroule par percolation du digestat liquide à travers la biomasse solide et diffusion du digestat liquide dans la biomasse solide, laquelle est totalement immergée (et éventuellement partiellement mise en suspension), dans des conditions anaérobies au sein de la cuve des digesteurs.

La digestion de la biomasse solide par percolation du digestat liquide à travers la biomasse solide et diffusion du digestat liquide dans la biomasse solide permet de s'affranchir de la nécessité d'agiter le mélange de biomasse solide et de digestat liquide dans la cuve du digesteur, évitant ainsi des investissements en moyens mécaniques d'agitation et des coûts d'exploitation.

La cuve de chacun des digesteurs de biomasse solide comporte en outre une ou plusieurs zone(s) d'évacuation de la biomasse solide digérée résiduelle une fois la digestion terminée, c'est-à-dire d'évacuation du digestat solide.

De cette façon, le système de production selon l'invention permet donc de valoriser plus efficacement la biomasse solide en réduisant les investissements en divers équipements, les dépenses énergétiques, de personnel et de maintenance des équipements, l'usure des matériels et les risques d'endommagement.

Selon une caractéristique de l'invention, certains desdits digesteurs de biomasse solide sont surmontés d'un moyen de préhension apte à permettre l'alimentation de la biomasse solide.

En d'autres termes, le moyen de préhension est apte à charger de la biomasse solide dans certains digesteurs de biomasse solide sur le lieu d'implantation du système de production de biogaz.

De préférence, le moyen de préhension est apte à charger de la biomasse solide dans au moins un digesteur anaérobie immobile et à décharger la biomasse solide digérée résiduelle, une fois la digestion terminée, à travers la zone d'admission du digesteur.

Le moyen de préhension permet donc de saisir la biomasse solide et de l'incorporer dans le digesteur sans avoir à effectuer une étape de tri ou de séparation préalable des éléments indésirables susceptibles d'endommager les installations composant l'unité de méthanisation, et sans avoir à effectuer une étape de broyage ni de dilution de la biomasse solide.

Le moyen de préhension permet aussi de récupérer la biomasse solide digérée résiduelle une fois la digestion terminée, c'est-à-dire le digestat solide, et de l'évacuer du digesteur.

Ainsi le moyen de préhension permet d'alimenter et de décharger le digesteur par le haut à travers la zone d'admission (respectivement d'évacuation) située sur au moins une partie de la surface supérieure de la cuve sur laquelle se referme le toit.

De préférence, le moyen de préhension est un grappin comportant une pluralité de crochets ou une benne comportant des godets.

Selon une caractéristique de l'invention, dans certains desdits digesteurs de biomasse solide, au moins l'une des ouvertures d'évacuation du digestat liquide comprend au moins un élément de séparation perforé d'évacuation du digestat liquide, situé en amont de ladite ouverture d'évacuation du digestat liquide, apte à séparer, d'une part, le digestat liquide évacué et, d'autre part, la biomasse solide.

En particulier, l'élément de séparation perforé d'évacuation du digestat liquide permet l'évacuation du digestat liquide au cours de l'étape de digestion de la biomasse solide tout en retenant la biomasse solide au sein du digesteur.

En outre, l'évacuation du biogaz peut se faire et se fait de préférence concomitamment à l'évacuation et la recirculation du digestat liquide vers l'unité centrale de digestion complémentaire et de stockage du digestat liquide.

Selon une caractéristique de l'invention, dans certains desdits digesteurs de biomasse solide, au moins l'une des ouvertures de vidange du digestat liquide comprend au moins un élément de séparation perforé de vidange du digestat liquide, situé en amont de ladite ouverture de vidange du digestat liquide, apte à séparer, d'une part, le digestat liquide vidangé et, d'autre part, la biomasse solide.

En particulier, l'élément de séparation perforé de vidange du digestat liquide permet la circulation du digestat liquide lors des étapes de vidanges anaérobies et aérobies du digesteur tout en retenant la biomasse solide dans la cuve desdits digesteurs anaérobies de biomasse solide.

De préférence, certains éléments de séparation perforés sont des plaques perforées.

Plus préférentiellement, l'élément de séparation perforé d'évacuation du digestat liquide et l'élément de séparation perforé de vidange du digestat liquide sont des plaques perforées.

En particulier, l'élément de séparation perforé de vidange du digestat liquide est un plancher perforé pouvant être situé sur la totalité ou une partie du sol du digesteur anaérobie.

Selon une caractéristique de l'invention, au moins un des digesteurs amovibles comporte, au niveau d'au moins l'une de ses parties latérales, au moins une trappe de déchargement de la biomasse solide digérée résiduelle, laquelle trappe est apte à être ouverte et refermée de façon étanche.

En d'autres termes, selon un mode de réalisation, au moins l'un des digesteurs anaérobies amovibles comporte, au niveau d'au moins l'une de ses parties latérales, au moins une zone de déchargement de la biomasse solide digérée résiduelle.

Plus préférentiellement, chacun des digesteurs anaérobies amovibles peut être un container de camion, équipé d'un toit ouvrant capable d'être fermé de façon étanche, et équipé d'un côté ouvrant, situé au niveau de l'une de ses parties latérales, capable d'être fermé de façon étanche, rendant ainsi le digesteur anaérobie amovible apte à être vidé par le côté ouvrant, par renversement.

Selon une caractéristique de l'invention, sur certains desdits digesteurs de biomasse solide :
- la ou les ouverture(s) d'alimentation en digestat liquide, est (sont) munie(s) d'un moyen d'obstruction, de préférence une vanne, apte à obturer ou permettre le passage du digestat liquide entre l'extérieur et l'intérieur du digesteur, et/ou
- la ou les ouverture(s) d'évacuation de digestat liquide, est (sont) munie(s) d'un moyen d'obstruction, de préférence une vanne, apte à obturer ou permettre le passage du digestat liquide entre l'intérieur et l'extérieur du digesteur, et/ou
- la ou les ouverture(s) d'évacuation de biogaz, est (sont) munie(s) d'un moyen d'obstruction, de préférence une vanne, apte à obturer ou permettre le passage du biogaz entre l'intérieur et l'extérieur du digesteur, et/ou
- la ou les ouverture(s) de vidange de digestat liquide, est (sont) munie(s) d'un moyen d'obstruction, de préférence une vanne, apte à obturer ou permettre le passage du digestat liquide entre l'intérieur et l'extérieur du digesteur, et/ou
- la ou les ouverture(s) d'introduction et/ou d'évacuation d'air, est (sont) munie(s) d'un moyen d'obstruction, de préférence une vanne, apte à obturer ou permettre le passage de l'air entre l'intérieur et l'extérieur du digesteur.

En particulier, le ou les moyens d'obstruction, situé(s) sur la ou les ouvertures d'alimentation en digestat liquide, est ou sont apte(s) à permettre l'alimentation en digestat liquide du ou des digesteur(s) en provenance du réseau d'alimentation de digestat liquide.

En particulier, le ou les moyens d'obstruction, situé(s) sur la ou les ouvertures d'évacuation de digestat liquide, est ou sont apte(s) à permettre l'évacuation du digestat liquide depuis le (ou lesdits) digesteur(s) vers le réseau d'évacuation du digestat liquide.

En particulier, le ou les moyens d'obstruction, situé(s) sur la ou les ouvertures d'évacuation de biogaz, est ou sont apte(s) à permettre l'évacuation du biogaz depuis le (ou lesdits) digesteur(s) vers le réseau d'évacuation de biogaz.

En particulier, le ou les moyens d'obstruction, situé(s) sur la ou les ouverture(s) de vidange de digestat liquide, est ou sont apte(s) à permettre la vidange du digestat liquide depuis le (ou lesdits) digesteur(s) vers l'unité centrale de stockage de digestat liquide et de digestion complémentaire.

En particulier, le ou les moyens d'obstruction, situé(s) sur la ou les ouverture(s) d'introduction et/ou d'évacuation d'air, est ou sont apte(s) à permettre l'introduction ou l'évacuation de l'air dans le digesteur.

De préférence, lorsque certaines ouvertures sont communes alors les moyens d'obstruction desdites ouvertures sont également communs.

De préférence, lorsque l'ouverture d'évacuation de digestat liquide et l'ouverture d'évacuation de biogaz sont communs alors le ou les moyens d'obstruction, situé(s) sur chacune desdites ouvertures, sont communs.

Ainsi le ou les moyens d'obstruction, situé(s) sur la ou les ouvertures d'évacuation de digestat liquide et de biogaz, est ou sont apte(s) à permettre l'évacuation du digestat liquide et du biogaz vers le réseau d'évacuation de digestat liquide et le réseau d'évacuation de biogaz, notamment vers leurs parties communes.

De préférence, les moyens d'obstructions présents dans le système de production de biogaz sont des vannes.

Conformément à cette caractéristique de l'invention, le ou les digesteurs de biomasse solide est ou sont amovibles et inamovibles.

Selon une caractéristique de l'invention, certains desdits digesteurs anaérobies de biomasse solide comportent un joint liquide positionné autour du toit de la cuve, apte à empêcher la sortie du biogaz contenu dans ledit digesteur lorsque le toit est fermé, et apte à empêcher les entrées d'air dans ledit digesteur lorsque le toit est fermé.

De préférence, le joint liquide est positionné autour du toit de la cuve d'un digesteur anaérobie immobile.

Le joint liquide permet d'assurer une meilleure étanchéité du toit et d'éviter les fuites éventuelles de biogaz. De préférence, le joint liquide est de l'eau.

Selon un mode de réalisation, le joint liquide est équipé d'un robinet d'eau apte à remplir d'eau le joint liquide, de façon continue ou séquentielle, de telle sorte que le joint liquide soit toujours rempli d'eau et qu'aucune fuite de biogaz hors du digesteur anaérobie ni aucune entrée d'air dans le digesteur anaérobie ne puisse survenir.

Selon une caractéristique de l'invention, certains ouvrages composant l'unité centrale de stockage de digestat liquide et de digestion complémentaire, d'une part, et certains ouvrages composant l'unité centrale de stockage de biogaz, d'autre part, sont communs.

En variante, l'unité centrale de stockage de digestat liquide et de digestion complémentaire et l'unité centrale de stockage de biogaz constituent deux ouvrages distincts.

L'unité centrale de stockage du digestat liquide et de digestion complémentaire peut aussi correspondre à un digesteur apte à stocker conjointement du digestat liquide et du biogaz ou à deux cuves distinctes capables de stocker séparément du digestat liquide et du biogaz.

Dans certains cas, l'unité centrale de stockage du digestat liquide et de digestion complémentaire peut comporter plusieurs digesteurs aptes à stocker conjointement du digestat liquide et du biogaz ou plusieurs cuves distinctes capables de stocker séparément du digestat liquide et du biogaz.

Dans certains cas, l'unité centrale de stockage de digestat liquide et de digestion complémentaire peut notamment être constituée de plusieurs digesteurs anaérobies amovibles, éventuellement identiques ou semblables au digesteur anaérobie amovible décrit dans l'invention, aptes à stocker conjointement du digestat liquide. Dans ce cas, le biogaz peut avantageusement être stocké dans un ou plusieurs réservoirs souples dédiés et séparés.

De préférence, l'unité centrale de stockage de digestat liquide et de digestion complémentaire est distincte d'un digesteur anaérobie amovible.

De préférence, l'unité centrale de stockage de digestat liquide et de digestion complémentaire, d'une part, et l'unité centrale de stockage de biogaz, d'autre part, sont communes et constituent une seule et même unité centrale de stockage de digestat liquide et de biogaz et de digestion complémentaire.

Ainsi l'unité centrale de stockage du digestat liquide et de digestion complémentaire correspond à un digesteur apte à stocker conjointement du digestat liquide et du biogaz et constitué d'un seul ouvrage, en particulier comprenant du digestat liquide et du biogaz.

Selon une caractéristique de l'invention, sur certains des digesteurs de biomasse solide, au moins une ouverture d'évacuation de digestat liquide et une ouverture d'évacuation de biogaz sont communes et sont reliées à au moins un circuit d'évacuation d'un mélange de digestat liquide et de biogaz, lequel circuit est connecté à un moyen de séparation apte à séparer le biogaz et le digestat liquide.

De préférence, au moins un digesteur amovible est calorifugé.

L'invention concerne également un procédé de production de biogaz comprenant au moins les étapes successives suivantes :
- une étape de chargement en biomasse solide d'au moins un digesteur amovible, tel que défini précédemment, mise en œuvre dans un lieu de chargement autre que le lieu d'implantation du système de production de biogaz, tel que défini précédemment, manuellement ou par au moins un moyen de chargement, à travers la zone d'admission,
- une étape de transport dudit digesteur amovible chargé en biomasse solide, depuis le lieu de chargement de la biomasse solide jusqu'au lieu d'implantation dudit système de production de biogaz,
- une étape de connexion dudit digesteur amovible chargé en biomasse solide à au moins un circuit d'alimentation de digestat liquide et à au moins un circuit d'évacuation de digestat liquide et à au moins un circuit d'évacuation de biogaz,
- une étape d'alimentation dudit digesteur amovible avec du digestat liquide pour immerger la biomasse solide contenue dans ledit digesteur amovible,
- une étape de digestion de la biomasse solide dans ledit digesteur amovible, comprenant la percolation du digestat liquide à travers la biomasse solide et la diffusion du digestat liquide dans la biomasse solide dans des conditions anaérobies pour générer puis récupérer du biogaz, et
- une étape de vidange aérobie du digestat liquide du digesteur amovible.

De préférence, l'étape de chargement en biomasse solide est mise en œuvre sur le lieu de récupération de la biomasse solide, par exemple sur une ferme agricole.

L'étape de chargement permet d'introduire la biomasse solide manuellement ou par au moins un moyen de chargement, à travers la zone d'admission du digesteur anaérobie amovible.

De préférence, le chargement peut être mis en œuvre par un godet pouvant être relié à un camion chargeur à godet, un tracteur agricole à godet ou de tout autre moyen de chargement.

L'étape de chargement peut également avoir lieu sur la route.

Durant l'étape de digestion, de préférence, une circulation du digestat liquide en aller et retour entre l'unité centrale de stockage du digestat liquide et de digestion complémentaire et le digesteur anaérobie amovible, est maintenue de façon continue ou séquentielle, afin que :
- la température du digestat liquide dans le digesteur anaérobie amovible soit maintenue à peu près constante à une valeur proche de la température du digestat liquide dans l'unité centrale de digestion complémentaire et de stockage du digestat liquide,
- le digestat liquide présent dans le digesteur anaérobie amovible soit renouvelé ainsi que la flore bactérienne qu'il contient afin de favoriser les réactions biologiques à l'œuvre au cours de l'étape de digestion anaérobie,
- des matières solubles (par exemple des acides gras volatils) ou des particules solides fines, issues de la biomasse solide à digérer, soient entrainées dans l'unité centrale de stockage du digestat liquide et de digestion complémentaire, où leur digestion anaérobie sera complétée.

De préférence, l'étape de vidange aérobie du digestat liquide peut consister en un pompage à partir de la partie inférieure du digesteur anaérobie amovible vers l'unité centrale de stockage du digestat liquide et de digestion complémentaire à travers l'élément de séparation de vidange du digestat liquide.

De façon alternative, l'étape de vidange aérobie du digestat liquide peut consister en un écoulement gravitaire du digestat liquide vers une zone de récupération du digestat liquide, depuis laquelle le digestat liquide peut être repompé ou non dans l'unité centrale de stockage du digestat liquide et de digestion complémentaire. Durant cette étape, l'espace vacant laissé par l'évacuation du digestat liquide est rempli par de l'air en provenance de l'extérieur du digesteur anaérobie amovible. A l'issue de cette étape, la biomasse solide partiellement ou totalement digérée peut venir reposer sur le fond du digesteur anaérobie amovible et/ou sur l'élément de séparation inférieur.

Dans ce cas, la zone de récupération du digestat liquide, depuis laquelle le digestat liquide peut être pompé ou non dans l'unité centrale de digestion complémentaire et de stockage du digestat liquide, est de préférence une fosse de relevage.

De préférence, le procédé comprend, en outre, après l'étape de vidange aérobie du digestat liquide du digesteur amovible, les étapes suivantes :
- une étape de déconnexion dudit digesteur amovible, chargé en biomasse solide digérée résiduelle, du circuit d'alimentation de digestat liquide et du circuit d'évacuation de digestat liquide et du circuit d'évacuation de biogaz,
- une étape de transport dudit digesteur amovible chargé en biomasse solide digérée résiduelle, depuis le lieu d'implantation du système de production de biogaz jusqu'à un lieu de déchargement de la biomasse solide digérée,
- une étape d'évacuation de la biomasse solide digérée résiduelle, mise en œuvre, de préférence, par renversement du digesteur amovible provoquant l'évacuation de la biomasse solide digérée résiduelle à travers une zone de déchargement latérale, ou par au moins un moyen de préhension, à travers une zone d'évacuation de la biomasse solide digérée résiduelle situé sur la partie supérieure de la cuve, sur laquelle un toit est apte à s'ouvrir et à se refermer.

De préférence, l'étape de chargement ainsi que l'étape d'évacuation de la biomasse solide digérée peut être mise en œuvre par un grappin ou des godets.

En d'autres termes, le digesteur anaérobie amovible est alimenté par le haut en biomasse solide à travers une zone sur laquelle le toit du digesteur s'ouvre et se referme. Parallèlement, le digesteur anaérobie amovible peut être vidé de la biomasse solide digérée résiduelle soit par renversement du digesteur anaérobie amovible et évacuation de la biomasse solide à travers une paroi latérale ouvrante, soit par le haut, par exemple à l'aide d'un moyen de préhension, à travers une zone sur laquelle le toit du digesteur s'ouvre et se referme.

Le procédé de production du biogaz présente donc l'avantage de ne pas nécessiter une étape de préparation et/ou de séparation des éléments indésirables et/ou de broyage et/ou de dilution préalable de la biomasse solide ni de nécessiter de brassage de la biomasse solide dans le digesteur, ce qui permet de minimiser les investissements en équipements et les dépenses énergétiques associées à de telles étapes ainsi que les risques éventuels d'endommagement des équipements du digesteur ou des équipements mis en œuvre au cours du procédé et de diminuer les coûts de personnel et de maintenance liés aux différentes étapes évitées.

Le procédé de production de biogaz selon l'invention est un procédé de méthanisation discontinu, ou en bâchées.

De préférence, le procédé de production de biogaz comprend en outre entre l'étape de digestion et l'étape de vidange aérobie du digestat liquide au moins les étapes suivantes :
- une étape de vidange anaérobie du digestat liquide du digesteur amovible, et
- une étape de re-remplissage du digesteur amovible avec un digestat liquide après vidange anaérobie.

De préférence, l'étape de vidange anaérobie du digestat liquide consiste en un pompage à partir de la partie inférieure du digesteur anaérobie amovible vers l'unité centrale de stockage du digestat liquide et de digestion complémentaire à travers l'élément de séparation inférieur dans des conditions anaérobies.

De façon alternative, cette étape vidange peut consister en un écoulement gravitaire du digestat liquide vers une zone de récupération du digestat liquide, depuis laquelle le digestat liquide peut être repompé ou non dans l'unité centrale de stockage du digestat liquide et de digestion complémentaire.

Durant cette étape, l'espace vacant laissé par l'évacuation du digestat liquide est rempli par du biogaz en provenance de l'unité centrale de stockage du digestat liquide et de digestion complémentaire. A l'issue de cette étape la biomasse solide partiellement ou totalement digérée vient reposer sur le fond du digesteur anaérobie amovible ou sur l'élément de séparation inférieur situé sur le fond du digesteur anaérobie amovible.

Dans ce cas, la zone de récupération du digestat liquide, depuis laquelle le digestat liquide peut être pompé ou non dans l'unité centrale de digestion complémentaire et de stockage du digestat liquide, est de préférence une fosse de relevage.

De préférence, le procédé de production de biogaz comprend en outre, après l'étape de re-remplissage du digesteur amovible avec un digestat liquide, au moins une étape de digestion, constituée d'une percolation du digestat liquide à travers la biomasse solide et d'une diffusion du digestat liquide dans la biomasse solide, pour générer puis récupérer du biogaz.

De préférence, au cours de l'étape de re-remplissage, le digestat liquide provient de l'unité centrale de stockage du digestat liquide et de digestion complémentaire.

Selon une caractéristique de l'invention, entre l'étape de re-remplissage du digesteur anaérobie amovible avec du digestat liquide et l'étape de vidange aérobie du digestat liquide, le procédé comprend en outre au moins une étape de digestion constituée par une percolation du digestat liquide à travers la biomasse solide et une diffusion du digestat liquide dans la biomasse solide pour générer puis récupérer le biogaz à travers la zone d'évacuation du biogaz située dans la partie supérieure du digesteur.

L'étape de digestion supplémentaire est identique à l'étape de digestion précédemment décrite.

En particulier, le procédé selon l'invention comporte une étape d'ouverture du toit du digesteur anaérobie amovible avant l'étape de chargement afin de permettre le remplissage par le haut du digesteur anaérobie amovible avec de la biomasse solide. La zone d'admission est ainsi située sur au moins une partie de la surface supérieure du digesteur anaérobie amovible, sur laquelle le toit se referme.

Selon un mode de réalisation, le procédé comporte une étape de fermeture du toit sur la zone d'admission du digesteur anaérobie amovible préalablement à l'étape de transport du digesteur anaérobie amovible vers le lieu de digestion où est installée l'unité centrale de digestion complémentaire et de stockage du digestat liquide.

De préférence, l'étape de remplissage et de fermeture du toit du digesteur anaérobie amovible a lieu sur un lieu différent du lieu de digestion où est installée l'unité centrale de digestion et de stockage du digestat liquide.

Selon un mode de réalisation, l'étape chargement du digesteur anaérobie amovible et de fermeture du toit du digesteur anaérobie amovible est suivie par l'étape de transport du digesteur anaérobie amovible rempli de la matière organique destinée à être digérée, durant laquelle le digesteur anaérobie amovible est transporté depuis le lieu de remplissage vers le lieu de digestion où est installée l'unité centrale de digestion complémentaire et de stockage du digestat liquide.

Selon un mode de réalisation, l'étape de transport du digesteur anaérobie amovible rempli de la matière organique destinée à être digérée est suivie par une étape de connexion du digesteur anaérobie amovible à l'unité centrale de stockage du digestat liquide et de digestion complémentaire, durant laquelle le digesteur anaérobie amovible est connecté par plusieurs liaisons à l'unité centrale de stockage du digestat liquide et de digestion complémentaire et l'unité centrale de stockage de biogaz :
- en premier lieu, le digesteur anaérobie amovible est connecté au circuit d'alimentation en digestat liquide reliant le digesteur anaérobie amovible à l'unité centrale de stockage du digestat liquide et de digestion complémentaire par le biais du réseau d'alimentation en digestat liquide. Cette alimentation en digestat liquide a lieu dans la partie inférieure du digesteur anaérobie amovible,
- en deuxième lieu, le digesteur anaérobie amovible est connecté à un circuit d'évacuation de digestat liquide reliant le digesteur anaérobie amovible à l'unité centrale de stockage du digestat liquide et de digestion complémentaire par le biais du réseau de d'évacuation de digestat liquide. Cette évacuation a lieu depuis la partie haute du digesteur anaérobie amovible
- en troisième lieu, le digesteur anaérobie amovible est connecté à un circuit d'évacuation en biogaz reliant le digesteur anaérobie amovible à l'unité centrale de stockage en biogaz et l'unité centrale de stockage du digestat liquide et de digestion complémentaire par le biais du réseau d'évacuation de biogaz. Cette évacuation en biogaz a lieu depuis la partie haute du digesteur anaérobie amovible.

De préférence, le digesteur anaérobie amovible est connecté à un circuit d'évacuation de digestat liquide et de biogaz, c'est-à-dire à un circuit commun au réseau d'évacuation de digestat liquide et au réseau d'évacuation de biogaz.

En variante, l'alimentation en digestat liquide peut être effectuée dans la partie supérieure du digesteur anaérobie amovible de manière à ce que le digestat liquide circule du haut vers le bas afin d'empêcher tout phénomène de croutage ou d'accumulation de biomasse solide en haut du digesteur anaérobie amovible.

Le digestat liquide est introduit à l'intérieur du digesteur anaérobie amovible par le biais d'une ou plusieurs pompes d'alimentation, en provenance de l'unité centrale de stockage du digestat liquide et de digestion complémentaire.

Selon un mode de réalisation, après l'étape de remplissage du digesteur anaérobie amovible par la biomasse solide à digérer et du digestat liquide, le ou les moyens d'obstruction, situé(s) sur l'ouverture d'introduction et/ou d'évacuation d'air, sont en fermés de manière à ce que la réaction de méthanisation se déroule dans des conditions anaérobies.

L'étape de percolation du digestat liquide à travers la biomasse diffusion du digestat liquide dans la biomasse et de digestion anaérobie conduit, au niveau de la partie supérieure du digesteur, à la formation d'un flux de biogaz et de digestat liquide.

La percolation et la diffusion sont assurées par la mise en œuvre d'une ou plusieurs pompes de circulation située(s) entre l'unité centrale de stockage du digestat liquide et de digestion complémentaire et le digesteur anaérobie amovible.

En particulier, la ou les pompe(s) de circulation permet(tent) d'assurer une pression du digestat liquide sur la biomasse solide, l'obligeant à la traverser, ce qui favorise un contact intime entre les bactéries contenues notamment dans le digestat liquide et la biomasse solide à dégrader.

La percolation et la diffusion sont favorisées par la pression exercée par le pompage du digestat liquide sur la biomasse solide. Cette percolation et cette diffusion favorisent le contact intime entre les bactéries et la biomasse solide à traiter.

De plus, le pompage et la circulation forcée du digestat liquide entre l'unité centrale de stockage du digestat liquide et de digestion complémentaire et le digesteur anaérobie amovible présentent l'avantage de faire circuler une très grande quantité de digestat liquide à travers un volume plus faible de biomasse solide à traiter, ce qui limite les risques de survenue d'acidose dans le digesteur anaérobie amovible, aux alentours de la biomasse solide à traiter, ainsi que les risques d'inhibition de certaines réactions biologiques à l'œuvre lors de la digestion anaérobie susceptibles de se produire en cas d'accumulation locale de substances inhibitrices ou toxiques.

En effet, dans les autres procédés de méthanisation, des quantités trop importantes de biomasse à traiter dans le digesteur principal peuvent conduire à une réaction d'acidogénèse excessive, pouvant provoquer une acidification de la totalité du digestat liquide, ou acidose, laquelle entraine une inhibition partielle, voire totale, de la méthanogénèse et donc de la production de méthane. En synthèse, une telle réaction est provoquée lorsque la production d'acides gras volatils, induite par des bactéries acidogènes, est plus importante que la capacité de transformation de ces acides gras en méthane par les bactéries méthanogènes.

Selon un mode de réalisation, le procédé comprend, au moment de l'évacuation du biogaz et digestat liquide l'unité centrale de stockage du digestat liquide et de digestion complémentaire, une étape de séparation, mise en œuvre au moyen d'au moins un élément de séparation perforé d'évacuation du digestat liquide.

L'élément de séparation perforé d'évacuation du digestat liquide permet d'extraire le digestat liquide vers l'unité centrale de digestion et de stockage du digestat liquide et de retenir dans le digesteur anaérobie amovible la biomasse solide qui se trouve en suspension dans le digesteur anaérobie amovible.

En particulier, pendant la phase de digestion anaérobie, le ou les moyens d'obstructions, situé(s) sur le ou les ouvertures de digestat liquide, sont ouvertes afin de permettre l'évacuation du digestat liquide vers l'unité centrale de stockage du digestat liquide et de digestion complémentaire.

Selon un mode de réalisation, l'étape de vidange anaérobie du digestat liquide est mise en œuvre en vidant, au niveau de la partie inférieure de la cuve du digestat liquide, ce qui provoque le tassement de la biomasse solide au fond du digesteur anaérobie amovible, ou sur l'élément de séparation de vidange de digestat liquide, et permet d'éliminer les éventuels chemins préférentiels de diffusion ainsi que les éventuelles poches de biogaz bloquées dans la biomasse solide.

L'étape de vidange anaérobie peut être mise en œuvre en aspirant le digestat liquide par le biais d'une ou plusieurs pompe(s) montée(s) sur un circuit de vidange entre le digesteur anaérobie amovible et l'unité centrale de stockage du digestat liquide et de digestion complémentaire.

L'aspiration du digestat liquide peut de préférence avoir lieu en aval de l'élément perforé de vidange de digestat liquide pour éviter que la biomasse solide soit entrainée avec le digestat liquide vers l'unité centrale de digestion complémentaire et de stockage du digestat liquide.

De façon alternative, l'étape de vidange anaérobie peut être mise en œuvre par l'ouverture d'au moins un moyen d'obstruction, situé sur le ou les ouvertures de vidange de digestat liquide, et en laissant s'écouler de manière gravitaire le digestat liquide par le conduit de vidange. Dans ce cas de figure, le digestat liquide s'écoule de préférence dans une fausse de vidange, depuis laquelle il pourra opportunément être pompé vers l'unité centrale de de stockage du digestat liquide et de digestion complémentaire.

La vidange gravitaire du digestat liquide peut de préférence avoir lieu en aval de l'élément perforé de digestat liquide pour éviter que la biomasse solide soit entrainée avec le digestat liquide et extraite du digesteur anaérobie amovible. L'étape de vidange anaérobie permet au biogaz en provenance de l'unité centrale de stockage du digestat liquide et de digestion complémentaire de remplir l'ensemble du volume libéré dans la cuve en remplacement du digestat liquide qui est aspiré par la pompe vers l'unité centrale de stockage du digestat liquide et de digestion complémentaire ou bien vidangé gravitairement du digesteur anaérobie amovible.

Après l'étape de vidange anaérobie, une étape de re-remplissage du digesteur anaérobie amovible avec du digestat liquide est à nouveau mise en œuvre afin de poursuivre la réaction de méthanisation.

De la même façon que précédemment, l'étape de digestion conduit à la formation de biogaz récupéré au niveau de l'ouverture d'évacuation de biogaz.

Selon un mode de réalisation, l'étape de vidange aérobie du digestat liquide du digesteur anaérobie amovible est réalisée à la fin du cycle de méthanisation.

Une telle étape est mise en œuvre en fermant le ou les moyens d'obstruction, de préférence la ou les vannes, situé(s) sur le ou les ouvertures d'évacuation de biogaz et le ou les ouvertures d'évacuation de digestat liquide, et en ouvrant le moyen d'obstruction, de préférence la ou les vannes, situé(s) sur le ou les ouvertures d'introduction et/ou d'évacuation d'air, et en ouvrant le ou les moyens d'obstruction, situé(s) sur le ou les ouvertures de vidange de digestat liquide.

L'étape de vidange aérobie est mise en œuvre en ouvrant le ou les moyens d'obstruction, situé(s) sur l'ouverture de vidange de digestat liquide, et en laissant s'écouler de manière gravitaire le digestat liquide par le conduit de vidange. Dans ce cas de figure, le digestat liquide s'écoulera de préférence dans une fausse de vidange, depuis laquelle il pourra opportunément être pompé vers l'unité centrale de digestion et de stockage du digestat liquide.

L'étape de vidange aérobie peut être mise en œuvre en aspirant le digestat liquide par le biais de la ou les pompe(s) située(s) entre le digesteur anaérobie amovible et l'unité centrale de digestion et de stockage du digestat liquide.

L'aspiration du digestat liquide a lieu de préférence en aval de l'élément perforé de vidange de digestat liquide pour éviter que la biomasse solide soit entrainée avec le digestat liquide vers l'unité centrale de digestion complémentaire et de stockage du digestat liquide.

L'étape de vidange aérobie permet à l'air en provenance de l'extérieur de remplir l'ensemble du volume libéré dans la cuve en remplacement du digestat liquide qui est aspiré par la pompe vers l'unité centrale de stockage du digestat liquide et de digestion complémentaire ou bien vidangé gravitairement du digesteur anaérobie amovible.

L'intérêt de la vidange gravitaire du digestat liquide contenu dans le digesteur anaérobie amovible réside dans le fait qu'une telle opération peut être réalisée pendant un temps long, plusieurs heures, voire plusieurs jours. Ceci permet à la biomasse solide digérée résiduelle, restée retenue dans le digesteur anaérobie amovible par l'élément de séparation perforé de vidange de digestat liquide, de pouvoir s'égoutter convenablement afin d'atteindre une plus grande siccité, ce qui pourra faciliter la manipulation et l'usage ultérieur qui seront fait de cette matière solide digérée, tel que, par exemple, un épandage sur des terres agricoles.

Selon un mode de réalisation, l'étape de digestion de la biomasse est suivie par une étape de déconnexion du digesteur anaérobie amovible de l'unité centrale de digestion complémentaire et de stockage du digestat liquide, durant laquelle le digesteur anaérobie amovible est déconnecté :
- en premier lieu, dans sa partie supérieure, du circuit d'évacuation en biogaz reliant le digesteur anaérobie amovible à l'unité centrale de stockage en biogaz et l'unité centrale de stockage du digestat liquide et de digestion complémentaire par le biais du réseau d'évacuation de biogaz,
- en deuxième lieu, dans sa partie supérieure, du circuit d'évacuation en digestat liquide reliant le digesteur anaérobie amovible à l'unité centrale de stockage du digestat liquide et de digestion complémentaire par le biais du réseau d'évacuation de digestat liquide et du réseau de circulation en biogaz,
- en troisième lieu, dans sa partie basse, du circuit d'alimentation en digestat liquide reliant le digesteur anaérobie amovible à l'unité centrale de stockage du digestat liquide et de digestion complémentaire par le biais du réseau d'alimentation en digestat liquide.

Selon un mode de réalisation, l'étape de déconnexion du digesteur anaérobie amovible de l'unité centrale de stockage du digestat liquide et de digestion complémentaire est suivie par une étape de transport du digesteur anaérobie amovible rempli de la biomasse solide digérée.

Le digesteur anaérobie amovible est ainsi transporté depuis le lieu où se trouve le système selon la présente invention vers le lieu de déchargement de la biomasse solide digérée (ce lieu peut être, par exemple, une ferme agricole sur les terres de laquelle la biomasse solide digérée sera épandue comme fertilisant agricole, ou bien encore un lieu de traitement de déchets si la biomasse solide digérée est impropre à un épandage agricole, etc.).

Selon un mode de réalisation, le procédé comporte une étape de d'évacuation de la biomasse solide digérée contenue dans le digesteur anaérobie amovible. De préférence, cette évacuation est réalisée par reversement du digesteur anaérobie amovible, et versement de la biomasse solide digérée à travers la zone latérale ouvrante du digesteur anaérobie amovible, La biomasse solide digérée peut être versée par exemple dans une fosse prévue à cet effet, ou encore sur une aire plane, éventuellement munie d'un dispositif de récupération des jus.

Selon un mode de réalisation alternatif de l'étape d'évacuation de la biomasse solide digérée contenue dans le digesteur anaérobie amovible, le procédé comporte une étape d'ouverture du toit sur la zone d'admission de la biomasse solide préalablement à l'étape d'évacuation du digestat solide. L'étape d'évacuation de la biomasse solide digérée résiduelle peut alors être effectuée par le biais du moyen de préhension apte à saisir la biomasse solide digérée résiduelle.

Une telle étape présente l'avantage de pouvoir se dérouler en toute sécurité après la vidange aérobie initiale du digestat liquide contenu dans le digesteur anaérobie amovible, et son remplacement par de l'air, et donc en absence de biogaz.

La présente invention concerne également un digesteur anaérobie de biomasse solide amovible, comportant :
- une cuve apte à contenir de la biomasse solide, comportant au moins une zone d'admission de la biomasse solide, située sur la partie supérieure de la cuve, sur laquelle un toit est apte à s'ouvrir et à se refermer, et comportant en outre au moins une zone d'évacuation de la biomasse solide digérée résiduelle, certaines desdites zones d'admission et d'évacuation de biomasse solide pouvant être communes,
- au moins une ouverture d'alimentation en digestat liquide, située sur la partie inférieure du digesteur anaérobie, comportant au moins un moyen configuré pour permettre d'être reliée à au moins un circuit d'alimentation de digestat liquide, apte à permettre l'introduction de digestat liquide dans le digesteur,
- au moins une ouverture d'évacuation de digestat liquide, située sur la partie supérieure du digesteur anaérobie, comportant au moins un moyen configuré pour permettre d'être reliée à au moins un circuit d'évacuation de digestat liquide, apte à permettre l'évacuation de digestat liquide dudit digesteur,
- au moins une ouverture d'évacuation de biogaz, située sur la partie supérieure du digesteur anaérobie, comportant au moins un moyen configuré pour permettre d'être reliée à au moins un circuit d'évacuation de biogaz, apte à permettre l'évacuation de biogaz depuis ledit digesteur, et
- au moins une ouverture de vidange de digestat liquide, située sur la partie inférieure du digesteur anaérobie, apte(s) permettre la vidange de digestat liquide dudit digesteur de biomasse solide, et
- au moins une ouverture d'introduction et/ou d'évacuation d'air, située sur la partie supérieure du digesteur anaérobie, apte(s) permettre l'introduction et/ou l'évacuation d'air dans ledit digesteur de biomasse solide,
   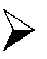 certaines ouvertures parmi lesdites ouvertures pouvant être communes, à l'exception des ouvertures d'alimentation et d'évacuation de digestat liquide qui sont distinctes ;
   ledit digesteur étant apte à être connecté et déconnecté d'un réseau d'alimentation en digestat liquide, d'un réseau d'évacuation de digestat liquide et d'un réseau d'évacuation de biogaz.

En particulier, le digesteur anaérobie de biomasse solide amovible est apte à être connecté et déconnecté d'un réseau d'alimentation en digestat liquide, d'un réseau d'évacuation de digestat liquide et d'un réseau d'évacuation de biogaz permettant de le relier à une unité de stockage de digestat liquide et de digestion complémentaire et une unité centrale de stockage de biogaz.

De préférence, le digesteur anaérobie de biomasse solide amovible est apte à être connecté et déconnecté d'un circuit d'alimentation en digestat liquide, d'un circuit d'évacuation de digestat liquide et d'un circuit d'évacuation de biogaz ; lesdits circuits étant reliés à une unité de stockage de digestat liquide et de digestion complémentaire et une unité centrale de stockage de biogaz.

Ainsi le digesteur anaérobie de biomasse solide amovible est apte à être connecté et déconnecté de tout type d'unité de stockage de digestat liquide et de digestion complémentaire et une unité centrale de stockage de biogaz.

De préférence, de biomasse solide amovible est apte à être connecté et déconnecté d'une unité de stockage de digestat liquide et de digestion complémentaire et une unité centrale de stockage de biogaz tels que définies ci-avant.

De préférence, le digesteur anaérobie amovible est muni de deux éléments mobiles situées sur une même extrémité longitudinale de la cuve et fixées sur une face inférieure de ladite cuve.

Les éléments mobiles ont pour fonction d'assurer la mobilité du digesteur anaérobie, notamment vers le lieu de chargement en biomasse solide, le lieu d'implantation du système de production de biogaz et/ou le lieu de déchargement de biomasse solide digérée résiduelle.

De préférence, le digesteur anaérobie amovible est muni de deux roues situées sur une même extrémité longitudinale de la cuve et fixées sur une face inférieure de la cuve.

D'autres avantages et caractéristiques de l'invention apparaîtront à l'examen de la description détaillée d'un mode de réalisation de l'invention nullement limitatif, et des dessins annexés, sur lesquels :

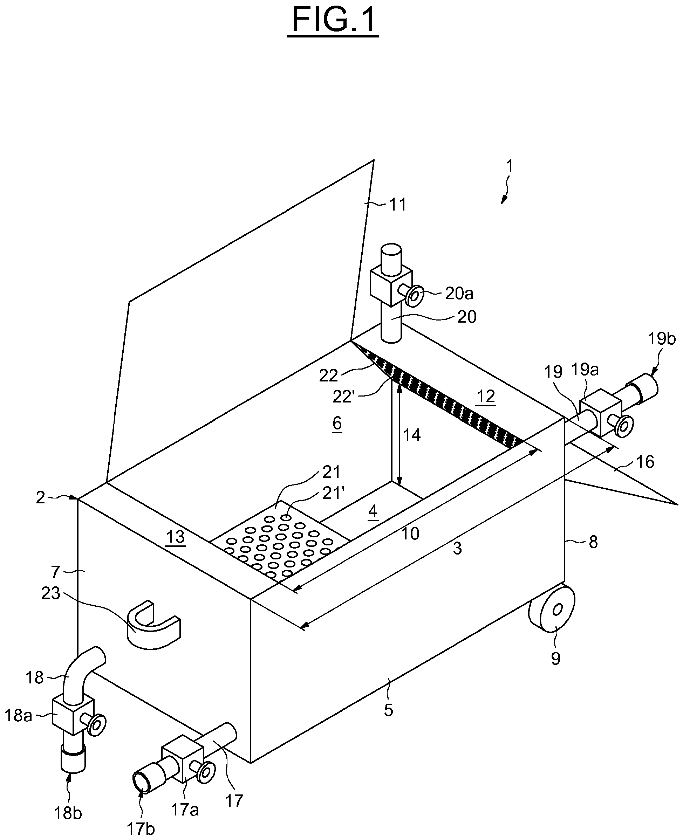

- la figure 1 représente une vue en perspective d'un digesteur anaérobie amovible de biomasse solide selon l'invention,

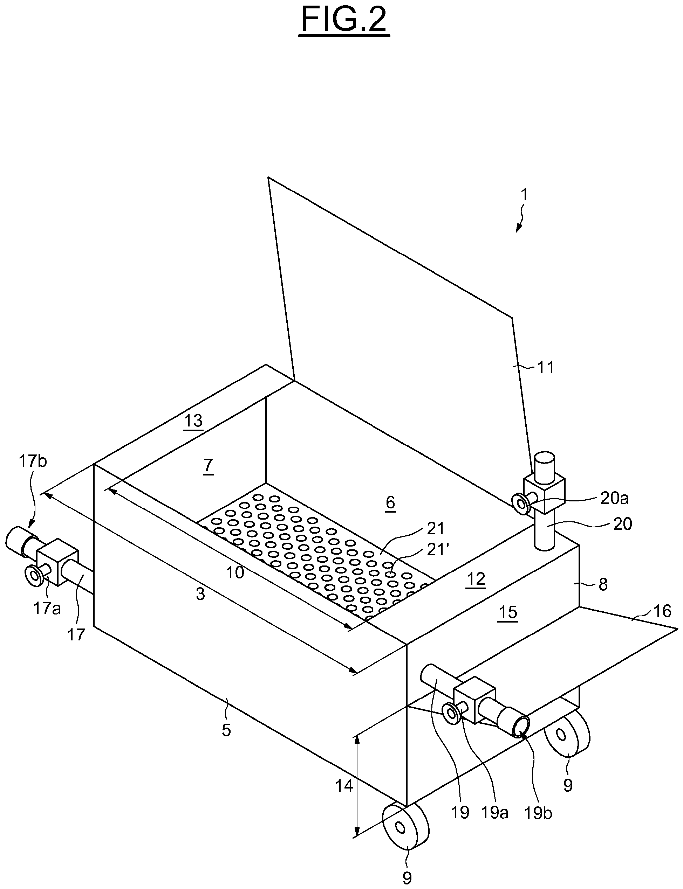

- la figure 2 représente une vue en perspective isométrique du digesteur anaérobie amovible de biomasse solide selon l'invention,

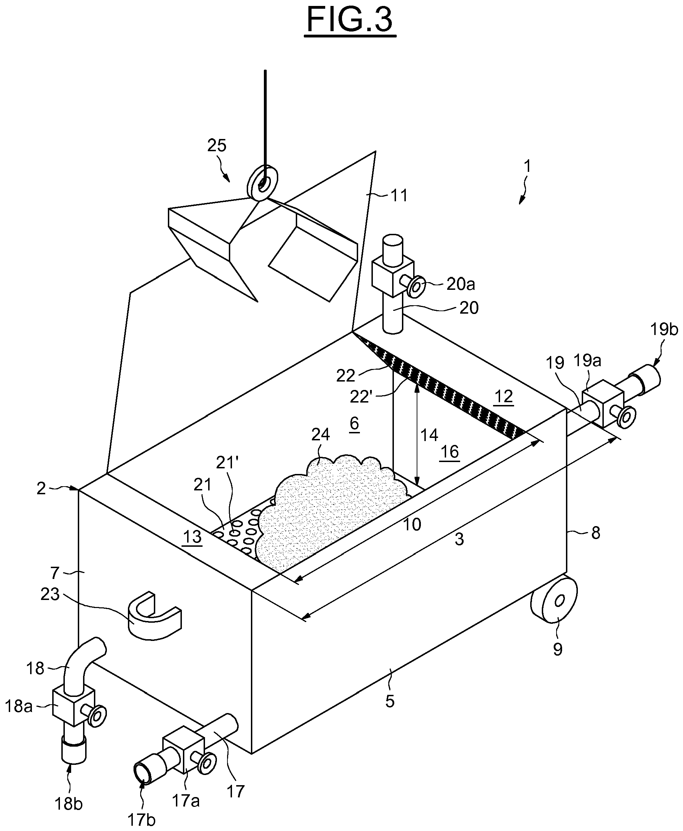

- la figure 3 représente une vue en perspective d'un digesteur anaérobie amovible selon l'invention lors de l'étape de chargement en biomasse solide,

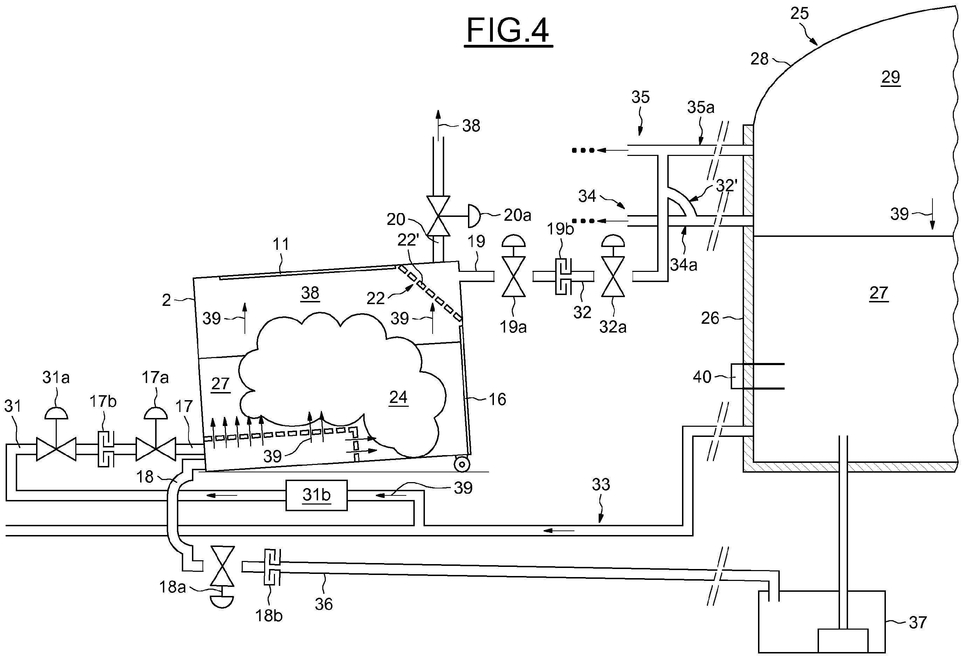

- les figures 4 à 9 représentent schématiquement des vues en coupe d'un digesteur anaérobie amovible de biomasse solide, au cours des différentes étapes du procédé de production de biogaz, directement alimenté (liaison en parallèle) en digestat liquide en provenance d'une unité centrale de stockage de digestat liquide et de digestion complémentaire et d'une unité centrale de stockage de biogaz constitués d'une seule et même unité,

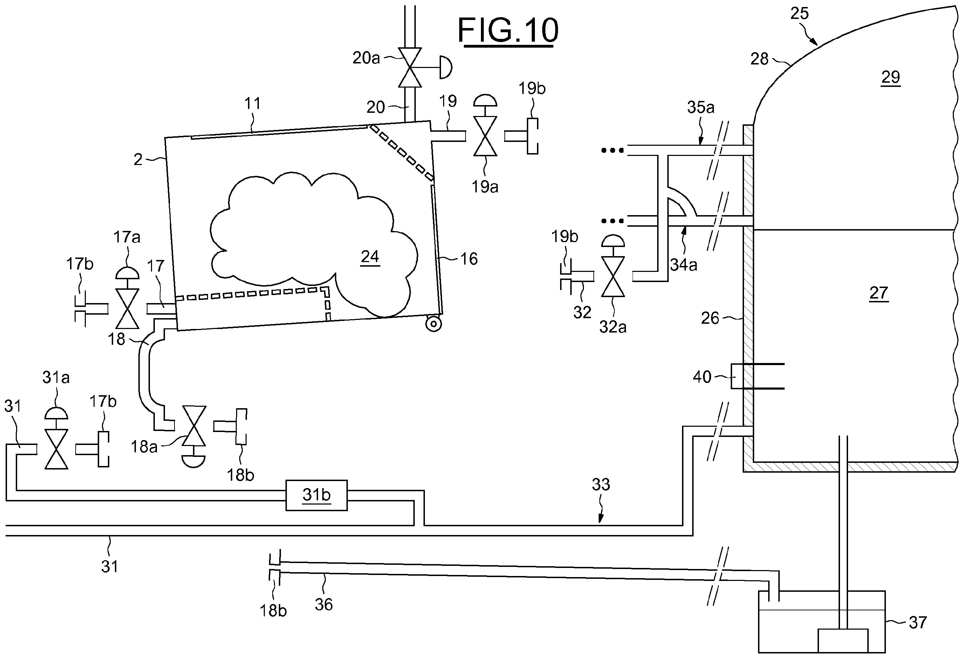

- la figure 10 représente schématiquement une vue en coupe d'un digesteur anaérobie amovible de biomasse solide déconnecté du système de production de biogaz une fois la digestion terminée (après vidange aérobie),

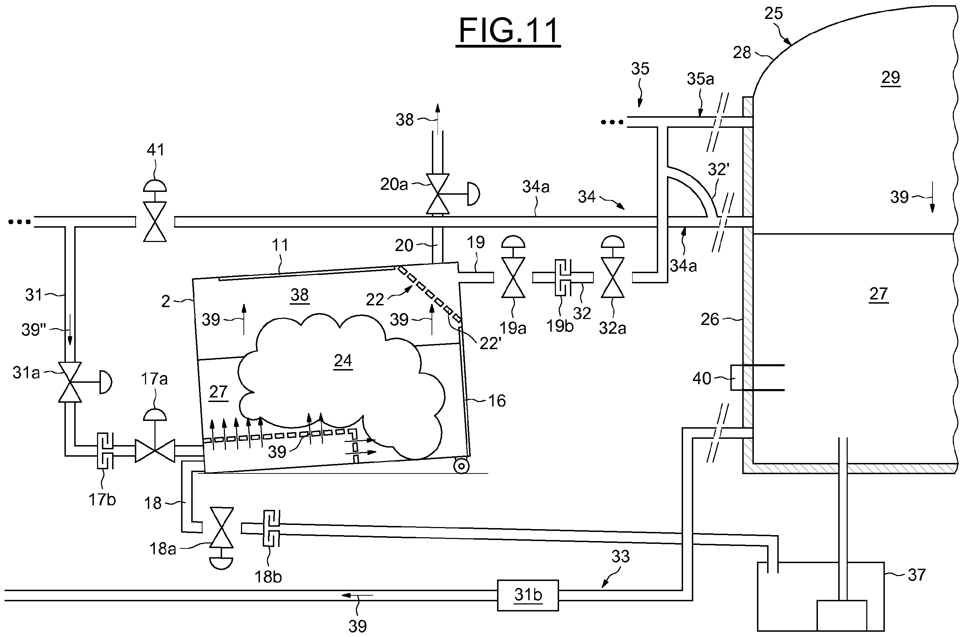

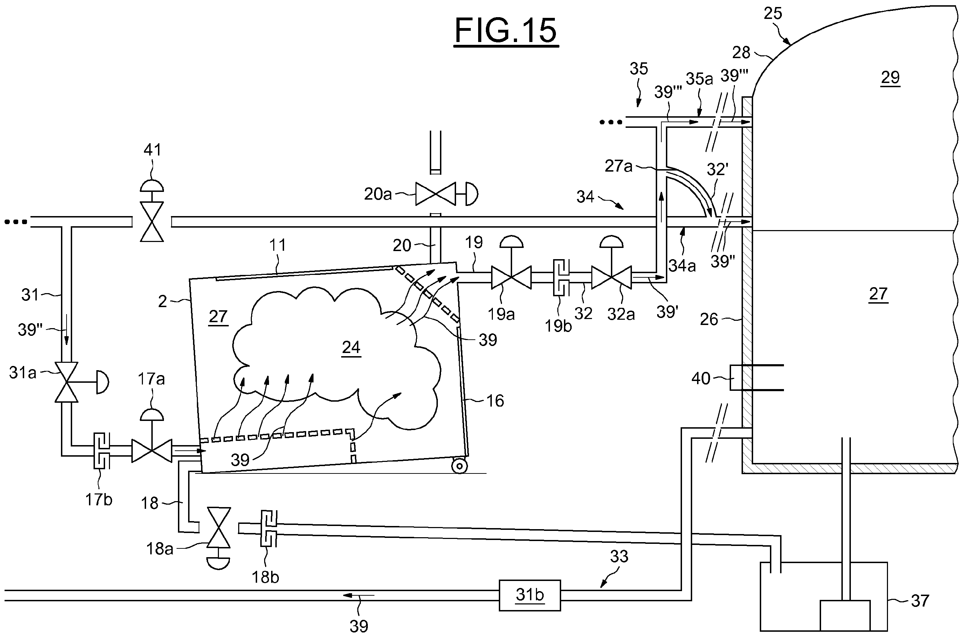

- les figures 11 à 16 représentent schématiquement des vues en coupe d'un digesteur anaérobie amovible de biomasse solide, au cours des différentes étapes du procédé de production de biogaz, indirectement alimenté en digestat liquide en provenance d'un autre digesteur anaérobie amovible (liaison en série),

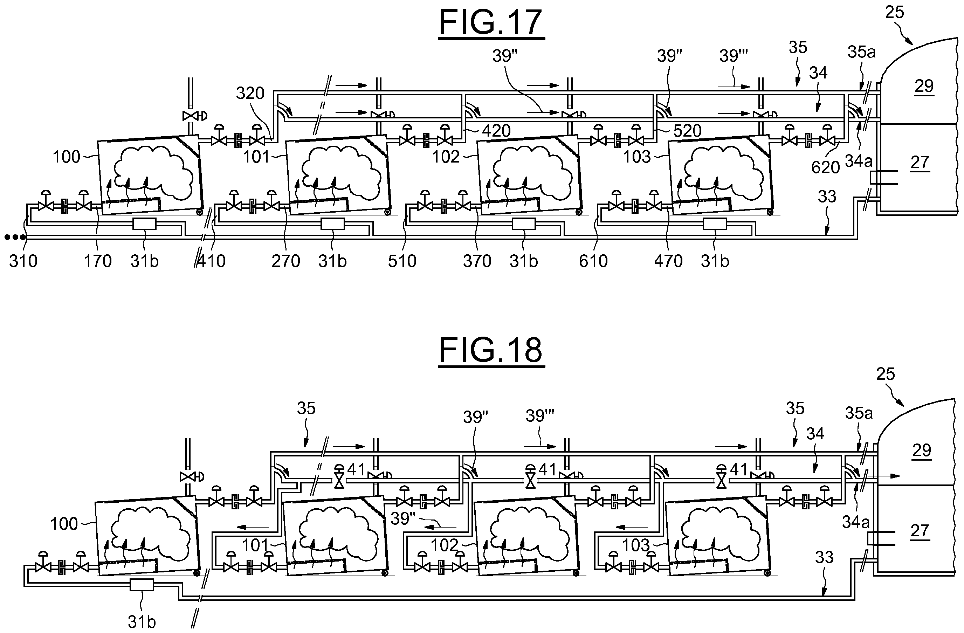

- la figure 17 représente schématiquement une vue en coupe d'une pluralité de digesteurs anaérobies amovibles de biomasse solide alimentés directement (liaison parallèle) en digestat liquide par une unité centrale de stockage de digestat liquide et de digestion complémentaire et d'une unité centrale de stockage de biogaz constitués d'une seule et même unité
- la figure 18 représente schématiquement une vue en coupe d'une pluralité de digesteurs anaérobies amovibles de biomasse solide alimentés indirectement (liaison en série) en digestat liquide par une unité centrale de stockage de digestat liquide et de digestion complémentaire et d'une unité centrale de stockage de biogaz constitués d'une seule et même unité,

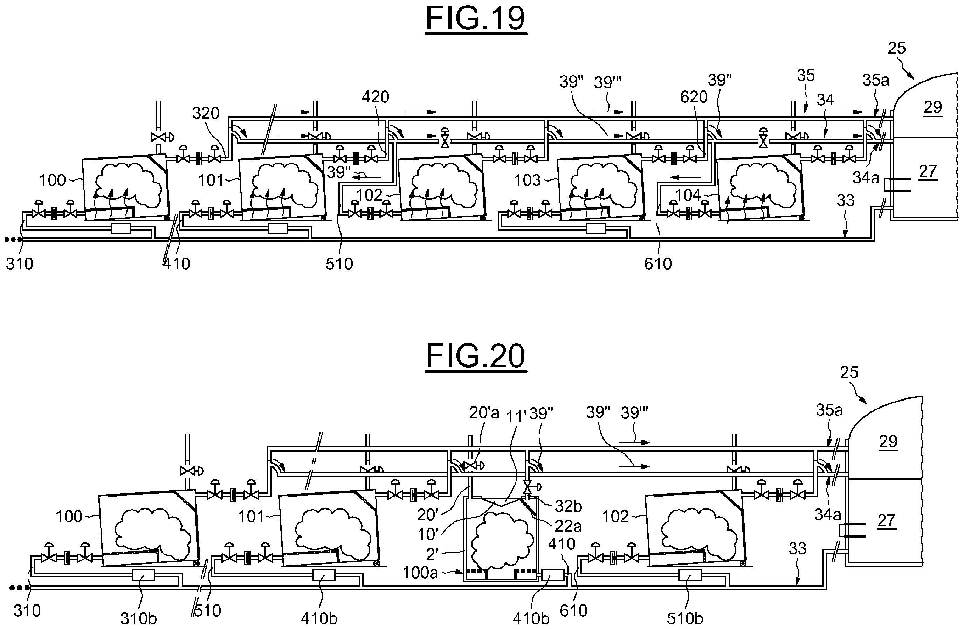

- la figure 19 représente schématiquement une vue en coupe d'une pluralité de digesteurs anaérobies amovibles de biomasse solide alimentés à la fois directement ou indirectement (liaison en série et en parallèle) en digestat liquide par une unité centrale de stockage de digestat liquide et de digestion complémentaire et d'une unité centrale de stockage de biogaz constitués d'une seule et même unité,
- la figure 20 illustre schématiquement une vue en coupe d'une pluralité de digesteurs de biomasse solide composés de digesteurs anaérobies amovibles et de digesteurs anaérobies immobiles alimentés directement en digestat liquide (liaison en parallèle) par une unité centrale de stockage de digestat liquide et de digestion complémentaire et d'une unité centrale de stockage de biogaz constitués d'une seule et même unité,

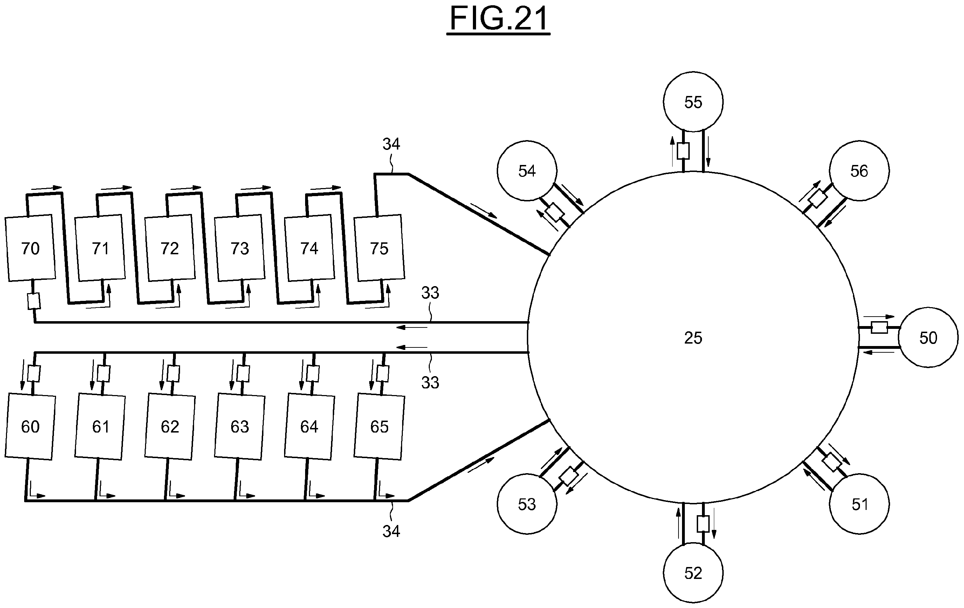

- la figure 21 illustre une vue de dessus d'un système de production de biogaz comprenant une pluralité de digesteurs anaérobies amovibles et non amovibles, un réseau de circulation de digestat liquide et un réseau de circulation de biogaz.

La figure 1 représente une vue en perspective d'un digesteur anaérobie amovible 1 de biomasse solide comprenant une cuve 2, ayant une forme essentiellement parallélépipédique, dotée d'une face supérieure 3, d'une face inférieure 4, qui est parallèle et opposée à la face supérieure 3, de deux faces latérales 5 et 6, qui sont parallèles et opposées l'une de l'autre, et d'une première face frontale 7 et d'une deuxième face frontale 8 parallèle et opposée à la première.

Le digesteur anaérobie amovible 1 est muni de deux roues, une seule roue 9 ayant été illustrée sur la figure 1, qui sont situées sur une même extrémité longitudinale de la cuve 2 et fixées sous la face inférieure 4. En particulier, les deux roues sont situées aux deux extrémités latérales de l'arête entre la face inférieure 4 et la deuxième face frontale 8.

La face supérieure 3 comprend un orifice d'entrée 10, sur laquelle un toit 11 est apte à s'ouvrir et à se refermer, une paroi rectangulaire 12 adjacente à la deuxième face frontale 8 et une paroi rectangulaire 13 adjacente à la première face frontale 7. Le toit 11 est apte à s'ouvrir et se refermer sur l'orifice d'entrée 10 de manière à permettre l'introduction de la biomasse solide (non représentée sur la figure 1) dans le digesteur anaérobie amovible 1 lors d'une étape de chargement et éventuellement l'évacuation de la biomasse solide digérée résiduelle lors d'une étape de déchargement. Le toit 11 se referme de manière étanche sur l'orifice d'entrée 10 de manière à permettre qu'il n'y ait pas de fuites de biogaz ou de digestat liquide pendant les étapes de digestion de la biomasse solide.

Sur la figure 1, l'orifice d'entrée 10 présente une surface inférieure à la surface totale de la face supérieure 3. En particulier, l'orifice d'entrée 10 se trouve comprise entre les parois rectangulaires 12 et 13. Cependant, en variante, l'orifice d'entrée 10 peut également présenter une surface égale à la face supérieure 3.

Une tuyauterie 17 est fixée, de préférence, sur la partie inférieure du digesteur anaérobie 1, notamment sur la partie inférieure de la face frontale 7, et est destinée à alimenter la cuve 2 en digestat liquide lors d'une étape de remplissage en digestat liquide. En particulier, la tuyauterie 17 constitue une ouverture d'alimentation en digestat liquide.

La tuyauterie 17 comporte en outre une vanne 17a, destinée à obturer ou permettre le passage du digestat liquide entre l'extérieur et l'intérieur du digesteur 1, et un moyen 17b configuré pour relier la tuyauterie 17 à un circuit d'alimentation en digestat liquide. De préférence, le moyen 17b est un raccord pompier circulaire.

Une tuyauterie 18 est fixée, de préférence, sur la partie inférieure de la face frontale 7, et est destinée à vidanger la cuve 2 lors des étapes de vidange anaérobie et aérobie du digestat liquide. En particulier, la tuyauterie 18 constitue une ouverture de vidange de digestat liquide.

La tuyauterie 18 comporte en outre une vanne 18a, destinée à obturer ou permettre le passage du digestat liquide entre l'intérieur et l'extérieur du digesteur 1, et un moyen 18b configuré pour relier la tuyauterie 18 à un circuit de vidange de digestat liquide.

De préférence, le moyen 18b est également un raccord pompier circulaire.

La cuve 2 comprend en outre une tuyauterie 19 fixée sur la partie supérieure du digesteur anaérobie amovible 1 et est destinée à évacuer à la fois du biogaz et du digestat liquide, au cours d'une étape de digestion de la biomasse solide.

La tuyauterie 19 comporte également une vanne 19a, destinée à obturer ou permettre le passage du digestat liquide et du biogaz entre l'extérieur et l'intérieur du digesteur 1, et un moyen 19b configuré pour relier la tuyauterie 19 à un circuit d'évacuation de biogaz et de digestat liquide. De préférence, le moyen 19b est un raccord pompier circulaire.

Conformément au mode de réalisation illustré sur la figure 1, la tuyauterie 19 constitue une ouverture commune à l'évacuation du biogaz et l'évacuation du digestat liquide.

En variante, l'ouverture d'évacuation du biogaz peut être distincte de l'ouverture d'évacuation de digestat liquide. Conformément à cette variante, l'ouverture d'évacuation du biogaz est située sur la partie supérieure du digesteur anaérobie 1 et l'ouverture d'évacuation de digestat liquide peut être située à n'importe quel endroit sur le digesteur anaérobie 1.

Par ailleurs, de manière générale, l'ouverture d'alimentation en digestat liquide, l'ouverture d'évacuation de digestat liquide, l'ouverture d'évacuation de biogaz, l'ouverture de vidange de digestat liquide et l'ouverture d'introduction et/ou d'évacuation d'air peuvent être communes à l'exception des ouvertures d'alimentation et d'évacuation de digestat liquide.

Ainsi la tuyauterie 17 est distincte de la tuyauterie 19 conformément à la figure 1.

Par ailleurs, d'autres moyens d'obstruction que les vannes 17a, 18a, 19a et 20a peuvent être mis en œuvre afin d'obturer ou permettre le passage des fluides.

La cuve 2 comprend également une tuyauterie 20 fixée sur la partie supérieure du digesteur anaérobie amovible 1, notamment sur la paroi rectangulaire 12. En particulier, la tuyauterie 20 est en saillie vers l'extérieur par rapport à la surface de la paroi rectangulaire 12.

La tuyauterie 20 comporte également une vanne 20a, destinée à permettre le passage de l'air de l'intérieur vers l'extérieur et inversement, et constitue une ouverture d'introduction et/ou d'évacuation d'air.

Le digesteur anaérobie amovible 1 comprend également un élément de séparation perforé 21 situé en amont de la tuyauterie de vidange 18.

Conformément à la figure 1, l'élément de séparation perforé 21 est fixé sur la face inférieure 4 du digesteur 1 et occupe partiellement ou totalement le fond du digesteur 1.

L'élément de séparation perforé 21 est de préférence un plancher perforé mais peut également correspondre à tout autre type d'élément perforé situé en amont de la tuyauterie de vidange 18.

L'élément de séparation perforé 21 comprend une pluralité d'interstices 21' afin de permettre la circulation du digestat liquide lors des étapes de vidanges anaérobies et aérobies du digesteur 1 tout en retenant la biomasse solide dans la cuve 2.

La cuve 2 comporte également un élément de séparation perforé 22 qui est situé en amont de la tuyauterie 19.

L'élément de séparation perforé 22 est fixé, soit sur la face supérieure 3, soit sur la deuxième face frontale 8, et est de préférence fixée à la fois sur la face supérieure 3 et la deuxième face frontale 8. L'élément 22 s'étend sur un plan sécant ou oblique par rapport à la face supérieure 3, soit par rapport à la deuxième face frontale 8, soit même par rapport à la face supérieure 3 et la deuxième face frontale 8.

L'élément de séparation perforé 22 est de préférence une plaque perforée mais peut également correspondre à tout autre type d'élément perforé situé en amont de la tuyauterie 19.

L'élément de séparation perforé 22 comprend une pluralité d'interstices 22' afin de permettre l'évacuation du digestat liquide au cours de l'étape de digestion de la biomasse solide tout en retenant la biomasse solide dans la cuve 2.

La première face frontale 7 comprend également une poignée 23 destinée à pouvoir saisir le digesteur anaérobie amovible et à permettre le transport du digesteur anaérobie amovible 1 vers le lieu de chargement en biomasse solide, vers le lieu d'une installation de méthanisation et/ou vers le lieu de déchargement en biomasse solide digérée résiduelle.

Sur la figure 1, la poignée 23 présente une forme recourbée en U mais peut présenter tout autre type de forme de manière à faciliter l'accrochage du digesteur anaérobie amovible 1 à un camion ou tout autre type de moyen de transport.

La poignée 23 est fixée sur la surface de la première face frontale 7.

De préférence, le digesteur anaérobie amovible 1 peut être un container de camion, équipé d'un toit ouvrant capable d'être fermé de façon étanche et d'un côté ouvrant, situé au niveau de l'une ses faces, capable d'être fermé de façon étanche.

De préférence, le digesteur anaérobie amovible 1 est un container conçu pour être chargé sur un camion apte à transporter des containers, afin de pouvoir permettre le transport du digesteur anaérobie amovible 1 vers le lieu de chargement en biomasse solide, vers le lieu d'une installation de méthanisation et/ou vers le lieu de déchargement en biomasse solide digérée résiduelle.

La figure 2 représente une vue en perspective isométrique du digesteur anaérobie amovible 1.

Conformément à cette figure, la deuxième face frontale 8 comporte également un orifice d'entrée 14, sur lequel une trappe 16 est apte à s'ouvrir et se refermer, ainsi qu'une paroi rectangulaire 15 adjacente à la face supérieure 3. La trappe 16 est apte à s'ouvrir et se refermer sur l'orifice d'entrée 14 afin de permettre l'évacuation de la biomasse solide digérée résiduelle lors de l'étape de déchargement par renversement du digesteur anaérobie amovible 1. La trappe 16 se referme de manière étanche sur l'orifice d'entrée 14 de manière à permettre qu'il n'y pas de fuites de digestat liquide lorsque le digesteur amovible est rempli de digestat liquide, notamment pendant les étapes de digestion de la biomasse solide.

En variante, l'une des faces latérales 5 et 6 et/ou la première face frontale 7 peuvent comporter respectivement un orifice de sortie de manière à permettre l'évacuation de la biomasse solide digérée résiduelle lors de l'étape de déchargement par renversement du digesteur anaérobie amovible 1.

Ainsi la cuve 2 du digesteur anaérobie amovible 1 constitue une enveloppe étanche. De manière plus générale, la cuve 2 peut présenter une forme oblongue.

La figure 3 représente une vue en perspective du digesteur anaérobie amovible 1 contenant de la biomasse solide 24 qui a été introduite à travers l'orifice d'entrée 10 lorsque le toit 11 de la cuve 2 est en position relevée c'est-à-dire en position ouverte. Une fois chargée dans le digesteur 1, la biomasse solide 24 repose à la fois sur l'élément de séparation perforé inférieur 21 et la face inférieure 4 de la cuve 2. Au cours de cette opération de chargement, la trappe 16 est refermée sur l'orifice d'entrée 14.

Le chargement en biomasse solide 24 peut être mis en œuvre par d'un godet 25 qui peut être positionné au-dessus de l'orifice d'entrée 10 du digesteur anaérobie amovible 1 et être reliée à un camion chargeur à godet (non illustré sur la figure 3).

De manière alternative, le chargement en biomasse solide 24 peut être effectué à l'aide d'un tracteur agricole à godet, ou de tout autre moyen de chargement. En particulier, le chargement peut aussi se faire au moyen d'une installation de chargement fixe, par exemple un grappin, située sur le lieu de chargement.

La benne 25 permet donc de saisir la biomasse solide 24 stockée à l'extérieur du digesteur 1 afin de la déposer à l'intérieur du digesteur 1. En d'autres termes, la benne 25 permet d'alimenter, par le haut, la cuve 2 du digesteur 1, en biomasse solide 24, à travers l'orifice d'entrée 10 qui est situé au niveau de la face supérieure 3 du digesteur anaérobie mobile 1. Ainsi l'orifice d'entrée 10 constitue une zone d'admission de la biomasse solide.

La benne 25 permet de saisir la biomasse solide 24 contenant éventuellement des éléments indésirables, tels que des cailloux, du gravier, du sable, des barbelés, des ficelles, des pièces métalliques provenant d'outils agricoles ou encore des sacs plastiques, sans avoir besoin d'effectuer d'opération de tri préalable, ni de broyage, ni de dilution de la biomasse solide dans un liquide.

En fonction de son taux de matière sèche, une fraction liquide de la biomasse peut éventuellement couler du grappin au moment de l'étape d'alimentation du réacteur anaérobie amovible 1.

En variante, la benne 25 peut également servir à ouvrir le toit amovible 11 pour le déposer à côté du réacteur anaérobie mobile 1 ou plus loin, et à le refermer par la suite. Dans ce cas, le toit 11 est apte à se décrocher de la partie supérieure 3 de la cuve 2.

La biomasse solide 24 se trouve alors à l'intérieur du réacteur anaérobie amovible 1 et repose sur le fond et/ou tout ou partie de l'élément de séparation perforé inférieur 21.

Les interstices 21' de l'élément de séparation perforé 21 présentent une taille suffisamment faible pour éviter que la biomasse ne puisse pénétrer dans la tuyauterie 18 lors des étapes de vidange anaérobies et aérobies du digesteur anaérobie amovible 1.

L'étape d'alimentation est arrêtée à partir du moment où la quantité en biomasse solide introduite est jugée suffisante pour démarrer la méthanisation.

De manière plus générale, le chargement en biomasse solide 24 peut être effectué mécaniquement, par le biais d'un moyen de préhension tel qu'une benne ou un grappin comportant une pluralité de crochets, ou encore manuellement.

Une fois le chargement en biomasse solide 24 terminé, le toit 11 est alors refermé de manière étanche sur l'orifice d'entrée 10. La cuve 2 est alors transportée, de préférence par camion ou tracteur, vers le lieu où se trouve le système de production de biogaz.

Ainsi l'étape de chargement en biomasse solide 24 s'effectue de préférence dans un lieu distinct du lieu où se situe le système de production de biogaz.

La biomasse solide 24 peut être en particulier fibreuse et/ou hétérogène.

Les figures 4 à 9 décrivent les différentes étapes successives d'un procédé de production de biogaz qui se déroulent au sein du digesteur anaérobie amovible 1 dans le cas où ce dernier est directement (liaison en parallèle) alimenté en digestat liquide en provenance d'une unité centrale de stockage de digestat liquide et de digestion complémentaire et d'une unité de stockage de biogaz.

Les figures 4 à 9 se concentrent donc seulement sur une partie du système de production de biogaz, c'est-à-dire sur un digesteur anaérobie amovible qui est alimenté directement en digestat liquide en provenance d'une unité centrale de stockage de digestat liquide et de digestion complémentaire et d'une unité de stockage de biogaz.

Sur la figure 4 est représentée de manière schématique, une unité centrale de stockage de digestat liquide et de digestion complémentaire et une unité de stockage de biogaz constituant une seule et même unité, dénommé unité 25.

En d'autres termes, l'unité 25 constitue une seule et même unité centrale de stockage de digestat liquide, de biogaz et de digestion complémentaire.

L'unité 25 comprend une cuve 26, ayant une forme essentiellement cylindrique, remplie de digestat liquide 27, qui est surmontée d'une membrane étanche 28 constituant un espace de stockage de gaz, lequel est rempli de biogaz 29. La membrane 28 occupe donc la partie en hauteur de l'unité 25 formant ainsi un dôme contenant un ciel gazeux. La cuve 26 correspond à une réserve de digestat liquide 27.

En variante, la cuve 26 peut présenter une forme cubique ou parallélépipédique.

La cuve 26 peut être fabriquée à partir d'acier, de béton, de matière plastique ou de tout autre matériau tandis que la membrane étanche 28 peut être réalisée en polymère, notamment en polyéthylène, polypropylène ou chlorure de polyvinyle.

En variante, la membrane étanche 28 peut être surmontée par au moins une membrane supplémentaire de manière à ce que de l'air soit pris en sandwich entre la membrane supplémentaire et la membrane étanche 28.

Le digesteur anaérobie amovible 1 est relié à l'unité 25, d'un part, par le biais du moyen de raccordement 17b configuré pour connecter la tuyauterie 17 à un circuit d'alimentation 31 en digestat liquide 27, et d'autre part, par le biais du moyen de raccordement 19b configuré pour connecter la tuyauterie 19 au circuit d'évacuation 32 du biogaz 29 et du digestat liquide 27.

Le circuit d'alimentation 31 fait ainsi partie intégrante d'un réseau d'alimentation 33 en digestat liquide 27 qui relie l'unité 25 au digesteur anaérobie amovible 1.

Le réseau d'alimentation 33 en digestat liquide 27 est composé d'une pluralité de circuits d'alimentation en digestat liquide 27, similaires ou identiques au circuit d'alimentation 31, et permet de relier l'unité 25 au digesteur anaérobie amovible 1 mais également à d'autres digesteurs anaérobies de biomasse solide (représentés sur les figures 17 à 20). Ainsi le circuit d'alimentation 31 constitue l'un des circuits d'alimentation appartenant au réseau d'alimentation 33.

Le circuit d'évacuation 32 en biogaz 29 et digestat liquide 27 est un circuit appartenant à un réseau d'évacuation 34 de digestat liquide 27 et un réseau d'évacuation 35 de biogaz 29.

Le circuit d'évacuation 32 est donc commun au réseau d'évacuation 34 de digestat liquide 27 et au réseau d'évacuation 35 de biogaz 29.

Le circuit d'évacuation 32 est ainsi relié à l'unité 25, notamment à l'espace de stockage en biogaz 29 et à la cuve 26 remplie en digestat liquide 27, par le biais du réseau d'évacuation 34 de digestat liquide 27 et du réseau d'évacuation 35 de biogaz 29.

Le réseau d'évacuation 34 de digestat liquide 27 comporte donc une pluralité de circuits d'évacuation en digestat liquide et le réseau d'évacuation 35 de biogaz 29 comporte une pluralité de circuits d'évacuation ; le circuit d'évacuation 32, sur le schéma, étant commun aux deux réseaux.

Le réseau d'évacuation 34 de digestat liquide 27 et le réseau d'évacuation 35 de biogaz 29 relient l'unité 25 au digesteur anaérobie amovible 1 mais également à d'autres digesteurs anaérobies de biomasse solide (représentés sur les figures 17 à 20).

Ainsi, conformément à la figure 4, le digesteur anaérobie amovible 1 est connecté à un réseau d'alimentation 33 de digestat liquide 27, un réseau d'évacuation 34 de digestat liquide 27 et un réseau d'évacuation 35 de biogaz 29.

Plus généralement, le réseau d'alimentation 33 en digestat liquide 27, le réseau d'évacuation 34 de digestat liquide 27 et le réseau d'évacuation 35 de biogaz 29 permettent de relier indirectement l'unité 25 à d'autres digesteurs anaérobies de biomasse solide qu'ils soient amovibles ou non amovibles (non représentés sur la figure 4).

La tuyauterie 18 est munie d'un moyen d'un raccordement 18b et connectée à un circuit de vidange 36 de digestat liquide qui est relié à une fosse de relevage 37.

De manière alternative, le circuit de vidange 36 peut être raccordé à un réseau de vidange. Dans ce cas, le réseau de vidange comprend plusieurs circuits de vidange visant à réaliser les vidanges anaérobies et aérobies du digesteur anaérobie 1.

Le circuit d'alimentation 31 est muni d'une vanne 31a située en amont du moyen de raccordement 17b. En d'autres termes, la vanne 31a et la vanne 17a se trouvent de part et d'autre du moyen de raccordement 17b.

Le circuit d'alimentation 31 est également muni d'une pompe 31b qui permet la circulation du digestat liquide dans le digesteur anaérobie mobile 1 provenant de l'unité 25 lorsque la vanne 31a et la vanne 17a sont dans une position ouverte.

De la même façon, le circuit d'évacuation 32 est muni d'une vanne 32a située en aval d'un moyen de raccordement 19b. Autrement dit, la vanne 32a et la vanne 19a se trouvent de part et d'autre du moyen de raccordement 17b.

Conformément à la figure 4, le circuit d'évacuation 32 se divise en un circuit d'évacuation 34a de digestat liquide 27 et un circuit d'évacuation 35a de biogaz 29.

Le circuit 34a permet d'évacuer du digestat liquide vers la cuve 26, remplie de digestat liquide 27, de l'unité 25 et le circuit d'évacuation 35a permet d'évacuer du biogaz vers l'espace de stockage rempli de biogaz 29 de l'unité 25.

Le circuit d'évacuation 32 de digestat liquide 27 et de biogaz 29 comporte deux portions, à savoir une portion horizontale et une portion verticale orientée vers le haut. En particulier, les deux portions sont deux segments, à savoir un segment horizontal et un segment vertical sensiblement perpendiculaires entre eux ; le segment vertical étant orienté vers le haut.

D'une part, le segment vertical du circuit d'évacuation 32 est relié au circuit 34a par le biais d'une ramification 32', ayant de préférence la forme d'un coude arrondi, qui est piquée à la fois sur le segment vertical du circuit 32 et le circuit 34a d'évacuation en digestat liquide 27. En particulier, la ramification 32' présente la forme d'un coude arrondi orienté vers le bas qui fait la jonction entre le segment vertical du circuit 32 et le circuit 34a d'évacuation de digestat liquide 27.

Le haut de la ramification 32', qui correspond au point de séparation 27a du digestat liquide 27 et du biogaz 29 lors de des phases de digestion anaérobies, est situé à un niveau plus élevé que le niveau maximal de remplissage en digestat liquide 27 dans l'unité 25, de manière à ce que lorsque les vannes 19a et 32a sont ouvertes le digestat liquide 27 contenu dans l'unité centrale 25 ne puisse pas s'écouler gravitairement vers le digesteur amovible 1.

La ramification 32' peut présenter tout type de forme orientée vers le bas.

D'autre part, le segment vertical du circuit d'évacuation 32 est relié à son extrémité au circuit 35a d'évacuation en biogaz 29 par le biais d'une jonction à angle droit.

Le circuit 35a d'évacuation en biogaz 29 est positionné au-dessus du circuit 34a de digestat liquide 27 et est de préférence sensiblement parallèle à celui-ci.

Ainsi le circuit d'évacuation 34a de digestat liquide 27 et le circuit d'évacuation 35a en biogaz 29 sont de préférence parallèles à la partie horizontale du circuit 32 et sont perpendiculaires à la partie verticale, orientée vers le haut, du circuit 32.

En d'autres termes, le circuit d'évacuation 32 comprend deux types embranchements situés l'un au-dessus de l'autre, à savoir une ramification 32' ayant la forme d'un coude arrondi et une jonction à angle droit, situé au-dessus de la ramification 32'. Les deux embranchements donnent naissance aux circuits 34a et 35a de manière à relier le digesteur anaérobie mobile 1 respectivement à l'espace de stockage en biogaz 29 et la cuve 26 remplie en digestat liquide 27 de l'unité 25.

En particulier, le circuit 34a est positionné de manière à permettre l'évacuation du digestat liquide directement dans la cuve 26 de l'unité 25, c'es-à-dire à proximité du niveau du digestat liquide 27 au sein de la cuve 26 de l'unité 25.

Parallèlement, le circuit 35a est positionné de manière à permettre l'évacuation du biogaz directement dans l'espace de stockage en biogaz de l'unité 25.

Le circuit d'évacuation 34a appartient au réseau d'évacuation 34 en digestat liquide 27 et le circuit d'évacuation 35b en biogaz 29 appartient au réseau d'évacuation 35 en biogaz 29.

Il en résulte que, d'une part, le réseau d'évacuation 34 de digestat liquide 27 comprend notamment le circuit d'évacuation 34a de digestat liquide 27 et le circuit d'évacuation 32 de digestat liquide 27 et de biogaz 29 et, d'autre part, le réseau d'évacuation 35 de biogaz 29 comprend notamment le circuit d'évacuation 35a de biogaz 29 et le circuit d'évacuation 32 de digestat liquide 27 et biogaz 29.

Autrement dit, le réseau d'évacuation 34 de digestat liquide 27 et le réseau d'évacuation 35 de biogaz 29 comprennent respectivement un circuit d'évacuation 34a en digestat liquide 27 et un circuit d'évacuation 35a en biogaz 29 qui sont distincts l'un de l'autre.

Le système de production tel que représenté comprend également un dispositif de chauffage 40 du digestat liquide.

Le dispositif est plus particulièrement positionné au niveau de la cuve 26 de l'unité 25 de manière à chauffer le digestat liquide 27 de telle sorte que le digestat liquide contenu dans l'unité centrale 25 ait une température constante généralement comprise entre 35 et 40°C pour un régime mésophile, et entre 45 et 55°C pour un régime thermophile.

La figure 4 illustre la cuve 2 du digesteur 1, après fermeture du toit amovible 11, qui est remplie avec du digestat liquide 27, acheminé par le biais d'une pompe d'alimentation 31b, en provenance de la cuve 26 de l'unité 25. En particulier, sur la figure 4, le digestat liquide 27 est acheminé au niveau de la partie inférieure du digesteur anaérobie mobile 1 sous l'élément perforé 21 et circule à travers les interstices 21'.

La cuve 2 du digesteur 1 peut également être remplie avec du digestat liquide 27 à travers une entrée située dans une zone située au-dessus de l'élément perforé 21.

Ainsi la cuve 2 peut être remplie avec du digestat liquide 27 qui ne passe pas à travers l'élément perforé 21.

De manière alternative, le digestat liquide 27 peut être aussi acheminé par une entrée située en tout autre endroit du digesteur anaérobie mobile 1.

Au cours de cette étape de remplissage du digesteur anaérobie amovible 1 par du digestat liquide 27, la vanne 20a est laissée en position ouverte de manière à ce que l'air, présent dans la cuve 2, soit progressivement évacué du digesteur 1 par l'intermédiaire de la tuyauterie 20. Sur la figure 4 est représenté schématiquement, l'air 38 qui s'échappe de la tuyauterie 20 pendant le remplissage en digestat liquide 27 du digesteur anaérobie amovible 1. Les vannes 19a et 32a sont en position fermée de manière à ce que l'air 38 ne circule pas vers l'unité 25.

De même, la vanne 18a est en position fermée de manière à ce que le digestat liquide 27 ne soit pas vidangé vers la fosse de relevage 37.

La biomasse solide 24, et éventuellement une partie de la biomasse solide digérée résiduelle issue d'un précédent cycle de méthanisation, se retrouve alors progressivement immergée dans le digestat liquide 27 à l'intérieur du digesteur anaérobie amovible 1.

Le niveau du digestat liquide 27 dans la cuve 26 de l'unité 25 diminue tandis qu'il augmente à l'intérieur du digesteur anaérobie amovible 1, chassant l'air se trouvant à l'intérieur du digesteur anaérobie amovible 1, qui s'échappe à travers la tuyauterie 20. L'étape de remplissage est considérée comme étant terminée lorsque le niveau du digestat liquide 27 aura atteint et légèrement dépassé vers le haut le niveau de la vanne 20a, et que la totalité de l'air contenu dans la cuve 2 aura été évacuée.

Sur la figure 4 est représenté, d'une part, le sens de circulation 39 du digestat liquide 27 à l'intérieur du digesteur anaérobie amovible 1 qui reflète l'augmentation de niveau dans la cuve 2 et, d'autre part, le sens de circulation 39 qui illustre la baisse de niveau du digestat liquide 27 à l'intérieur de l'unité 25.

De plus, le sens de circulation 39 du digestat liquide 27 est représenté à l'intérieur du circuit d'alimentation 31 ainsi qu'à travers les interstices 21'.

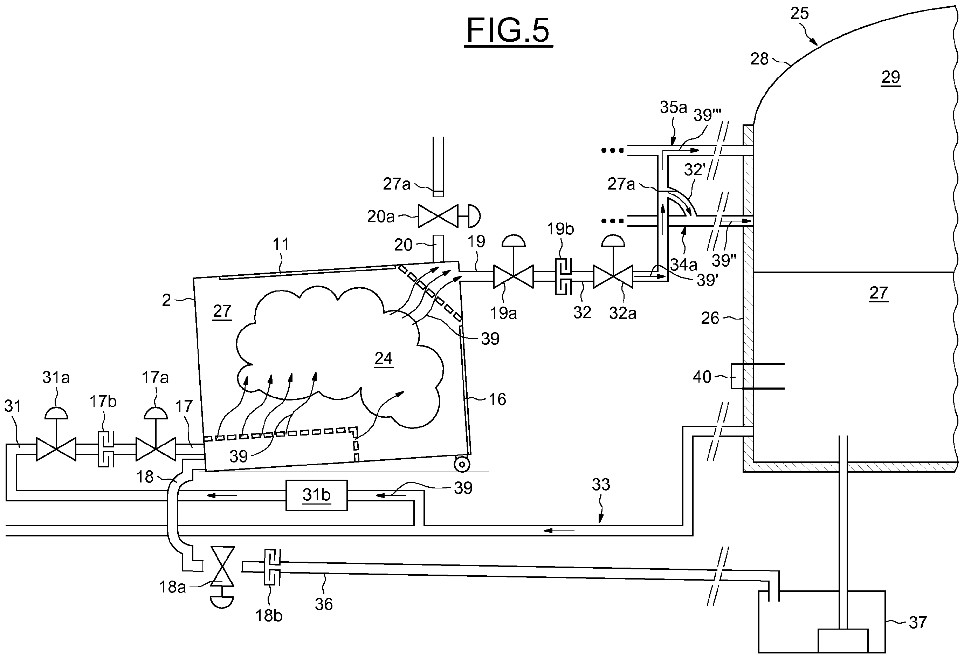

Conformément à la figure 5, une fois la cuve 2 remplie par le digestat liquide 27, la vanne 20a est fermée de manière à placer le digesteur 1 dans des conditions anaérobies, c'est-à-dire de manière à empêcher toute introduction d'air dans la cuve 2, tandis que les vannes 19a et 32a sont en position ouverte afin de recueillir le mélange biogaz et digestat liquide.

En particulier, lorsque le niveau du digestat liquide 27 a atteint le niveau 27a, qui se situe légèrement plus haut que le niveau de la vanne 20a, et que le digestat liquide 27 commence à déborder dans le circuit 34a, la vanne 20a est alors fermée, afin de placer le digesteur amovible 1 dans des conditions anaérobies.

La figure 5 représente donc l'étape de digestion de la biomasse solide 24 dans des conditions anaérobies. Au cours de cette étape, du biogaz se forme au sein du digesteur anaérobie amovible 1.

Une percolation du digestat liquide 27 à travers la biomasse 24 est mise en œuvre par le pompage du digestat liquide 27 vers la biomasse solide 24 en provenance de l'unité 25. La percolation se déroule à travers la biomasse solide 24 de manière à ce que la plus grande partie possible de la biomasse solide 24 soit mise en contact intime avec les bactéries contenues dans le digestat liquide 27 et puisse ainsi réagir au cours de la digestion.

Dans la figure 5 est représenté schématiquement le flux de digestat liquide 27 circulant, par percolation verticale ascendante, à travers l'ensemble de la biomasse solide 24.

Conformément à la figure 5, de préférence, l'alimentation en digestat liquide 27 est continue au cours de toute l'étape de digestion afin que le digestat liquide 27 circule à travers la biomasse solide 24, ce qui diminue le risque de survenue de réactions chimiques ou biologiques ponctuelles indésirables en certains endroits du tas de biomasse solide 24, tel que l'acidose par exemple, lesquelles pourraient altérer la qualité et l'efficacité de la flore bactérienne.

Cependant, l'alimentation en digestat liquide 27 peut être, dans certains cas, temporairement ou définitivement interrompue, en particulier vers la fin du cycle de méthanisation, moment où les réactions chimiques et biologiques sont moins virulentes.

Au cours de cette étape, un processus de diffusion du digestat liquide 27 dans la biomasse solide 24 se produit. Un tel processus de diffusion est favorisé, d'une part, par l'immersion de la biomasse solide 24 dans le digestat liquide 27 et, d'autre part, par la pression du digestat liquide 27 sur la biomasse solide 24 qui résulte de l'action de la pompe 31b.

Le biogaz 29 et le digestat liquide 27 sont extraits à travers les interstices 22' de l'élément de séparation perforé 22 tandis que la biomasse solide 24 est retenue par l'élément de séparation perforé 22 et reste immergée dans la cuve 2. Le mélange de biogaz 26 et de digestat liquide 27, circulant à travers l'élément de séparation perforé 22, est représenté schématiquement sur la figure 5.

Le biogaz 29 et le digestat liquide 27 sont acheminés ensemble dans la tuyauterie 19 et circulent ensuite dans le circuit d'évacuation 32 jusque dans la ramification 32' qui est piquée à la fois sur la partie verticale du circuit 32 et le circuit d'évacuation 34a.

Plus précisément, le biogaz 29 et le digestat liquide 27, issus de la tuyauterie 19, sont acheminés dans la partie horizontale et la partie verticale du circuit d'évacuation 32 jusque dans la ramification 32'.

Le sens de circulation 39' du biogaz 29 et du digestat liquide 27 a été représenté dans les parties horizontales et verticales du circuit d'évacuation 32.

Le biogaz 29 et le digestat liquide 27 circulent dans la partie verticale 32 pour être ensuite séparés au niveau de l'entrée de la ramification 32' ayant la forme d'un coude arrondi orienté vers le bas.

En particulier, lors de la circulation du biogaz 29 et du digestat liquide 27 dans la partie horizontale du circuit 32, le digestat liquide 27 va déborder à un niveau 27a au niveau de l'entrée de la ramification 32'.

Au moment où le digestat liquide 27 atteint le niveau 27a dans la partie verticale du circuit 32 alors celui-ci va circuler vers le bas dans la ramification 32' puis dans le circuit d'évacuation 34a tandis que le biogaz 29 continue à circuler vers le haut dans la partie verticale du circuit 32 pour être ensuite acheminé dans le circuit 35a.

Le niveau 27a correspond à un niveau de débordement du digestat liquide 27, c'est-à-dire le niveau à partir duquel le digestat liquide 27 va déborder et, par conséquent, circuler dans la ramification 32 pour être acheminé dans le circuit d'évacuation 34a.

Le sens de circulation 39" du digestat liquide 27 dans la ramification arrondie 32' et dans le circuit 34a d'évacuation et le sens de circulation 39'" du biogaz dans le circuit d'évacuation 35a ont été représentés.

La ramification 32' permet donc de séparer le biogaz 29 et le digestat liquide 27 afin d'acheminer principalement du biogaz 29 dans le circuit d'évacuation 35a, i.e. vers l'espace de stockage en biogaz dans l'unité 25, et principalement du digestat liquide 27 dans le circuit d'évacuation 34a, i.e. vers la cuve 26 remplie en digestat liquide 27 de l'unité 25.

Le niveau 27a du digestat liquide 27 correspond au niveau de digestat liquide situé au-dessus de la vanne 20a.

La ramification 32' présente donc une fonction de séparation du digestat liquide et du biogaz au cours de l'étape de digestion.

L'étape de digestion permet donc de produire du biogaz sans avoir à mettre en œuvre une étape de brassage de la biomasse solide 24 ce qui permet de diminuer les investissements en matériels de brassage et d'agitation et de réduire les dépenses énergétiques et de maintenance généralement afférentes à cette étape.

En variante, les ouvertures d'évacuation du digestat liquide et du biogaz peuvent être distinctes, et la séparation du biogaz 29 et du digestat liquide 27 peut s'effectuer sans la présence d'une ramification 32'. Dans ce cas, la séparation du biogaz 29 et du digestat liquide 27 a lieu directement dans la cuve 2.

Durant l'étape de digestion, la biomasse solide 24 est immergée dans le digestat liquide 27 et peut en partie flotter en haut du digesteur anaérobie amovible 1, se trouver en partie en suspension au milieu du digesteur anaérobie amovible 1 ou en partie décanter et se trouver en bas du réacteur anaérobie mobile 1.

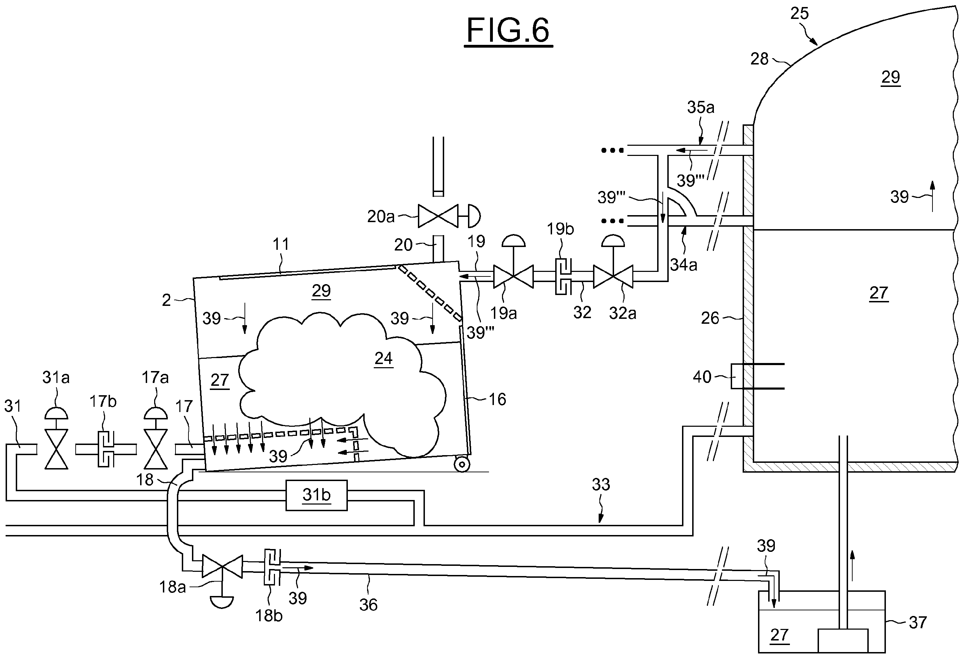

Sur la figure 6 est représentée schématiquement une étape de vidange anaérobie du digestat liquide 27 du digesteur anaérobie amovible 1 vers la fosse de relevage 37 par l'intermédiaire du circuit de vidange 36.

Au cours de cette étape de vidange, les vannes 19a et 32a sont en position ouverte, la vanne 20a reste en position fermée, les vannes 17a et 31a sont en position fermée tandis que la vanne 18a est en position ouverte pour permettre un écoulement gravitaire du digestat liquide 27 vers la fosse de relevage 37, depuis laquelle le digestat liquide 27 est ensuite pompé une nouvelle fois dans l'unité 25.

Selon une mode de réalisation de l'invention, le circuit 36 pourrait être équipé d'une pompe de vidange et la vidange de la cuve 2 pourrait être réalisée par pompage.

Selon un autre mode de l'invention, la vidange de la cuve 2 pourrait être réalisée par pompage du digestat liquide avec la pompe 31b dont le sens de pompage serait inversé. Dans cette dernière configuration le digestat liquide vidangé serait transféré dans le réseau 33.

Durant cette étape, l'espace vacant laissé par l'évacuation du digestat liquide est rempli par du biogaz en provenance de l'unité 25. A l'issue de cette étape, la biomasse solide 24 partiellement ou totalement digérée vient reposer partiellement sur le fond du réacteur anaérobie amovible 1 et sur l'élément de séparation 21 situé sur le fond du digesteur anaérobie amovible 1.

L'étape de vidange anaérobie permet, par le tassement au fond du digesteur anaérobie amovible 1 de la biomasse solide 24 partiellement digérée suite à l'étape de digestion et de diffusion, de supprimer les éventuels chemins préférentiels de percolation ainsi que les éventuelles poches de biogaz, bloquées dans la biomasse 24. Ainsi le biogaz 29, se trouvant dans la tuyauterie 19 ou le circuit 32, circule depuis l'unité 25 en direction de l'intérieur du digesteur anaérobie amovible 1 en passant à travers l'élément de séparation perforé supérieur 22.

Le biogaz vient ainsi remplir l'espace laissé vacant par la vidange du digestat liquide 27. Au cours de cette étape de vidange, la biomasse solide 24 redescend vers le fond du digesteur anaérobie amovible 1 en direction de l'élément de séparation perforé 21, et vient finalement se déposer sur le fond du digesteur anaérobie amovible 1 et/ou sur l'élément de séparation perforé 21.

Sur la figure 6 est représenté le sens de circulation du biogaz 29 dans la tuyauterie 19 et le circuit 32 en direction du digesteur anaérobie amovible 1 ainsi que le sens de circulation du digestat liquide 27 dans le digesteur anaérobie mobile 1.

L'élément de séparation perforé 21 présente l'avantage de laisser passer le digestat liquide 27 au moment de la vidange anaérobie en retenant dans la cuve 2 la biomasse solide 24 partiellement ou totalement digérée. L'élément de séparation perforé 21 permet donc de séparer le digestat liquide 27, en le laissant circuler, et la biomasse solide 24 ce qui permet de ne pas risquer d'obstruer la tuyauterie 18 et le circuit 36 ainsi que de ne pas introduire du digestat solide 24 dans l'unité 25.

Conformément à la figure 6, le niveau du digestat liquide 27 augmente dans l'unité 25 au moment de l'étape de vidange anaérobie.

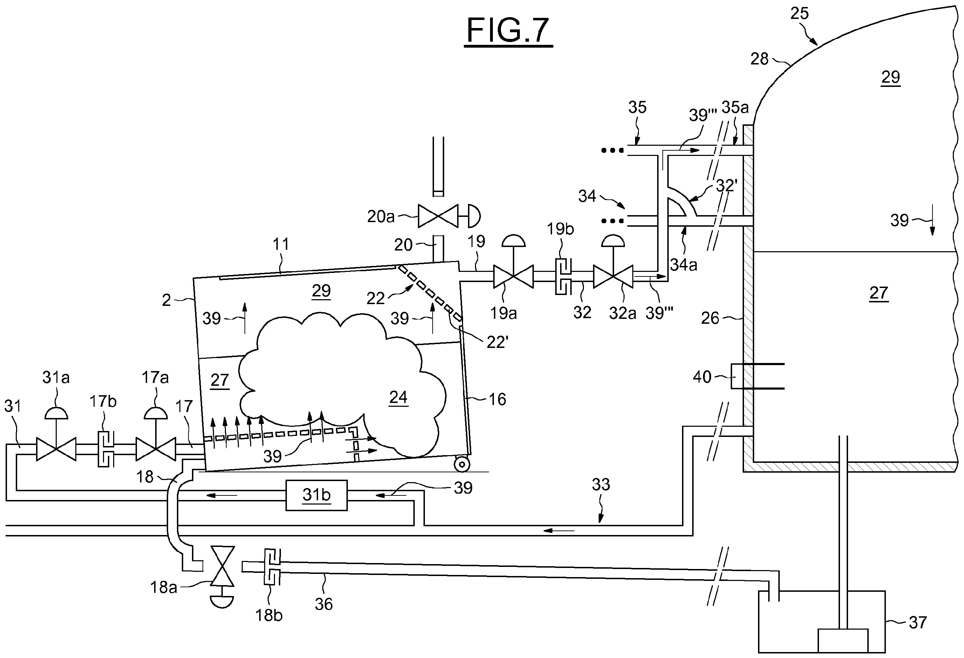

Après la vidange anaérobie du digesteur anaérobie amovible 1, une nouvelle étape de remplissage du digesteur anaérobie amovible 1 avec du digestat liquide 27 est mise en œuvre comme représenté sur la figure 7. L'étape de remplissage est identique à la précédente étape de remplissage, représentée sur la figure 4, à l'exception du fait que la vanne 20a est en position fermée et que les vannes 19a et 32a sont en position ouverte. En particulier, la vanne 18a est en position fermée au cours de l'étape de remplissage en digestat liquide du digesteur anaérobie amovible 1.

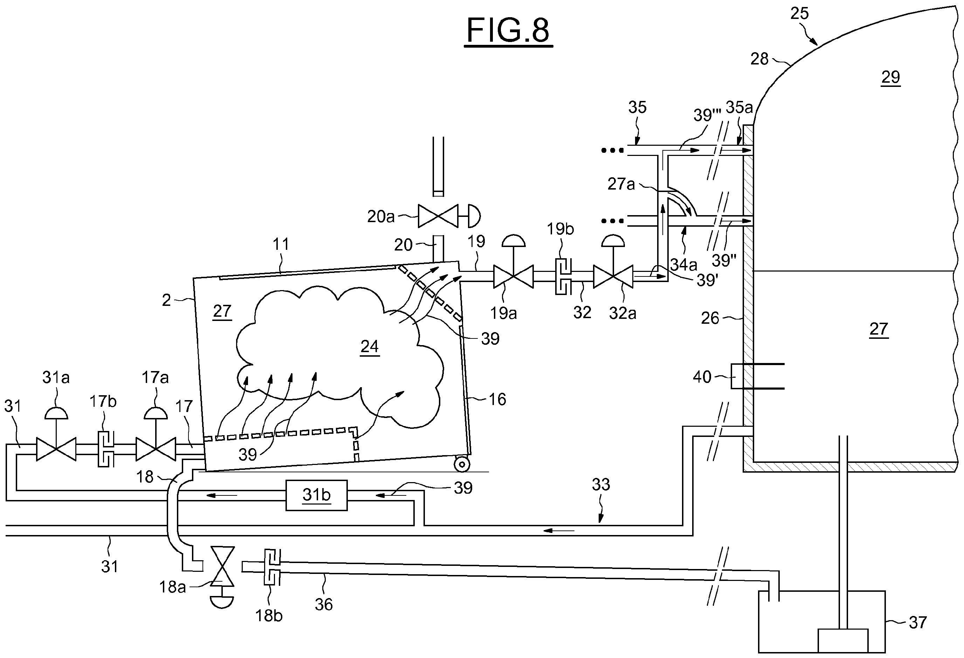

Conformément à la figure 8, une fois l'étape de remplissage terminée, l'étape de digestion de la biomasse solide 24 est une nouvelle fois mise en œuvre afin de continuer la production de biogaz 29.

De la même façon que précédemment, la ramification 32' permet de séparer le biogaz 29 et le digestat liquide 27. Ainsi le biogaz 29, qui transite par le circuit 32, est principalement acheminé par le biais du circuit 35a vers l'espace de stockage en biogaz de l'unité 25 et le digestat liquide 27 est principalement acheminé par le biais du circuit 34a vers la cuve 25 de l'unité 24.

L'étape de digestion de la biomasse solide 24, telle que décrite dans la figure 8, est identique à celle décrite dans la figure 5.

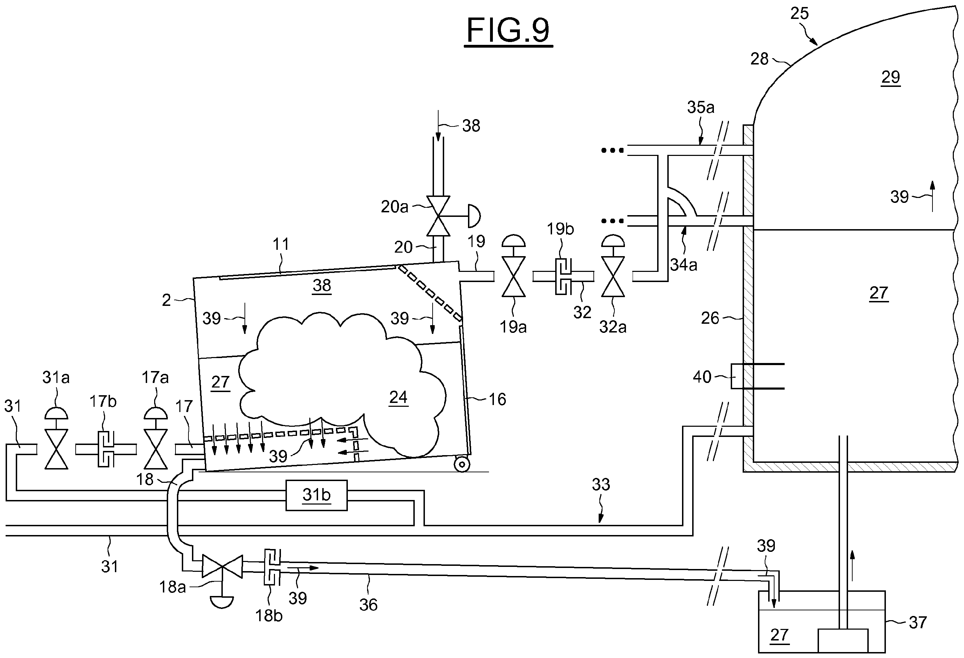

Une fois l'étape de digestion terminée, c'est-à-dire en fin de cycle au bout d'un délai généralement compris entre 20 et 80 jours, une étape de vidange aérobie du réacteur anaérobie mobile 1 est mise en œuvre comme illustré sur la figure 9.

L'étape de vidange aérobie est mise en œuvre en fermant les vannes 19a et 32a de manière à empêcher toute introduction de biogaz 29 et de digestat liquide 27 à l'intérieur du digesteur anaérobie amovible 1. Au cours de l'étape de vidange aérobie, la vanne 20 est ouverte afin d'introduire de l'air 38 à l'intérieur du digesteur anaérobie amovible 1 de manière à remplacer le digestat liquide 27 évacué, qui est vidangé à travers la tuyauterie 18 et le circuit 36, après ouverture de la vanne 18a, à travers l'élément de séparation perforé 21, directement vers la fosse de relevage 37, dans laquelle le digestat liquide peut être éventuellement pompé vers la cuve 26 de l'unité 25.

Cette étape permet de vidanger le digestat liquide 27 dans le digesteur anaérobie amovible 1 en évitant la présence de biogaz dans la cuve 2, ce qui permet de minimiser les fuites/rejets de biogaz dans l'atmosphère au moment de la mise en œuvre ultérieure de l'étape d'évacuation de la biomasse solide résiduelle après digestion, c'est-à-dire le digestat solide.

Sur cette figure, le niveau du digestat liquide 26 diminue et l'air 38 s'introduit progressivement à l'intérieur digesteur anaérobie amovible 1 en comblant l'espace laissé vacant par la vidange du digestat liquide 27.

La figure 10 représente de manière schématique le digesteur anaérobie amovible 1 déconnecté de l'unité 25 et, plus largement, du système de production de biogaz.

Pour ce faire, le digesteur anaérobie amovible 1 est déconnecté du reste du système de production de biogaz en déconnectant les moyens 17b, 18b et 19b respectivement du réseau d'alimentation 33 en digestat liquide 27, du circuit de vidange 36 ainsi que du circuit d'évacuation 32 qui appartient au réseau d'évacuation 34 de digestat liquide 27 et au réseau d'évacuation 35 de biogaz 29.

En particulier, la vanne 18a est en position fermée lors de la déconnection du digesteur amovible 1.

Ainsi le digesteur anaérobie amovible 1 est déconnecté de l'unité 25 et du reste du système de production de biogaz de manière à pouvoir transporter le digesteur 1 et permettre le déchargement de la biomasse solide digérée résiduelle dans un autre lieu que le lieu d'implantation du système de production de biogaz. Le lieu de déchargement de la biomasse solide digérée résiduelle peut être différent du lieu de chargement en biomasse solide effectué au cours de l'étape de chargement.

Une fois la digestion terminée, l'étape d'évacuation de la biomasse solide digérée résiduelle est préférentiellement réalisée par renversement du digesteur anaérobie amovible 1 et évacuation de la biomasse solide digérée résiduelle au travers de la trappe 16.

La biomasse solide digérée résiduelle est donc évacuée à travers la trappe 16.

En variante, le toit amovible 11 peut être relevé et ouvert de manière à ce qu'un grappin puisse être actionné à travers l'orifice d'entrée 10 du digesteur anaérobie amovible 1. De cette façon, le grappin permet de saisir la biomasse solide digérée résiduelle, une fois la digestion terminée, de manière à l'évacuer soit vers une zone de stockage ou vers une zone de traitement ou encore vers un engin de transport.

En particulier, le grappin saisit la biomasse solide digérée résiduelle dans la cuve 2 du digesteur anaérobie amovible 1 en laissant éventuellement subsister des résidus de biomasse solide digérée au niveau des parois ou du fond du digesteur anaérobie amovible 1.

Des résidus de biomasse solide digérée peuvent être laissés au niveau des parois, ou dans le fond du digesteur anaérobie amovible 1, ou encore sur l'élément de séparation inférieur 21, car ils peuvent constituer un bon substrat, riche en bactéries qui seront susceptibles d'agir lors du prochain cycle de méthanisation.

Grâce à l'étape de vidange aérobie précédente, l'étape de récupération de la biomasse solide digérée résiduelle après méthanisation, correspondant au digestat solide, peut se dérouler en toute sécurité en minimisant les risques de rejet de biogaz dans l'atmosphère ainsi que les risques de mise en contact avec le biogaz les personnes qui effectuent les opérations de déchargement.

Ainsi l'orifice d'entrée 10 du digesteur anaérobie amovible 1 peut représenter à la fois une zone d'admission de la biomasse solide 24 avant méthanisation ainsi qu'une zone d'évacuation de la biomasse solide digérée résiduelle après méthanisation, appelée digestat solide.

En d'autres termes, l'orifice d'entrée 10 du digesteur anaérobie amovible 1 constitue un espace d'introduction de la biomasse solide et éventuellement un espace d'évacuation de la biomasse solide digérée résiduelle.

De préférence, l'étape d'évacuation de la biomasse solide digérée résiduelle est mise en œuvre par renversement du digesteur anaérobie amovible 1 à travers la trappe 16.

Comme indiqué précédemment, les figures 11 à 16 représentent schématiquement des vues en coupe d'un digesteur anaérobie amovible 1 dans lequel ont lieu différentes étapes successives d'un procédé de production de biogaz dans le cas où le digesteur est indirectement (liaison série) alimenté en digestat liquide en provenance d'un autre digesteur anaérobie mobile de biomasse solide.

Les figures 11 à 16 se concentrent donc seulement sur une partie du système de production de biogaz, c'est-à-dire sur un digesteur anaérobie amovible alimenté indirectement par une unité centrale de stockage de digestat liquide et de digestion complémentaire et d'une unité de stockage de biogaz.

De la même façon que sur la figure 4, la figure 11 représente une unité centrale de stockage de digestat liquide et de digestion complémentaire et une unité de stockage de biogaz constituant une seule et même unité, dénommé unité 25.

Le dispositif de chauffage 40 de digestat liquide est positionné sur la cuve 26 remplie de digestat liquide 27 de l'unité 25.

La figure 11 est identique la figure 4 à l'exception du fait que le circuit d'alimentation 31 en digestat liquide 27 n'est pas relié directement à la cuve 26 de l'unité 25 mais est relié à un des digesteurs anaérobies précédents (non représenté sur la figure 11 mais représenté sur la figure 18).

Plus précisément, au cours de l'étape de remplissage, le digestat liquide 27 peut provenir du digesteur anaérobie qui précède directement le digesteur anaérobie amovible 1 dans le système de production en biogaz.

Autrement dit, le digestat liquide 27 peut provenir du digesteur anaérobie amovible situé directement ou non en amont du digesteur anaérobie amovible 1.

Le circuit d'alimentation 31 est ainsi relié au circuit d'évacuation 34a évacuant le digestat liquide 27 issu du digesteur anaérobie amovible situé directement ou non en aval du digesteur anaérobie amovible 1, préférentiellement du digesteur anaérobie amovible situé directement en aval.

Le circuit d'alimentation 31 appartient à un réseau d'alimentation 33 en digestat liquide qui relie de façon indirecte le digesteur anaérobie amovible 1 à l'unité 25 et aux autres digesteurs anaérobies amovibles appartenant au système de production de biogaz (tels que représentés sur la figure 18).

De la même façon que, dans les figures 4 à 9, le réseau d'évacuation 34 en digestat liquide comprend le circuit d'évacuation 32 en digestat liquide 27 et biogaz 29 et le circuit d'évacuation 34a en digestat liquide 27.

Le réseau d'évacuation 35 en biogaz comprend le circuit d'évacuation 32 en digestat liquide 27 et biogaz 29 et le circuit d'évacuation 35a en biogaz.

Le circuit d'évacuation 34a comprend une vanne 41 en position fermé.

La vanne 41 est située en amont de l'intersection entre le circuit d'évacuation 34a du digesteur anaérobie situé en amont et le circuit d'alimentation 31 de manière à ce que le digestat liquide 27 soit acheminé vers le digesteur anaérobie amovible 1 lors de l'étape de remplissage.

Ainsi la figure 11 représente, après fermeture du toit amovible 11, une étape de remplissage en digestat liquide du digesteur anaérobie amovible 1. En particulier, le digestat liquide 27 est acheminé au niveau de la partie inférieure du digesteur anaérobie mobile 1 sous l'élément perforé 21 et circule à travers les interstices 21'.

L'étape de remplissage en digestat liquide est identique à l'étape décrite dans la figure 4.

La pompe de circulation 31b, non illustrée sur la figure 11, est située sur le circuit alimentant en digestat liquide le digesteur anaérobie se trouvant à l'extrémité aval de la pluralité des digesteurs (tel que représenté sur la figure 18).

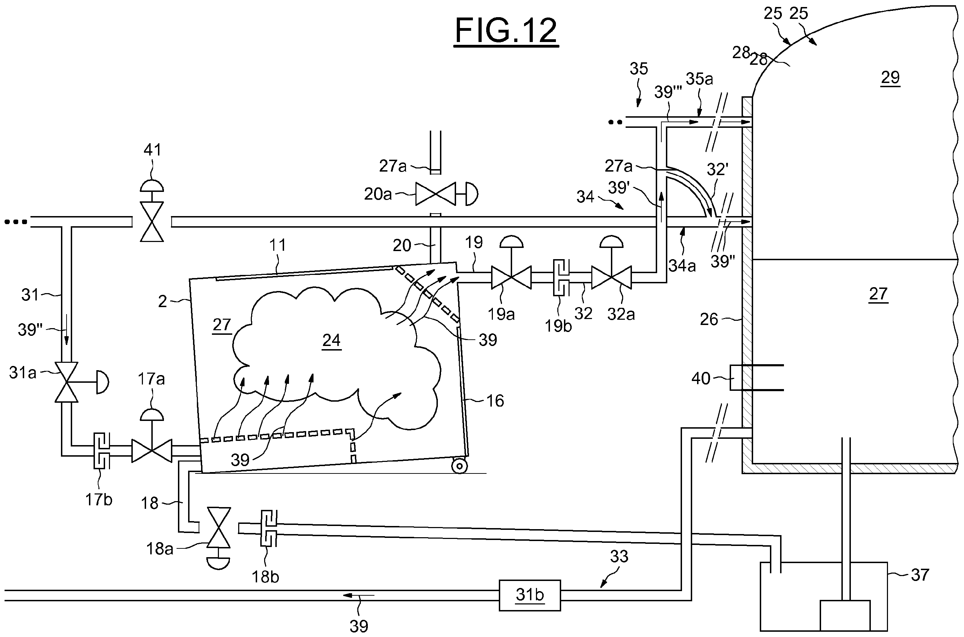

La figure 12 illustre l'étape de digestion de la biomasse solide 24 dans des conditions anaérobies. Au cours de cette étape, du biogaz se forme au sein du digesteur anaérobie amovible 1.

Une percolation du digestat liquide 27 à travers la biomasse 24 est mise en œuvre par le pompage du digestat liquide 27 en provenance de l'unité 25. La percolation se déroule à travers la biomasse solide 24 de manière à ce que la plus grande partie possible de la biomasse solide 24 soit mise en contact intime avec les bactéries contenues dans le digestat liquide 27 et puisse ainsi réagir au cours de la digestion.

L'étape de digestion est identique à l'étape décrite dans la figure 5.

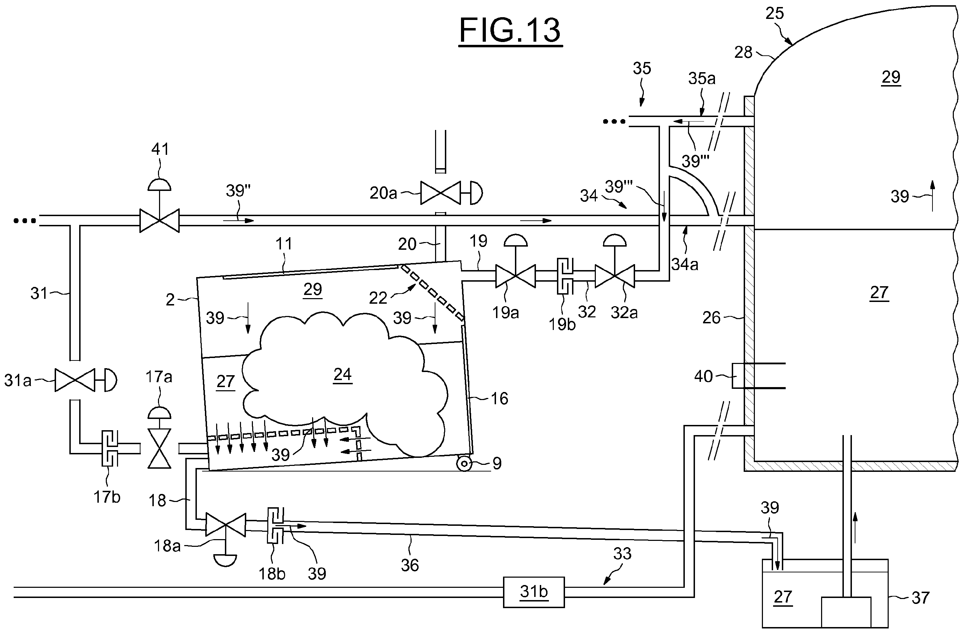

La figure 13 décrit une étape de vidange anaérobie dans laquelle le digestat liquide 27 est évacué par le biais de la tuyauterie 18 relié au circuit de vidange 36 jusqu'à la fosse de relevage 37.

Au cours de cette étape, la vanne 20a est en position fermée, pour éviter que de l'air ne rentre dans le digesteur anaérobie 1. Les vannes 19a et 32a sont en position ouverte afin de permettre au biogaz 29, en provenance directe ou indirecte de l'unité 25, de remplir, dans le digesteur anaérobie amovible 1, l'espace laissé vacant par la vidange du digestat liquide 27. La vanne 31a est en position fermée pour éviter que du digestat liquide 27 en provenance du digesteur anaérobie situé en amont ne rentre dans le digesteur anaérobie amovible 1. La vanne 41 est en position ouverte de manière à ce que le digestat liquide 27, en provenance du digesteur anaérobie situé en amont, puisse être dirigé vers le digesteur anaérobie situé en aval du digesteur anaérobie amovible 1 ou bien directement vers l'unité 25.

Sur la figure 13, le digestat liquide circule directement vers l'unité 25.

L'étape de vidange anaérobie est identique à l'étape décrite dans la figure 6. De la même façon que précédemment, l'étape de vidange anaérobie permet de supprimer les éventuels chemins préférentiels de percolation ainsi que les éventuelles poches de biogaz bloqués dans la biomasse solide 24.

La figure 14 décrit une nouvelle étape de remplissage en digestat liquide dans le digesteur anaérobie amovible 1. De la même façon que dans la figure 11, le digestat liquide peut provenir du digesteur anaérobie amovible situé directement ou non en amont du digesteur anaérobie amovible 1.

La figure 15 décrit une nouvelle étape de percolation du digestat liquide à travers la biomasse solide 24 qui est identique à l'étape décrite dans la figure 12.

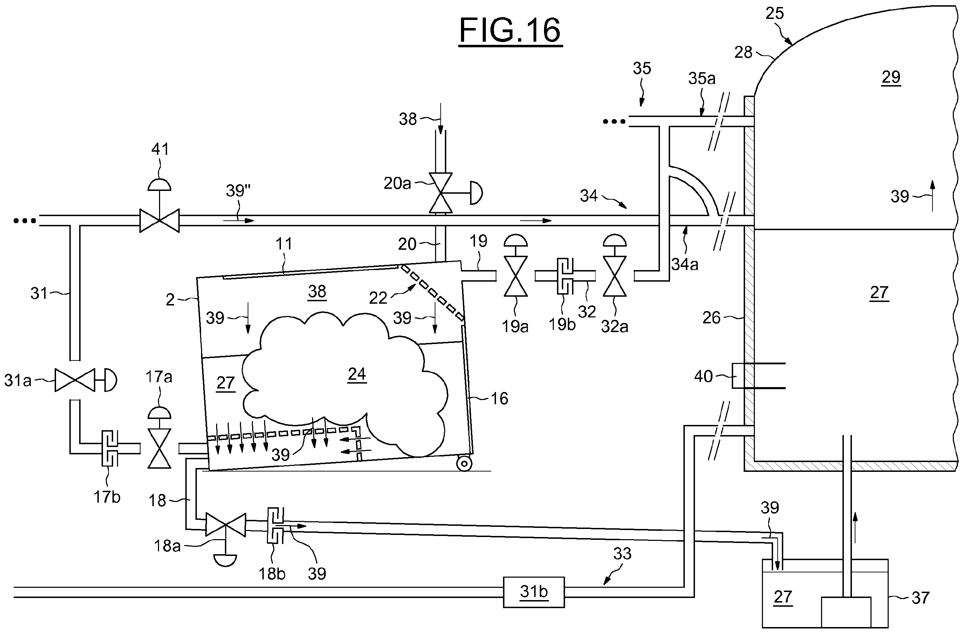

La figure 16 décrit une étape de vidange aérobie mise en œuvre une fois l'étape de digestion terminée en fermant les vannes 19a et 32a de manière à empêcher toute introduction de biogaz 29 à l'intérieur du digesteur anaérobie amovible 1. Au cours de l'étape de vidange aérobie, la vanne 20a est ouverte afin d'introduire de l'air 38 à l'intérieur du digesteur anaérobie amovible 1 de manière à remplacer le digestat liquide 27 évacué, qui est pompé par le biais de la pompe 31b, à travers l'élément de séparation perforé inférieur 21 afin d'évacuer le digestat liquide 27 vers la cuve 26 de l'unité 25.

La vanne 31a est en position fermée pour éviter que du digestat liquide 27 en provenance du digesteur anaérobie situé en amont ne rentre dans le digesteur anaérobie amovible 1. La vanne 40 est en position ouverte pour permettre que le digestat liquide 27 en provenance du digesteur anaérobie situé en amont puisse être dirigé vers le digesteur anaérobie situé en aval du digesteur anaérobie amovible 1 ou bien directement vers l'unité centrale de stockage de digestat liquide et de biogaz et de digestion complémentaire 25.

L'étape de vidange aérobie est identique à l'étape décrite dans la figure 9.

Une fois la digestion terminée et l'étape de vidange aérobie réalisées, le digesteur anaérobie amovible 1 est déconnecté du reste du système de production de biogaz en déconnectant les moyens 17a, 18a et 19a.

La vanne 31a est en position fermée pour éviter que du digestat liquide 27 en provenance du digesteur anaérobie situé en amont ne se répande sur le sol. La vanne 41 est en position ouverte de manière à ce que le digestat liquide 27 en provenance du digesteur anaérobie situé en amont puisse être dirigé vers le digesteur anaérobie situé en aval du digesteur anaérobie amovible 1 ou bien directement vers l'unité 25.

L'étape de déchargement de la biomasse solide digérée résiduelle peut être réalisée de la même façon que précédemment.

La figure 17 représente schématiquement une vue en coupe d'une pluralité de digesteurs anaérobies amovibles de biomasse solide alimentés directement en digestat liquide par une unité de digestion complémentaire et de stockage de digestat liquide et de biogaz (liaison en parallèle) au cours d'une étape de digestion dans des conditions anaérobies.

La figure 17 se concentre donc sur un système de production de biogaz comprenant une pluralité de digesteurs anaérobies amovibles 100, 101, 102 et 103 contenant de la biomasse solide directement alimentée en digestat liquide 27 en provenance d'une unité centrale de stockage de digestat liquide et de digestion complémentaire et une unité de stockage de biogaz constituant une seule et même unité, dénommé unité 25.

L'unité 25 est identique à l'unité décrite dans les figures 4 à 16, c'est-à-dire qu'elle comprend notamment une espace de stockage du biogaz 29 et une cuve 26 remplie en digestat liquide 27.

Conformément à la figure 17, le système de production de biogaz peut comprendre une pluralité de digesteurs anaérobies amovibles entre le digesteur 100 et le digesteur 101.

Chaque digesteur 100, 101, 102 et 103 représenté sur la figure 17 est identique au digesteur anaérobie amovible 1 représenté sur les figures 1 et 2.

En effet, chaque digesteur 100, 101, 102 et 103 comprend respectivement une tuyauterie 170, 270, 370 et 470 munie respectivement d'une vanne et d'un moyen de raccordement de manière à les raccorder à un circuit d'alimentation 310, 410, 510 et 610 en digestat liquide 27.

Les tuyauteries 170, 270, 370 et 470 constituent une ouverture d'alimentation en digestat liquide 27.

Chaque circuit d'alimentation 310, 410, 510 et 610 comprend respectivement une vanne et une pompe d'alimentation 310b, 410b, 510b et 610b.

Les moyens de raccordement permettent de connecter chacun des digesteurs anaérobies amovibles 100, 101, 102 et 103 aux circuits d'alimentation 310, 410, 510 et 610 en digestat liquide 27.

Les circuits d'alimentation 310, 410, 510 et 610 appartiennent au réseau d'alimentation 33 en digestat liquide 27 qui relie l'unité 25 à chaque digesteur anaérobie amovible 100, 101, 102 et 103 du système de production de biogaz.

Chaque digesteur 100, 101, 102 et 103 comprend également une ouverture d'évacuation 190, 290, 390 et 490 de digestat liquide 27 et de biogaz 29 connectée aux circuits d'évacuation 320, 420, 520 et 620 de digestat liquide 27 et de biogaz 29.

Les circuits d'évacuation 320, 420, 520 et 620 appartiennent au réseau d'évacuation 34 de digestat liquide 27 et au réseau d'évacuation 35 de biogaz 29.

Les circuits d'évacuation 320, 420, 520 et 620 se divisent en un circuit d'évacuation 34a de digestat liquide et un circuit d'évacuation 35a de biogaz.

Le réseau d'évacuation 34 de digestat liquide 27 comprend les circuits d'évacuation 320, 420, 520 et 620 de digestat liquide et de biogaz et un circuit d'évacuation 34a de digestat liquide.

Le réseau d'évacuation 35 de biogaz 29 comprend des circuits d'évacuation 320, 420, 520 et 620 de digestat liquide et biogaz et un circuit d'évacuation 35a de biogaz.

Chaque digesteur 100, 101, 102 et 103 comprend une ouverture de vidange non représentée sur la figure 17. L'ouverture de vidange est notamment identique à l'ouverture de vidange 18 du digesteur anaérobie amovible 1.

L'étape de digestion est identique à l'étape de digestion décrite dans la figure 5.

L'alimentation en digestat liquide 27 s'effectue dans chaque digesteur 100, 101, 102 et 103 par le biais des circuits d'alimentation 310, 410, 510 et 610 appartenant au réseau d'alimentation 33 en digestat liquide qui est relié à l'unité 25.

Conformément à ce mode de réalisation, chaque digesteur 100, 101, 102 et 103 est directement alimenté en digestat liquide 27 en provenance de l'unité 25.

Au cours de l'étape de digestion de la biomasse solide, d'une part, le biogaz 29 est évacué dans chaque circuit d'évacuation 320, 420, 520 et 620 de digestat liquide et de biogaz ainsi que dans le circuit d'évacuation 35a vers l'espace de stockage en biogaz de l'unité 25.

D'autre part, le digestat liquide 27 est évacué dans chaque circuit d'évacuation 320, 420, 520 et 620 de digestat liquide et de biogaz ainsi que dans le circuit d'évacuation 34a vers la cuve 26 remplie de digestat liquide 27 de l'unité 25.

Conformément à ce mode de réalisation, d'une part, le digestat liquide 27 et, d'autre part, le biogaz 29 sont directement évacués depuis chaque digesteur 100, 101, 102 et 103 vers l'unité 25.

La figure 17 représente un système de production de biogaz dans lequel une étape de digestion se déroule au même moment dans chaque digesteur 100, 101, 102 et 103.

Conformément à une variante selon la présente invention, les étapes de digestion peuvent ne pas se dérouler de manière simultanée dans chaque digesteur anaérobie lorsque les digesteurs anaérobies sont directement alimentés en digestat liquide en provenance de l'unité 25.

De préférence, après une étape de vidange aérobie dans un digesteur, ce dernier peut être soustrait du reste du système de production de biogaz en déconnectant les moyens de raccordement.

En particulier, l'un des digesteurs 100, 101, 102 et 103 peut être déconnecté du reste du système tandis que la digestion anaérobie continue à opérer dans les autres digesteurs.

De même, certains digesteurs peuvent être dans une phase de vidange anaérobie, ou de vidange aérobie ou de remplissage de digestat liquide, tandis que les autres digesteurs sont dans une phase de digestion anaérobie.

Il est à noter que, pour chaque digesteur anaérobie amovible, les étapes de remplissage, de vidange aérobie et anaérobie sont identiques à celles précédemment décrites dans les figures 4 à 9.

La figure 18 représente schématiquement une vue en coupe d'une pluralité de digesteurs anaérobies amovibles de biomasse solide alimentés indirectement en digestat liquide par une unité de digestion complémentaire et de stockage de digestat liquide et de biogaz (liaison en série) au cours d'une étape de digestion dans des conditions anaérobies.

La figure 18 se concentre donc sur un système de production de biogaz comprenant une pluralité de digesteurs anaérobies amovibles 100, 101, 102 et 103 contenant de la biomasse solide reliés en série à l'unité centrale de stockage de digestat liquide et de digestion complémentaire et une unité de stockage de biogaz constituant une seule et même unité, dénommé unité 25.

L'unité 25 est identique à l'unité décrite dans les figures 11 à 16, c'est-à-dire qu'elle comprend notamment une espace de stockage du biogaz 29 et une cuve 26 remplie en digestat liquide 27.

Chaque digesteur 100, 101, 102 et 103 représenté sur la figure 18 est identique au digesteur anaérobie amovible 1 représenté sur les figures 1 et 2.

L'étape de digestion se déroule de manière identique à l'étape de digestion décrite dans la figure 12.

Pour chaque digesteur anaérobie 101, 102 et 103, l'alimentation en digestat liquide 27 s'effectue de façon indirecte depuis l'unité 25 car le digestat liquide 27 transite par le ou les digesteurs anaérobies situés directement ou non en amont de chaque digesteur anaérobie 101, 102 et 103.

Conformément au mode de réalisation décrit dans la figure 18, l'unité 25 alimente directement en digestat liquide 27 le digesteur anaérobie 100 duquel est évacué, au cours de l'étape de digestion, du digestat liquide 27 et du biogaz par le biais du circuit d'évacuation de digestat liquide et de biogaz.

Lors de la séparation du biogaz 29 et du digestat liquide 27 au niveau de l'entrée de la ramification 32', le digestat liquide 27 est acheminé dans le circuit d'évacuation 34a puis dans le circuit d'alimentation en direction du digesteur situé directement ou non en aval, par exemple le digesteur anaérobie 101.

Chaque circuit d'alimentation récupère le digestat liquide 27 ayant transité dans le digesteur anaérobie précédent.

Par ailleurs, les vannes 41 montées sur le circuit d'évacuation 34a en digestat liquide sont en position fermée, notamment pour acheminer le digestat liquide vers le digesteur anaérobie suivant.

Conformément à une variante selon la présente invention, les étapes de digestion peuvent ne pas se dérouler de manière simultanée dans chaque digesteur anaérobie lorsque les digesteurs anaérobies sont indirectement alimentés en digestat liquide en provenance de l'unité 25.

Il est à noter que les étapes de remplissage, de vidange aérobie et anaérobie sont identiques à celles précédemment décrites dans les figures 11 à 16.

La figure 19 représente schématiquement une vue en coupe d'une pluralité de digesteurs anaérobies amovibles de biomasse solide alimentés à la fois directement ou indirectement en digestat liquide par une unité de digestion complémentaire et de stockage de digestat liquide et d'une unité centrale de biogaz (liaison en série et en parallèle) au cours d'une étape de digestion dans des conditions anaérobies.

La figure 19 se concentre sur un système de production de biogaz comprenant une pluralité de digesteurs anaérobies amovibles 100, 101, 102, 103 et 104 contenant de la biomasse solide.

Dans ce système, d'une part, les digesteurs anaérobies amovibles 100, 101 et 103 sont alimentés directement en digestat liquide 27 en provenance de l'unité 25, et, d'autre part, les digesteurs anaérobies amovibles 102 et 104 sont alimentés en digestat liquide 27 respectivement par les digesteurs 101 et 103.

En d'autres termes, les digesteurs anaérobies amovibles 102 et 104 sont alimentés indirectement en digestat liquide 27 en provenance de l'unité 25.

L'unité 25 est identique à l'unité décrite dans les figures 11 à 16, c'est-à-dire qu'elle comprend notamment un espace de stockage du biogaz 29 et une cuve 26 remplie en digestat liquide 27.

Chaque digesteur 100, 101, 102 et 103 représenté sur la figure 18 est identique au digesteur anaérobie amovible 1 représenté sur les figures 1 et 2.

L'étape de digestion se déroule de manière identique à l'étape de digestion décrite à la fois dans les figures 5 et 12.

Sur la figure 19 ont été représentés le sens de circulation 39" du digestat liquide et le sens de circulation 39''' du biogaz dans le réseau d'évacuation du digestat liquide, le réseau d'évacuation du biogaz vers l'unité 25 et dans le réseau d'alimentation en digestat liquide.

Conformément à une variante selon la présente invention, les étapes de digestion peuvent ne pas se dérouler de manière simultanée dans chaque digesteur anaérobie.

En particulier, certains digesteurs peuvent être dans une phase de vidange anaérobie, ou de vidange aérobie ou de remplissage de digestat liquide, tandis que les autres digesteurs sont dans une phase de digestion anaérobie.

La figure 20 illustre schématiquement une vue en coupe d'une pluralité de digesteurs de biomasse solide composés de digesteurs anaérobies amovibles 100, 101 et 102 et d'un digesteur anaérobie immobile 100a alimentés directement en digestat liquide par une unité de digestion complémentaire et de stockage de digestat liquide et de biogaz (liaison en parallèle) au cours de l'étape de digestion dans des conditions anaérobies.

Les digesteurs anaérobies amovibles 100, 101 et 102 sont identiques au digesteur anaérobie amovible 1 représenté sur les figures 1 et 2.

L'étape de digestion dans les digesteurs anaérobies amovibles 100, 101 et 102 est identique à l'étape de digestion décrite précédemment.

Le digesteur anaérobie immobile 100a comprend une cuve 2' contenant de la biomasse solide 24', comportant au moins une zone d'admission 10' de la biomasse solide, située sur la partie supérieure de la cuve, sur laquelle un toit 11' s'ouvre et se referme, et comportant en outre au moins une zone d'évacuation 10' de la biomasse solide digérée résiduelle ; les zones d'admission et d'évacuation de biomasse solide étant communes c'est-à-dire confondues.

Le toit 11' est amovible est apte à s'ouvrir et se refermer sur la zone d'admission 10' de la biomasse solide 24'.

La cuve 2' comporte également, au niveau de sa partie inférieure, un élément de séparation perforé 21a, de préférence un plancher perforé occupant partiellement ou totalement le fond de la cuve 2'. Ainsi l'élément de séparation perforé 21a peut correspondre à une couronne perforée occupant partiellement le fond de la cuve 2'.

De manière alternative, l'élément de séparation perforé 21 peut correspondre à un élément vertical situé en surépaisseur de la virole ou de la partie du mur vertical de la cuve 2'. L'élément de séparation perforé 21 peut également correspondre à tout autre type d'élément perforé situé en amont du circuit de vidange de digestat liquide 410.

L'élément de séparation 21a comprend une pluralité d'interstices afin de laisser circuler le digestat liquide lors des étapes de vidanges anaérobies et aérobies du digesteur tout en retenant le digestat solide dans la cuve 2' du digesteur 100a.

La cuve 2' comporte, au niveau de sa partie supérieure, un élément de séparation perforé 22a, qui est également fixée à la paroi latérale contigüe.

L'élément de séparation perforé 22a comprend une pluralité d'interstices 22'a.

L'élément de séparation perforé 22a est connecté à un circuit d'évacuation 32b de digestat liquide et de biogaz sur lequel est montée une vanne, située en amont de la ramification 32'.

La cuve 2' comporte également une tuyauterie 20' qui est en saillie vers l'extérieur par rapport à la surface supérieure de la cuve 2'. La tuyauterie 20' est ouverte vers l'extérieur ce qui permet d'acheminer l'air à l'intérieur de la cuve 2' du digesteur 100a ou de l'évacuer vers l'extérieur de la cuve 2'. La vanne 20'a, montée sur la tuyauterie 16, permet de rendre possible ou d'empêcher l'introduction ou l'évacuation de l'air à l'intérieur de la cuve 2'.

Le digesteur anaérobie 100a est alimenté directement en digestat liquide par le biais d'un circuit d'alimentation 410 muni d'une pompe 410b en provenance de l'unité 25.

L'étape de digestion dans le digesteur 100a est identique à l'étape décrite précédemment pour les digesteurs anaérobie amovibles, c'est-à-dire qu'une percolation du digestat liquide à travers la biomasse est mise en œuvre par pompage du digestat liquide 27 en provenance de l'unité 25. La percolation se déroule à travers la biomasse solide de manière à ce que la plus grande partie possible de la biomasse solide soit mise en contact intime avec les bactéries contenues dans le digestat liquide 27 et puisse ainsi réagir au cours de la digestion.

Au cours de cette étape, un processus de diffusion du digestat liquide 27 dans la biomasse solide se produit. Un tel processus de diffusion est favorisé, d'une part, par l'immersion de la biomasse solide dans le digestat liquide 27 et, d'autre part, par la pression du digestat liquide 27 sur la biomasse solide qui résulte de l'action de la pompe 410b.

Le biogaz et le digestat liquide sont extraits à travers les interstices de l'élément de séparation perforé 22' tandis que la biomasse solide reste immergée dans la cuve 2'.

Le biogaz et le digestat liquide sont acheminés ensemble dans le circuit d'évacuation 32 jusqu'à l'entrée de la ramification 32' où le biogaz et le digestat liquide sont séparés gravitairement.

De la même façon que précédemment, le circuit d'évacuation 32b se divise en un circuit d'évacuation 34a du digestat liquide et un circuit d'évacuation 35a du biogaz au niveau de la ramification 32'.

Le digesteur anaérobie 100a est fixe et ne comporte pas au niveau du circuit d'alimentation 410 en digestat liquide ou au niveau du circuit d'évacuation 32b de digestat liquide et biogaz des moyens configurés pour le connecter et/ou le déconnecter au reste du système de production de biogaz.

Par ailleurs, le digesteur anaérobie 100a est alimenté en biomasse solide par le haut, c'est-à-dire à travers la zone d'admission 10', lorsque le toit 11' est position ouverte, par le biais d'un moyen de préhension, de préférence un grappin comportant plusieurs crochets, sur le site d'implantation du système de production de biogaz.

De la même façon, une fois la digestion terminée, après la vidange anaérobie, la biomasse digérée résiduelle est enlevée à travers la zone 10', qui présente la fonction d'évacuer ladite biomasse, par le biais du moyen de préhension précédemment défini.

Conformément à ce mode de réalisation, la vidange aérobie et anaérobie s'effectue à travers le circuit 410 et la pompe 410b. En d'autres termes, conformément à ce mode de réalisation, le circuit d'alimentation de digestat liquide 410 et le circuit de vidange de digestat liquide 410 sont confondus et constituent un seul et même circuit.

Dans un autre mode de réalisation le circuit d'alimentation de digestat liquide et le circuit de vidange de digestat liquide sont distinct, et les vidange anaérobies et aérobies du digestat liquide se font à travers une ouverture et un circuit dédié.

Les digesteurs anaérobies amovibles 100, 101 et 102, ainsi que le digesteur anaérobie immobile 100a, sont alimentés directement en digestat liquide en provenance de l'unité 25.

Dans un autre mode de réalisation les digesteurs anaérobies amovibles 100, 101 et 102, ainsi que le digesteur anaérobie immobile 100a, sont alimentés indirectement en digestat liquide en provenance de l'unité 25, tel que décrit dans la figure 18.

Dans un autre mode de réalisation les digesteurs anaérobies amovibles 100, 101 et 102, ainsi que le digesteur anaérobie immobile 100a, sont alimentés pour certains directement et pour d'autres indirectement en digestat liquide en provenance de l'unité 25, tel que décrit dans la figure 19.

La figure 21 illustre une vue schématique de dessus d'un système de production de biogaz comprenant :
- une unité centrale de stockage de digestat liquide et de digestion complémentaire et une unité centrale de stockage de biogaz constituant une seule et même unité, dénommé unité 25,
- une pluralité de digesteurs anaérobies immobiles 50, 51, 52, 53, 54, 55 et 56 disposés autour de l'unité 25 formant ainsi un cercle autour de l'unité 25, chacun desdits digesteurs étant reliés à l'unité 25 par le biais d'un circuit d'alimentation en digestat liquide et un circuit d'évacuation en digestat liquide,
- une pluralité de digesteurs anaérobies amovibles 60, 61, 62, 63, 64 et 65 qui sont chacun alimentés directement en digestat liquide 27 en provenance de l'unité 25 par le biais d'un réseau d'alimentation 33 en digestat liquide (liaison en parallèle),
- une pluralité de digesteurs anaérobies amovibles 71, 72, 73, 74 et 75 qui sont chacun alimentés indirectement en digestat liquide 27 en provenance de l'unité 25 par le biais d'un réseau d'alimentation 33' en digestat liquide (liaison en série), et un digesteur anaérobie amovible 70 qui se trouve à la première place de la série et qui est donc alimenté directement en digestat liquide 27 en provenance de l'unité 25 par le biais d'un réseau d'alimentation 33' en digestat liquide.

L'unité 25 est identique à l'unité décrite dans les figures précédentes.

L'unité 25 comprend une cuve, ayant une forme essentiellement cylindrique, remplie de digestat liquide, qui est surmontée d'une membrane étanche constituant un espace de stockage de gaz, lequel est rempli de biogaz.

La pluralité de digesteurs anaérobies immobiles 50, 51, 52, 53, 54, 55 et 56 sont disposés de manière à former un cercle autour de l'unité 25.

Chaque digesteur anaérobie immobile 50, 51, 52, 53, 54, 55 et 56 est relié à l'unité 25 par le biais d'un circuit d'alimentation en digestat liquide et d'un circuit d'évacuation de digestat liquide.

Sur la figure 21, les sens de circulation du digestat liquide entre chaque digesteur anaérobie immobile 50, 51, 52, 53, 54, 55 et l'unité 25 ont été représentés.

Chaque digesteur anaérobie amovible 60, 61, 62, 63, 64 et 65 est identique au digesteur anaérobie 1 décrit précédemment.

Les digesteurs anaérobies amovibles 60, 61, 62, 63, 64 et 65 comprennent donc au moins un ouverture d'alimentation en digestat liquide, au moins une ouverture d'évacuation en digestat liquide, au moins une ouverture d'évacuation de biogaz, au moins une ouverture de vidange de digestat liquide et au moins une ouverture d'introduction et/ou d'évacuation d'air.

Pour des raisons de simplicité, seules les ouvertures d'alimentation en digestat liquide, les ouvertures d'évacuation de digestat liquide des digesteurs anaérobies amovibles 60, 61, 62, 63, 64 et 65 sont représentées.

Chaque digesteur anaérobie amovible 60, 61, 62, 63, 64 et 65 est alimenté directement en digestat liquide en provenance de l'unité 25 par le biais d'un réseau d'alimentation 33 en digestat liquide (liaison en parallèle).

Plus précisément, chaque ouverture d'alimentation en digestat liquide est reliée à un circuit d'alimentation en digestat liquide appartenant au réseau d'alimentation 33 en digestat liquide.

Chaque digesteur anaérobie amovible 60, 61, 62, 63, 64 et 65 est également relié à un circuit d'évacuation de digestat liquide appartenant à un réseau d'évacuation de digestat liquide.

Les sens de circulation du digestat liquide entre l'unité 25 et chaque digesteur anaérobie amovible 60, 61, 62, 63, 64 et 65 ont été représentés sur la figure 21.

Chaque digesteur anaérobie amovible 70, 71, 72, 73, 74 et 75 est identique au digesteur anaérobie 1 décrit précédemment

Pour des raisons de simplicité, seules les ouvertures d'alimentation en digestat liquide, les ouvertures d'évacuation de digestat liquide des digesteurs anaérobies amovibles 70, 71, 72, 73, 74 et 75 sont représentées.

Chaque digesteur anaérobie amovible 71, 72, 73, 74 et 75 est alimenté indirectement en digestat liquide en provenance de l'unité 25 par le biais d'un réseau d'alimentation 33' en digestat liquide (liaison en série).

En particulier, chaque digesteur anaérobie amovible 71, 72, 73, 74 et 75 est alimenté en digestat liquide par le biais du digesteur anaérobie précédent c'est-à-dire que le digestat liquide 27 a transité dans le précédent digesteur anaérobie avant d'alimenter le suivant.

Chaque ouverture d'alimentation en digestat liquide est reliée à un circuit d'alimentation en digestat liquide appartenant au réseau d'alimentation 33' en digestat liquide.

Chaque digesteur anaérobie amovible 70, 71, 72, 73, 74 et 75 est également relié à un circuit d'évacuation de digestat liquide.

Les sens de circulation du digestat liquide entre l'unité 25 et chaque digesteur anaérobie amovible 70, 71, 72, 73, 74 et 75 ont été représentés sur la figure 21.

Sur la figure 21 sont représentées des pompes de circulation du digestat liquide disposés sur les circuits d'alimentation en digestat liquide.

En outre, le système de production comporte un dispositif de chauffage du digestat liquide qui peut être placé à n'importe quel endroit du système. Le système de chauffage n'a pas été représenté sur la figure 21.

Le système de production de biogaz, illustré dans la figure 21, présente donc une pluralité de digesteurs anaérobies de biomasse solide composés :
- de plusieurs digesteurs anaérobies de biomasse solide immobiles,
- de plusieurs digesteurs anaérobies amovibles de biomasse solide, alimentés directement en digestat liquide en provenance d'une unité centrale de stockage de digestat liquide et de digestion complémentaire et d'une unité centrale de stockage de biogaz constituant une seule et même unité (liaisons en parallèle), et
- de plusieurs digesteurs anaérobies amovibles de biomasse solide alimentés indirectement en digestat liquide en provenance d'une unité centrale de stockage de digestat liquide et de digestion complémentaire et d'une unité centrale de stockage de biogaz constituant une seule et même unité (liaisons en série).

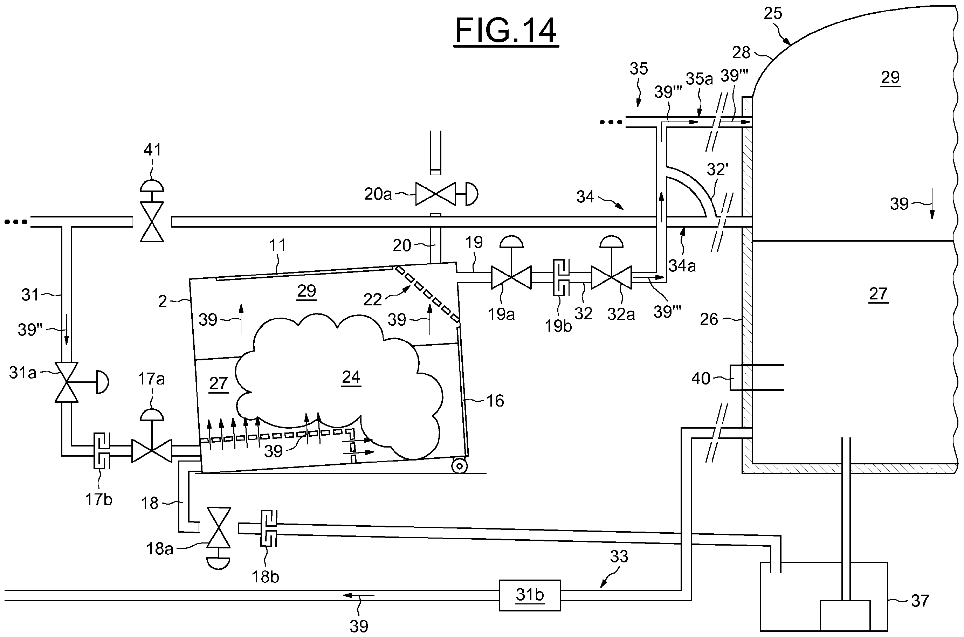

Comme indiqué ci-avant chacun des digesteurs, représentés dans la figure 14, présente une ouverture d'évacuation de biogaz, pouvant être commune à l'ouverture d'évacuation de digestat liquide, qui est reliée à au moins un circuit d'évacuation de biogaz appartenant au réseau d'évacuation de biogaz.

Conformément à ce mode de réalisation, l'unité centrale de stockage de digestat liquide et de digestion complémentaire et l'unité centrale de stockage de biogaz sont constituées d'une seule et même unité.

En variante, l'unité centrale de stockage de digestat liquide et de digestion complémentaire et unité centrale de stockage de biogaz peuvent être deux unités distinctes comprenant chacune un ou plusieurs ouvrages.

Dans le système de production de biogaz tel qu'illustré, un ou plusieurs digesteurs anaérobies amovibles peuvent être calorifugés.

De préférence, tous les digesteurs anaérobies de biomasse solide du système selon l'invention sont reliés à un réseau d'alimentation en digestat liquide, un réseau d'évacuation de digestat liquide et un réseau d'évacuation de biogaz.

De préférence, tous les digesteurs anaérobies amovibles du système sont reliés à un réseau d'alimentation en digestat liquide, un réseau d'évacuation de digestat liquide et un réseau d'évacuation de biogaz.

## Revendications

1. Système de production de biogaz comprenant :
• au moins une unité centrale (25) de stockage de digestat liquide (27) et de digestion complémentaire, constituée d'un ou plusieurs ouvrages, apte à contenir principalement du digestat liquide (27),
• au moins une unité centrale (25) de stockage de biogaz (29), constitué d'un ou plusieurs ouvrages, apte à contenir principalement du biogaz (29),
• une pluralité de digesteurs anaérobies dont certains sont des digesteurs de biomasse solide, chacun desdits digesteurs de biomasse solide (1, 50, 51, 52, 53, 54, 55, 56, 60, 61, 62, 63, 64, 65, 70, 71, 72, 73, 74, 75, 100, 101, 102, 103, 100a) comprenant :
- une cuve (2) apte à contenir de la biomasse solide (24), comportant au moins une zone d'admission (10) de la biomasse solide (24), située sur la partie supérieure de la cuve (2), sur laquelle un toit (11, 10') est apte à s'ouvrir et à se refermer, et comportant en outre au moins une zone d'évacuation (10, 16) de la biomasse solide digérée résiduelle, certaines desdites zones d'admission (10) et d'évacuation (10, 16) de biomasse solide (23) pouvant être communes, et
- au moins une ouverture d'alimentation (17, 170, 270, 370, 470) en digestat liquide (27), située sur la partie inférieure du digesteur anaérobie (1, 50, 60, 70, 100, 100a), reliée à au moins un circuit d'alimentation (31, 310, 410, 510, 610) de digestat liquide (27), apte à permettre l'introduction de digestat liquide (27) dans ledit digesteur de biomasse solide (1, 50, 60, 70, 100, 100a), et
- au moins une ouverture d'évacuation (19) de digestat liquide (27), située sur la partie supérieure du digesteur anaérobie (1, 50, 60, 70, 100, 100a), reliée à au moins un circuit d'évacuation (32, 320, 420, 520, 620, 34a) de digestat liquide (27), apte à permettre l'évacuation de digestat liquide (27) dudit digesteur de biomasse solide (1, 50, 60, 70, 100, 100a),
- au moins une ouverture d'évacuation (19) de biogaz (29), située sur la partie supérieure du digesteur anaérobie (1, 50, 60, 70, 100, 100a), reliée à au moins un circuit d'évacuation (32, 320, 420, 520, 620, 35a) de biogaz (29), apte à permettre l'évacuation de biogaz (29) depuis ledit digesteur de biomasse solide (1, 50, 60, 70, 100, 100a), et
- au moins une ouverture de vidange (18) de digestat liquide (27), située sur la partie inférieure du digesteur anaérobie (1, 50, 60, 70, 100, 100a), apte à permettre la vidange de digestat liquide (27) dudit digesteur de biomasse solide (1, 50, 60, 70, 100, 100a), et
- au moins une ouverture d'introduction et/ou d'évacuation (20) d'air (38), située sur la partie supérieure du digesteur anaérobie (1, 50, 60, 70, 100, 100a), apte à permettre l'introduction et/ou l'évacuation d'air (38) dans ledit digesteur de biomasse solide (1, 50, 60, 70, 100, 100a),
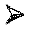 certaines ouvertures parmi lesdites ouvertures (17, 18, 19, 20) pouvant être communes, à l'exception des ouvertures d'alimentation et d'évacuation de digestat liquide qui sont distinctes, et
• au moins un réseau d'alimentation (33) de digestat liquide (27), incluant une pluralité de circuits d'alimentation (31, 310, 410, 510, 610) de digestat liquide (27), permettant de relier certaines ouvertures d'alimentation (17, 170, 270, 370, 470) de digestat liquide (27) de certains digesteurs de biomasse solide (1, 50, 60, 70, 100, 100a) à l'unité centrale de stockage de digestat liquide et de digestion complémentaire (25), apte à permettre l'alimentation de digestat liquide (27), par voie directe ou indirecte, dans lesdits digesteurs (1, 50, 60, 70, 100, 100a) depuis l'unité centrale de stockage de digestat liquide et de digestion complémentaire (25), et
• au moins un réseau d'évacuation (34) de digestat liquide (27), incluant une pluralité de circuits d'évacuation (32, 320, 420, 520, 620, 34a) de digestat liquide (27), permettant de relier certaines ouvertures d'évacuation (19) de digestat liquide de certains digesteurs de biomasse solide (1, 50, 60, 70, 100, 100a) à l'unité centrale de stockage de digestat liquide et de digestion complémentaire (25), apte à permettre l'évacuation de digestat liquide (27), par voie directe ou indirecte, depuis lesdits digesteurs (1, 50, 60, 70, 100, 100a) vers l'unité centrale de stockage de digestat liquide et de digestion complémentaire (25), et
• au moins un réseau d'évacuation (35) de biogaz (29), incluant une pluralité de circuits d'évacuation (32, 320, 420, 520, 620, 35a) de biogaz (29), permettant de relier certaines ouvertures d'évacuation (19) de biogaz de certains digesteurs de biomasse solide (1, 50, 60, 70, 100, 100a) à l'unité centrale de stockage de biogaz (25), apte à permettre l'évacuation de biogaz (29), par voie directe ou indirecte, depuis lesdits digesteurs (1, 50, 60, 70, 100, 100a) vers l'unité centrale de stockage de biogaz (25),
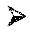 certaines parties desdits réseaux (33, 34, 35) pouvant être communes, et
• certains digesteurs parmi lesdits digesteurs de biomasse solide (1, 50, 51, 52, 53, 54, 55, 56, 60, 61, 62, 63, 64, 65, 70, 71, 72, 73, 74, 75, 100, 101, 102, 103, 100a) sont des digesteurs amovibles (1, 100, 101, 102, 103, 60, 61, 62, 63, 64, 65, 70, 71, 72, 73, 74, 75) et comportent des moyens (17b, 18b, 19b) configurés pour permettre, d'une part, la connexion chacun desdits digesteur amovibles à au moins un circuit d'alimentation (31) de digestat liquide et à au moins un circuit d'évacuation (32) de digestat liquide (27) et à au moins un circuit d'évacuation (32) de biogaz (29) et, d'autre part, la déconnection chacun desdits digesteurs desdits circuits, et
• au moins un moyen de circulation (31b, 310b, 410b, 510b, 610b) de digestat liquide (27), de préférence une pompe, située sur le réseau d'alimentation (31) de digestat liquide (27) et/ou sur le réseau d'évacuation (34) de digestat liquide (27), apte à permettre la circulation du digestat liquide (27) à travers certains digesteurs de biomasse solide , et
• au moins un dispositif de chauffage (40) de digestat liquide (27).

2. Système de production de biogaz selon la revendication 1, **caractérisé en ce que** certains desdits digesteurs de biomasses solide (100a) sont surmontés d'un moyen de préhension apte à permettre l'alimentation de la biomasse solide (24).

3. Système selon la revendication 2, **caractérisé en ce que** le moyen de préhension est un grappin comportant une pluralité de crochets ou une benne comportant des godets.

4. Système selon l'une quelconque des revendications précédentes, **caractérisé en ce que**, dans certains desdits digesteurs de biomasse solide (1, 50, 60, 70, 100, 100a), au moins l'une des ouvertures d'évacuation (19) du digestat liquide (27) comprend au moins un élément de séparation perforé (21) d'évacuation du digestat liquide (27), situé en amont de ladite ouverture d'évacuation (19) du digestat liquide (27), apte à séparer, d'une part, le digestat liquide (27) évacué et, d'autre part, la biomasse solide (24).

5. Système selon l'une quelconque des revendications précédentes, **caractérisé en ce que**, dans certains desdits digesteurs de biomasse solide (1, 50, 60, 70, 100, 100a), au moins l'une des ouvertures de vidange (18) du digestat liquide (27) comprend au moins un élément de séparation perforé (22) de vidange du digestat liquide (27), situé en amont de ladite ouverture de vidange (18) du digestat liquide, apte à séparer, d'une part, le digestat liquide (5) vidangé et, d'autre part, la biomasse solide (24).

6. Système selon la revendication 4 ou 5, **caractérisé en ce que** certains éléments de séparation perforés (21, 22) sont des plaques perforées.

7. Système selon la revendication 5, **caractérisé en ce que** l'élément de séparation perforé (21) de vidange du digestat liquide (27) est un plancher perforé.

8. Système de production de biogaz selon l'une quelconque des revendications précédentes, **caractérisé en ce que** au moins un des digesteurs amovibles (1, 100, 101, 102, 103, 60, 61, 62, 63, 64, 65, 70, 71, 72, 73, 74, 75) comporte, au niveau d'au moins l'une de ses parties latérales (8), au moins une trappe de déchargement (16) de la biomasse solide digérée résiduelle, laquelle trappe (16) est apte à être ouverte et refermée de façon étanche.

9. Système selon l'une quelconque des revendications précédentes, **caractérisé en ce que** sur certains desdits digesteur de biomasse solide (1, 50, 60, 70, 100, 100a) :
- la ou les ouverture(s) d'alimentation (17, 170, 270, 370, 470)) en digestat liquide (27), est (sont) munie(s) d'un moyen d'obstruction (17a), de préférence une vanne, apte à obturer ou permettre le passage du digestat liquide entre l'extérieur et l'intérieur du digesteur, et/ou
- la ou les ouverture(s) d'évacuation (19) de digestat liquide (27), est (sont) munie(s) d'un moyen d'obstruction (19a), de préférence une vanne, apte à obturer ou permettre le passage du digestat liquide (27) entre l'intérieur et l'extérieur du digesteur (1, 50, 60, 70, 100, 100a), et/ou
- la ou les ouverture(s) d'évacuation (19) de biogaz (29), est (sont) munie(s) d'un moyen d'obstruction (19a), de préférence une vanne, apte à obturer ou permettre le passage du biogaz entre l'intérieur et l'extérieur du digesteur, et/ou
- la ou les ouverture(s) de vidange (18) de digestat liquide, est (sont) munie(s) d'un moyen d'obstruction (18a), de préférence une vanne, apte à obturer ou permettre le passage du digestat liquide (27) entre l'intérieur et l'extérieur du digesteur (1, 50, 60, 70, 100, 100a), et/ou
- la ou les ouverture(s) d'introduction et/ou d'évacuation (20) d'air, est (sont) munie(s) d'un moyen d'obstruction (20a), de préférence une vanne, apte à obturer ou permettre le passage de l'air (38) entre l'intérieur et l'extérieur du digesteur (1, 50, 60, 70, 100, 100a).

10. Système selon l'une quelconque des revendications précédentes, **caractérisé en ce que** certains desdits digesteurs anaérobies de biomasse solide (1, 50, 60, 70, 100, 100a) comportent un joint liquide positionné autour du toit de la cuve, apte à empêcher la sortie du biogaz (29) contenu dans ledit digesteur (1, 50, 60, 70, 100, 100a) lorsque le toit est fermé, et apte à empêcher les entrées d'air (38) dans ledit digesteur lorsque le toit (11, 10') est fermé.

11. Système de production de biogaz selon l'une quelconque des revendications précédentes, **caractérisé en ce que** certains ouvrages composant l'unité centrale de stockage de digestat liquide et de digestion complémentaire (25), d'une part, et certains ouvrages composant l'unité centrale de stockage de biogaz (25), d'autre part, sont communs.

12. Système de production de biogaz selon l'une quelconque des revendications précédentes, **caractérisé en ce que** l'unité centrale de stockage de digestat liquide et de digestion complémentaire (25), d'une part, et l'unité centrale de stockage de biogaz (25), d'autre part, sont communes et constituent une seule et même unité centrale de de stockage de digestat liquide et de biogaz et de digestion complémentaire.

13. Système selon l'une quelconque des revendications précédentes, **caractérisé en ce que**, sur certains des digesteurs de biomasse solide (1, 50, 60, 70, 100, 100a), au moins une ouverture d'évacuation (19) de digestat liquide (27) et une ouverture d'évacuation (19) de biogaz (29) sont communes et sont reliées à au moins un circuit d'évacuation (32, 320, 420, 520, 620) d'un mélange de digestat liquide (27) et de biogaz (29), lequel circuit (32, 320, 420, 520, 620) est connecté à un moyen de séparation (32') apte à séparer le biogaz (29) et le digestat liquide (27).

14. Système selon l'une quelconque des revendications précédentes, **caractérisé en ce que**, au moins un digesteur amovible (1, 100, 101, 102, 103, 60, 61, 62, 63, 64, 65, 70, 71, 72, 73, 74, 75) est calorifugé.

15. Procédé de production de biogaz comprenant au moins les étapes successives suivantes :
- une étape de chargement en biomasse solide (24) d'au moins un digesteur amovible (1, 100, 101, 102, 103, 60, 61, 62, 63, 64, 65, 70, 71, 72, 73, 74, 75), tel que défini selon l'une quelconque des revendications précédentes, mise en œuvre dans un lieu de chargement autre que le lieu d'implantation du système de production de biogaz, tel que défini selon l'une quelconque des revendications précédente, manuellement ou par au moins un moyen de chargement, à travers la zone d'admission (10),
- une étape de transport dudit digesteur amovible (1, 100, 101, 102, 103, 60, 61, 62, 63, 64, 65, 70, 71, 72, 73, 74, 75) chargé en biomasse solide (24), depuis le lieu de chargement de la biomasse solide (24) jusqu'au lieu d'implantation dudit système de production de biogaz,
- une étape de connexion dudit digesteur amovible (1, 100, 101, 102, 103, 60, 61, 62, 63, 64, 65, 70, 71, 72, 73, 74, 75) chargé en biomasse solide (24) à au moins un circuit d'alimentation (31, 310, 410, 510, 610) de digestat liquide (27) et à au moins un circuit d'évacuation (32, 320, 420, 520, 620, 34a) de digestat liquide (27) et à au moins un circuit d'évacuation (32, 320, 420, 520, 620, 35a) de biogaz (29),
- une étape d'alimentation dudit digesteur amovible (1, 100, 101, 102, 103, 60, 61, 62, 63, 64, 65, 70, 71, 72, 73, 74, 75) avec du digestat liquide (27) pour immerger la biomasse solide (24) contenue dans ledit digesteur amovible (1, 100, 101, 102, 103, 60, 61, 62, 63, 64, 65, 70, 71, 72, 73, 74, 75),
- une étape de digestion de la biomasse solide (24) dans ledit digesteur amovible (1, 100, 101, 102, 103, 60, 61, 62, 63, 64, 65, 70, 71, 72, 73, 74, 75), comprenant la percolation du digestat liquide (27) à travers la biomasse solide (24) et la diffusion du digestat liquide (27) dans la biomasse solide (24) dans des conditions anaérobies pour générer puis récupérer du biogaz (29), et
- une étape de vidange aérobie du digestat liquide (27) du digesteur amovible (1, 100, 101, 102, 103, 60, 61, 62, 63, 64, 65, 70, 71, 72, 73, 74, 75).

16. Procédé de production de biogaz selon la revendication 15, **caractérisé en ce qu'**il comprend, en outre, après l'étape de vidange aérobie du digestat liquide (27) du digesteur amovible (1, 100, 101, 102, 103, 60, 61, 62, 63, 64, 65, 70, 71, 72, 73, 74, 75), les étapes suivantes :
- une étape de déconnexion dudit digesteur amovible (1, 100, 101, 102, 103, 60, 61, 62, 63, 64, 65, 70, 71, 72, 73, 74, 75), chargé en biomasse solide digérée résiduelle, du circuit d'alimentation (31, 310, 410, 510, 610) de digestat liquide (27) et du circuit d'évacuation (32, 320, 420, 520, 620, 34a) de digestat liquide (27) et du circuit d'évacuation (32, 320, 420, 520, 620, 35a) de biogaz (29),
- une étape de transport dudit digesteur amovible (1, 100, 101, 102, 103, 60, 61, 62, 63, 64, 65, 70, 71, 72, 73, 74, 75) chargé en biomasse solide digérée résiduelle, depuis le lieu d'implantation du système de production de biogaz jusqu'à un lieu de déchargement de la biomasse solide digérée,
- une étape d'évacuation de la biomasse solide digérée résiduelle, mise en œuvre, de préférence, par renversement du digesteur amovible (1, 100, 101, 102, 103, 60, 61, 62, 63, 64, 65, 70, 71, 72, 73, 74, 75) provoquant l'évacuation de la biomasse solide digérée résiduelle à travers une zone de déchargement latérale (16), ou par au moins un moyen de préhension, à travers une zone d'évacuation de la biomasse solide digérée résiduelle situé sur la partie supérieure de la cuve (2), sur laquelle un toit (11) est apte à s'ouvrir et à se refermer.

17. Procédé de production de biogaz, selon la revendication 15, **caractérisé en ce qu'**il comprend en outre entre l'étape de digestion et l'étape de vidange aérobie du digestat liquide au moins les étapes suivantes :
- une étape de vidange anaérobie du digestat liquide (27) du digesteur amovible (1, 100, 101, 102, 103, 60, 61, 62, 63, 64, 65, 70, 71, 72, 73, 74, 75), et
- une étape de re-remplissage du digesteur amovible (1, 100, 101, 102, 103, 60, 61, 62, 63, 64, 65, 70, 71, 72, 73, 74, 75) avec un digestat liquide (27) après vidange anaérobie.

18. Procédé de production de biogaz, selon la revendication 17, **caractérisé en ce qu'**il comprend en outre, après l'étape de re-remplissage du digesteur amovible (1, 100, 101, 102, 103, 60, 61, 62, 63, 64, 65, 70, 71, 72, 73, 74, 75) avec un digestat liquide (27), au moins une étape de digestion, constituée d'une percolation du digestat liquide (27) à travers la biomasse solide (24) et d'une diffusion du digestat liquide dans la biomasse solide, pour générer puis récupérer du biogaz.

19. Digesteur anaérobie de biomasse solide amovible (1, 100, 101, 102, 103, 60, 61, 62, 63, 64, 65, 70, 71, 72, 73, 74, 75), comportant :
- une cuve (2) apte à contenir de la biomasse solide, (24) comportant au moins une zone d'admission (10) de la biomasse solide (24), située sur la partie supérieure de la cuve (2), sur laquelle un toit (11) est apte à s'ouvrir et à se refermer, et comportant en outre au moins une zone d'évacuation (10, 16) de la biomasse solide digérée résiduelle, certaines desdites zones d'admission (10) et d'évacuation (10, 16) de biomasse solide pouvant être communes, et
- au moins une ouverture d'alimentation (17, 170, 270, 370, 470) en digestat liquide (27), située sur la partie inférieure du digesteur anaérobie (1, 50, 60, 70, 100, 100a) comportant au moins un moyen configuré (17b) pour permettre d'être reliée à au moins un circuit d'alimentation (31, 310, 410, 510, 610) de digestat liquide (27), apte à permettre l'introduction de digestat liquide (27) dans le digesteur,
- au moins une ouverture d'évacuation (19) de digestat liquide (27), située sur la partie supérieure du digesteur anaérobie, comportant au moins un moyen (19b) configuré pour permettre d'être reliée à au moins un circuit d'évacuation (32, 320, 420, 520, 620) de digestat liquide, apte à permettre l'évacuation de digestat liquide (27) dudit digesteur,
- au moins une ouverture d'évacuation (19) de biogaz (29), située sur la partie supérieure du digesteur anaérobie, comportant au moins un moyen configuré (19b) pour permettre d'être reliée à au moins un circuit d'évacuation (32, 320, 420, 520, 620) de biogaz (29), apte à permettre l'évacuation de biogaz (29) depuis ledit digesteur, et
- au moins une ouverture de vidange (18) de digestat liquide (27), située sur la partie inférieure du digesteur anaérobie, apte(s) permettre la vidange de digestat liquide dudit digesteur de biomasse solide, et
- au moins une ouverture d'introduction et/ou d'évacuation (20) d'air (38), située sur la partie supérieure du digesteur anaérobie, apte(s) permettre l'introduction et/ou l'évacuation d'air (38) dans ledit digesteur de biomasse solide,
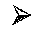 certaines ouvertures (17, 18, 19, 20) parmi lesdites ouvertures pouvant être communes, à l'exception des ouvertures d'alimentation (17) et d'évacuation (19) de digestat liquide qui sont distinctes ; ledit digesteur (1, 100, 101, 102, 103, 60, 61, 62, 63, 64, 65, 70, 71, 72, 73, 74, 75) étant apte :
- à être connecté et déconnecté d'un réseau d'alimentation (33) en digestat liquide, d'un réseau d'évacuation (34) de digestat liquide (27) et d'un réseau d'évacuation (35) de biogaz,

## Patentansprüche

1. System zur Herstellung von Biogas, umfassend:
• mindestens eine Zentraleinheit (25) zum Lagern von flüssigem Gärgut (27) und zur ergänzenden Gärung, bestehend aus einem oder mehreren Werken, die imstande ist, hauptsächlich flüssiges Gärgut (27) zu enthalten,
• mindestens eine Zentraleinheit (25) zum Lagern von Biogas (29), bestehend aus einem oder mehreren Werken, die imstande ist, hauptsächlich Biogas (29) zu enthalten,
• eine Vielzahl von anaeroben Gärbehältern, von denen bestimmte Gärbehälter für feste Biomasse sind, wobei jeder der Gärbehälter für feste Biomasse (1, 50, 51, 52, 53, 54, 55, 56, 60, 61, 62, 63, 64, 65, 70, 71, 72, 73, 74, 75, 100, 101, 102, 103, 100a) umfasst:
- einen Bottich (2), der imstande ist, feste Biomasse (24) zu enthalten, mindestens eine Einlasszone (10) für die feste Biomasse (24) beinhaltend, die sich im oberen Teil des Bottichs (2) befindet, auf dem ein Dach (11, 10') imstande ist, sich zu öffnen und zu schließen, und weiter mindestens eine Ausbringungszone (10, 16) für die restliche vergorene feste Biomasse beinhaltend, wobei bestimmte der Einlass- (10) und Ausbringungszonen (10, 16) für feste Biomasse (23) gemeinsam sein können, und
- mindestens eine Zuführungsöffnung (17, 170, 270, 370, 470) für flüssiges Gärgut (27), die sich im unteren Teil des anaeroben Gärbehälters (1, 50, 60, 70, 100, 100a) befindet, die mit mindestens einem Zuführungskreis (31, 310, 410, 510, 610) für flüssiges Gärgut (27) verbunden ist, die imstande ist, die Einführung von flüssigem Gärgut (27) in den Gärbehälter für feste Biomasse (1, 50, 60, 70, 100, 100a) zu ermöglichen, und
- mindestens eine Ausbringungsöffnung (19) für flüssiges Gärgut (27), die sich im oberen Teil des anaeroben Gärbehälters (1, 50, 60, 70, 100, 100a) befindet, die mit mindestens einem Ausbringungskreis (32, 320, 420, 520, 620, 34a) für flüssiges Gärgut (27) verbunden ist, die imstande ist, die Ausbringung des flüssigen Gärguts (27) aus dem Gärbehälter für feste Biomasse (1, 50, 60, 70, 100, 100a) zu ermöglichen,
- mindestens eine Ausbringungsöffnung (19) für Biogas (29), die sich im oberen Teil des anaeroben Gärbehälters (1, 50, 60, 70, 100, 100a) befindet, die mit mindesten einem Ausbringungskreis (32, 320, 420, 520, 620, 35a) für Biogas (29) verbunden ist, die imstande ist, die Ausbringung von Biogas (29) aus dem Gärbehälter für feste Biomasse (1, 50, 60, 70, 100, 100a) zu ermöglichen, und
- mindestens eine Entleerungsöffnung (18) für flüssiges Gärgut (27), die sich im unteren Teil des anaeroben Gärbehälters (1, 50, 60, 70, 100, 100a) befindet, die imstande ist, die Entleerung von flüssigem Gärgut (27) aus dem Gärbehälter für feste Biomasse (1, 50, 60, 70, 100, 100a) zu ermöglichen, und
- mindestens eine Einführungs- und/oder Ausbringungsöffnung (20) für Luft (38), die sich im oberen Teil des anaeroben Gärbehälters (1, 50, 60, 70, 100, 100a) befindet, die imstande ist, die Einführung und/oder Ausbringung von Luft (38) in dem Gärbehälter für feste Biomasse (1, 50, 60, 70, 100, 100a) zu ermöglichen,
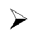 wobei bestimmte Öffnungen unter den Öffnungen (17, 18, 19, 20), mit Ausnahme der Zuführungs- und Ausbringungsöffnungen für flüssiges Gärgut, die unterschiedlich sind, gemeinsam sein können, und
• mindestens ein Zuführungsnetz (33) für flüssiges Gärgut (27), das eine Vielzahl von Zuführungskreisen (31, 310, 410, 510, 610) für flüssiges Gärgut (27) beinhaltet, die es ermöglichen, bestimmte Zuführungsöffnungen (17, 170, 270, 370, 470) für flüssiges Gärgut (27) bestimmter Gärbehälter für feste Biomasse (1, 50, 60, 70, 100, 100a) mit der Zentraleinheit zum Lagern von flüssigem Gärgut und zur ergänzenden Gärung (25) zu verbinden, die imstande ist, die Zuführung von flüssigem Gärgut (27) auf direktem oder indirektem Weg in die Gärbehälter (1, 50, 60, 70, 100, 100a) aus der der Zentraleinheit zum Lagern von flüssigem Gärgut und zur ergänzenden Gärung (25) zu ermöglichen, und
• mindestens ein Ausbringungsnetz (34) für flüssiges Gärgut (27), das eine Vielzahl von Ausbringungskreisen (32, 320, 420, 520, 620, 34a) für flüssiges Gärgut (27) beinhaltet, die es ermöglichen, bestimmte Ausbringungsöffnungen (19) für flüssiges Gärgut bestimmter Gärbehälter für feste Biomasse (1, 50, 60, 70, 100, 100a) mit der der Zentraleinheit zum Lagern von flüssigem Gärgut und zur ergänzenden Gärung (25) zu verbinden, die imstande ist, die Ausbringung von flüssigem Gärgut (27) auf direktem oder indirektem Weg aus den Gärbehältern (1, 50, 60, 70, 100, 100a) zur der Zentraleinheit zum Lagern von flüssigem Gärgut und zur ergänzenden Gärung (25) zu ermöglichen, und
• mindestens ein Ausbringungsnetz (35) für Biogas (29), das eine das eine Vielzahl von Ausbringungskreisen (32, 320, 420, 520, 620, 35a) für Biogas (29) beinhaltet, die es ermöglichen, bestimmte Ausbringungsöffnungen (19) für Biogas bestimmter Gärbehälter für feste Biomasse (1, 50, 60, 70, 100, 100a) mit der Zentraleinheit zum Lagern von Biogas (25) zu verbinden, die imstande ist, die Ausbringung von Biogas (29) auf direktem oder indirektem Weg aus den Gärbehältern (1, 50, 60, 70, 100, 100a) zur Zentraleinheit zum Lagern von Biogas (25) zu ermöglichen,
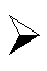 wobei bestimmte Teile der Netze (33, 34, 35) gemeinsam sein können, und
• bestimmte Gärbehälter unter den Gärbehältern für feste Biomasse (1, 50, 51, 52, 53, 54, 55, 56, 60, 61, 62, 63, 64, 65, 70, 71, 72, 73, 74, 75, 100, 101, 102, 103, 100a) abnehmbare Gärbehälter (1, 100, 101, 102, 103, 60, 61, 62, 63, 64, 65, 70, 71, 72, 73, 74, 75) sind und Mittel (17b, 18b, 19b) beinhalten, die konfiguriert sind, um einerseits die Verbindung jedes der abnehmbaren Gärbehälter mit mindestens einem Zuführungskreis (31) für flüssiges Gärgut und mit mindestens einem Ausbringungskreis (32) für flüssiges Gärgut (27) und mit mindestens einem Ausbringungskreis (32) für Biogas (29), und andererseits die Trennung jedes der Gärbehälter von den Kreisen zu ermöglichen, und
• mindestens ein Mittel zum Zirkulieren (31b, 310b, 410b, 510b, 610b) von flüssigem Gärgut (27), vorzugsweise eine Pumpe, die sich auf dem Zuführungsnetz (31) für flüssiges Gärgut (27) und/oder auf dem Ausbringungsnetz (34) für flüssiges Gärgut (27) befindet, die imstande ist, die Zirkulation des flüssigen Gärguts (27) durch bestimmte Gärbehälter für feste Biomasse zu ermöglichen, und
• mindestens eine Heizvorrichtung (40) für flüssiges Gärgut (27).

2. System zur Herstellung von Biogas nach Anspruch 1, **dadurch gekennzeichnet, dass** bestimmte der Gärbehälter für feste Biomasse (100a) von einem Greifmittel überragt werden, das imstande ist, die Zuführung von fester Biomasse (24) zu ermöglichen.

3. System nach Anspruch 2, **dadurch gekennzeichnet, dass** das Greifmittel ein Greifer, der eine Vielzahl von Haken beinhaltet oder ein Kipper ist, der Baggerschaufeln beinhaltet.

4. System nach einem der vorstehenden Ansprüche, **dadurch gekennzeichnet, dass** in bestimmten der Gärbehälter für feste Biomasse (1, 50, 60, 70, 100, 100a) mindestens eine der Ausbringungsöffnungen (19) des flüssigen Gärguts (27) mindestens ein perforiertes Trennelement (21) zum Ausbringen des flüssigen Gärguts (27) umfasst, das sich stromaufwärts der Ausbringungsöffnung (19) des flüssigen Gärguts (27) befindet, das imstande ist, einerseits das ausgebrachte flüssige Gärgut (27), und andererseits die feste Biomasse (24) zu trennen.

5. System nach einem der vorstehenden Ansprüche, **dadurch gekennzeichnet, dass** in bestimmten der Gärbehälter für feste Biomasse (1, 50, 60, 70, 100, 100a) mindestens eine der Entleerungsöffnungen (18) des flüssigen Gärguts (27) mindestens ein perforiertes Trennelement (22) zum Entleeren des flüssigen Gärguts (27) umfasst, das sich stromaufwärts der Ausbringungsöffnung (18) des flüssigen Gärguts befindet, das imstande ist, einerseits das entleerte flüssige Gärgut (5), und andererseits die feste Biomasse (24) zu trennen.

6. System nach Anspruch 4 oder 5, **dadurch gekennzeichnet, dass** bestimmte perforierte Trennelemente (21, 22) perforierte Platten sind.

7. System nach Anspruch 5, **dadurch gekennzeichnet, dass** das perforierte Trennelement (21) zur Entleerung des flüssigen Gärguts (27) ein perforierter Boden ist.

8. System zur Herstellung von Biogas nach einem der vorstehenden Ansprüche, **dadurch gekennzeichnet, dass** mindestens einer der abnehmbaren Gärbehälter (1, 100, 101, 102, 103, 60, 61, 62, 63, 64, 65, 70, 71, 72, 73, 74, 75) im Bereich mindestens eines seiner seitlichen Teile (8) mindestens eine Entladeklappe (16) der restlichen vergorenen festen Biomasse beinhaltet, wobei die Klappe (16) imstande ist, in dichter Form geöffnet und geschlossen zu werden.

9. System nach einem der vorstehenden Ansprüche, **dadurch gekennzeichnet, dass** auf bestimmten der Gärbehälter für feste Biomasse (1, 50, 60, 70, 100, 100a):
- die Zuführungsöffnung(en) (17, 170, 270, 370, 470) für flüssiges Gärgut (27) mit einem Verschlussmittel (17a), vorzugsweise einem Ventil versehen ist (sind), das imstande ist, den Durchgang des flüssigen Gärguts zwischen dem Außenbereich und dem Innenbereich des Gärbehälters zu verschließen oder zu ermöglichen, und/oder
- die Ausbringungsöffnung(en) (19) für flüssiges Gärgut (27) mit einem Verschlussmittel (19a), vorzugsweise einem Ventil versehen ist (sind), das imstande ist, den Durchgang des flüssigen Gärguts (27) zwischen dem Innenbereich und dem Außenbereich des Gärbehälters (1, 50, 60, 70, 100, 100a) zu verschließen oder zu ermöglichen, und/oder
- die Ausbringungsöffnung(en) (19) für Biogas (29) mit einem Verschlussmittel (19a), vorzugsweise einem Ventil versehen ist (sind), das imstande ist, den Durchgang des Biogases zwischen dem Innenbereich und dem Außenbereich des Gärbehälters zu verschließen oder zu ermöglichen, und/oder
- die Entleerungsöffnung(en) (18) für flüssiges Gärgut mit einem Verschlussmittel (18a), vorzugsweise einem Ventil versehen ist (sind), das imstande ist, den Durchgang des flüssigen Gärguts (27) zwischen dem Innenbereich und dem Außenbereich des Gärbehälters (1, 50, 60, 70, 100, 100a) zu verschließen oder zu ermöglichen, und/oder
- die Öffnung(en) zum Einführen und/oder Ausbringen (20) von Luft mit einem Verschlussmittel (20a), vorzugsweise einem Ventil versehen ist (sind), das imstande ist, den Durchgang der Luft (38) zwischen dem Innenbereich und dem Außenbereich des Gärbehälters (1, 50, 60, 70, 100, 100a) zu verschließen oder zu ermöglichen.

10. System nach einem der vorstehenden Ansprüche, **dadurch gekennzeichnet, dass** bestimmte der anaeroben Gärbehälter für feste Biomasse (1, 50, 60, 70, 100, 100a) eine Flüssigkeitsdichtung beinhalten, die um das Dach des Bottichs herum positioniert ist, die imstande ist, den Austritt des in dem Gärbehälter (1, 50, 60, 70, 100, 100a) enthaltenen Biogases (29) zu verhindern, wenn das Dach geschlossen ist, und imstande ist, die Eintritte von Luft (38) in den Gärbehälter zu verhindern, wenn das Dach (11, 10') geschlossen ist.

11. System zur Herstellung von Biogas nach einem der vorstehenden Ansprüche, **dadurch gekennzeichnet, dass** bestimmte Werke, aus denen sich die Zentraleinheit zum Lagern von flüssigem Gärgut und zur ergänzenden Gärung (25) zusammensetzt, einerseits, und bestimmte Werke, aus denen sich die Zentraleinheit zum Lagern von Biogas (25) zusammensetzt, andererseits, gemeinsam sind.

12. System zur Herstellung von Biogas nach einem der vorstehenden Ansprüche, **dadurch gekennzeichnet, dass** die Zentraleinheit zum Lagern von flüssigem Gärgut und zur ergänzenden Gärung (25) einerseits, und die Zentraleinheit zum Lagern von Biogas (25) andererseits, gemeinsam sind, und eine einzige und gleiche Zentraleinheit zum Lagern von flüssigem Gärgut und Biogas und zur ergänzenden Gärung bilden.

13. System nach einem der vorstehenden Ansprüche, **dadurch gekennzeichnet, dass** auf bestimmten der Gärbehälter für feste Biomasse (1, 50, 60, 70, 100, 100a) mindestens eine Ausbringungsöffnung (19) für flüssiges Gärgut (27) und eine Ausbringungsöffnung (19) für Biogas (29) gemeinsam sind, und mit mindestens einem Ausbringungskreis (32, 320, 420, 520, 620) für ein Gemisch aus flüssigem Gärgut (27) und Biogas (29) verbunden sind, wobei der Kreis (32, 320, 420, 520, 620) mit einem Trennmittel (32') verbunden ist, das imstande ist, das Biogas (29) und das flüssige Gärgut (27) zu trennen.

14. System nach einem der vorstehenden Ansprüche, **dadurch gekennzeichnet, dass** mindestens ein abnehmbarer Gärbehälter (1, 100, 101, 102, 103, 60, 61, 62, 63, 64, 65, 70, 71, 72, 73, 74, 75) wärmeisoliert ist.

15. Verfahren zur Herstellung von Biogas, mindestens einen der folgenden aufeinanderfolgenden Schritte umfassend:
- einen Schritt zum Beladen mit fester Biomasse (24) eines abnehmbaren Gärbehälters (1, 100, 101, 102, 103, 60, 61, 62, 63, 64, 65, 70, 71, 72, 73, 74, 75) nach einem der vorstehenden Ansprüche, der an einem anderen Beladeort als dem Aufstellungsort des Systems zur Herstellung von Biogas nach einem der vorstehenden Ansprüche umgesetzt wird, manuell oder durch mindestens ein Belademittel durch die Einlasszone (10) hindurch,
- einen Schritt zum Transportieren des abnehmbaren Gärbehälters (1, 100, 101, 102, 103, 60, 61, 62, 63, 64, 65, 70, 71, 72, 73, 74, 75), der mit fester Biomasse (24) beladen ist, von dem Beladeort der festen Biomasse (24) bis zum Aufstellungsort des Systems zur Herstellung von Biogas,
- einen Schritt zum Verbinden des abnehmbaren Gärbehälters (1, 100, 101, 102, 103, 60, 61, 62, 63, 64, 65, 70, 71, 72, 73, 74, 75), der mit fester Biomasse (24) beladen ist, mit mindestens einem Zuführungskreis (31, 310, 410, 510, 610) für flüssiges Gärgut (27) und mit mindestens einem Ausbringungskreis (32, 320, 420, 520, 620, 34a) für flüssiges Gärgut (27) und mit mindestens einem Ausbringungskreis (32, 320, 420, 520, 620, 35a) für Biogas (29),
- einen Schritt zum Zuführen zum abnehmbaren Gärbehälter (1, 100, 101, 102, 103, 60, 61, 62, 63, 64, 65, 70, 71, 72, 73, 74, 75) eines flüssigen Gärguts (27) zum Eintauchen der in dem abnehmbaren Gärbehälter (1, 100, 101, 102, 103, 60, 61, 62, 63, 64, 65, 70, 71, 72, 73, 74, 75) enthaltenen festen Biomasse (24),
- einen Schritt zum Vergären der festen Biomasse (24) in dem abnehmbaren Gärbehälter (1, 100, 101, 102, 103, 60, 61, 62, 63, 64, 65, 70, 71, 72, 73, 74, 75), umfassend die Perkolation des flüssigen Gärguts (27) durch die feste Biomasse (24) hindurch, und die Diffusion des flüssigen Gärguts (27) in der festen Biomasse (24) unter anaeroben Bedingungen zum Generieren und dann Auffangen von Biogas (29), und
- einen Schritt zum aeroben Entleeren des flüssigen Gärguts (27) aus dem abnehmbaren Gärbehälter (1, 100, 101, 102, 103, 60, 61, 62, 63, 64, 65, 70, 71, 72, 73, 74, 75).

16. Verfahren zur Herstellung von Biogas nach Anspruch 15, **dadurch gekennzeichnet, dass** es weiter nach dem Schritt zum aeroben Entleeren des flüssigen Gärguts (27) aus dem abnehmbaren Gärbehälter (1, 100, 101, 102, 103, 60, 61, 62, 63, 64, 65, 70, 71, 72, 73, 74, 75) die folgenden Schritte umfasst:
- einen Schritt zum Trennen des abnehmbaren Gärbehälters (1, 100, 101, 102, 103, 60, 61, 62, 63, 64, 65, 70, 71, 72, 73, 74, 75), der mit restlicher vergorener fester Biomasse beladen ist, von dem Zuführungskreis (31, 310, 410, 510, 610) für flüssiges Gärgut (27) und von dem Ausbringungskreis (32, 320, 420, 520, 620, 34a) für flüssiges Gärgut (27) und dem Ausbringungskreis (32, 320, 420, 520, 620, 35a) für Biogas (29),
- einen Schritt zum Transportieren des abnehmbaren Gärbehälters (1, 100, 101, 102, 103, 60, 61, 62, 63, 64, 65, 70, 71, 72, 73, 74, 75), der mit restlicher vergorener fester Biomasse beladen ist, von dem Aufstellungsort des Systems zur Herstellung von Biogas bis zum Ausbringungsort der vergorenen festen Biomasse,
- einen Schritt zum Ausbringen der restlichen vergorenen festen Biomasse, der vorzugsweise durch Umkippen des abnehmbaren Gärbehälters (1, 100, 101, 102, 103, 60, 61, 62, 63, 64, 65, 70, 71, 72, 73, 74, 75) umgesetzt wird, der für die Ausbringung der restlichen vergorenen festen Biomasse durch eine seitliche Entladezone (16) sorgt, oder durch mindestens ein Greifmittel durch eine Ausbringungszone der restlichen vergorenen festen Biomasse hindurch, auf dem oberen Teil des Bottichs (2), auf dem ein Dach (11) imstande ist, sich zu öffnen und zu schließen.

17. Verfahren zur Herstellung von Biogas nach Anspruch 15, **dadurch gekennzeichnet, dass** es weiter zwischen dem Schritt zum Vergären und dem Schritt zum aeroben Entleeren des flüssigen Gärguts mindestens die folgenden Schritte umfasst:
- einen Schritt zum anaeroben Entleeren des flüssigen Gärguts (27) aus dem abnehmbaren Gärbehälter (1, 100, 101, 102, 103, 60, 61, 62, 63, 64, 65, 70, 71, 72, 73, 74, 75), und
- einen Schritt zum erneuten Füllen des abnehmbaren Gärbehälters (1, 100, 101, 102, 103, 60, 61, 62, 63, 64, 65, 70, 71, 72, 73, 74, 75) mit einem flüssigen Gärgut (27) nach der anaeroben Entleerung.

18. Verfahren zur Herstellung von Biogas nach Anspruch 17, **dadurch gekennzeichnet, dass** es weiter nach dem Schritt des erneuten Füllens des abnehmbaren Gärbehälters (1, 100, 101, 102, 103, 60, 61, 62, 63, 64, 65, 70, 71, 72, 73, 74, 75) mit einem flüssigen Gärgut (27) mindestens einen Vergärungsschritt umfasst, der aus einer Perkolation des flüssigen Gärguts (27) durch die feste Biomasse (24) und einer Diffusion des flüssigen Gärguts in der festen Biomasse besteht, um Biogas zu generieren und dann aufzufangen.

19. Abnehmbarer anaerober Gärbehälter für feste Biomasse (1, 100, 101, 102, 103, 60, 61, 62, 63, 64, 65, 70, 71, 72, 73, 74, 75), Folgendes beinhaltend:
- einen Bottich (2), der imstande ist, feste Biomasse (24) zu enthalten, mindestens eine Einlasszone (10) für die feste Biomasse (24) beinhaltend, die sich im oberen Teil des Bottichs (2) befindet, auf dem ein Dach (11) imstande ist, sich zu öffnen und zu schließen, und weiter mindestens eine Ausbringungszone (10, 16) für die restliche vergorene feste Biomasse beinhaltend, wobei bestimmte der Einlass-(10) und Ausbringungszonen (10, 16) für feste Biomasse gemeinsam sein können, und
- mindestens eine Zuführungsöffnung (17, 170, 270, 370, 470) für flüssiges Gärgut (27), die sich im unteren Teil des anaeroben Gärbehälters (1, 50, 60, 70, 100, 100a) befindet, mindestens ein konfiguriertes Mittel (17b) beinhaltend, um es zu ermöglichen, mit mindestens einem Zuführungskreis (31, 310, 410, 510, 610) für flüssiges Gärgut (27) verbunden zu werden, der imstande ist, die Einführung von flüssigem Gärgut (27) in den Gärbehälter zu ermöglichen, und
- mindestens eine Ausbringungsöffnung (19) für flüssiges Gärgut (27), die sich im oberen Teil des anaeroben Gärbehälters befindet, mindestens ein Mittel (19b) beinhaltend, das konfiguriert ist, um es zu ermöglichen, mit mindestens einem Ausbringungskreis (32, 320, 420, 520, 620) für flüssiges Gärgut verbunden zu werden, der imstande ist, die Ausbringung des flüssigen Gärguts (27) aus dem Gärbehälter zu ermöglichen,
- mindestens eine Ausbringungsöffnung (19) für Biogas (29), die sich im oberen Teil des anaeroben Gärbehälters befindet, mindestens ein konfiguriertes Mittel (19b) beinhaltend, um es zu ermöglichen, mit mindesten einem Ausbringungskreis (32, 320, 420, 520, 620) für Biogas (29) verbunden zu werden, der imstande ist, die Ausbringung von Biogas (29) aus dem Gärbehälter zu ermöglichen, und
- mindestens eine Entleerungsöffnung (18) für flüssiges Gärgut (27), die sich im unteren Teil des anaeroben Gärbehälters befindet, die imstande ist (sind), die Entleerung von flüssigem Gärgut aus dem Gärbehälter für feste Biomasse zu ermöglichen, und
- mindestens eine Einführungs- und/oder Ausbringungsöffnung (20) für Luft (38), die sich im oberen Teil des anaeroben Gärbehälters befindet, die imstande ist (sind), die Einführung und/oder Ausbringung von Luft (38) in dem Gärbehälter für feste Biomasse zu ermöglichen,
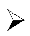 wobei bestimmte Öffnungen (17, 18, 19, 20) unter den Öffnungen, mit Ausnahme der Zuführungsöffnungen (17) und Ausbringungsöffnungen (19) für flüssiges Gärgut, die unterschiedlich sind, gemeinsam sein können;
wobei der Gärbehälter (1, 100, 101, 102, 103, 60, 61, 62, 63, 64, 65, 70, 71, 72, 73, 74, 75) imstande ist:
- mit einem Zuführungsnetz (33) für flüssiges Gärgut, einem Ausbringungsnetz (34) für flüssiges Gärgut (27) und einem Ausbringungsnetz (35) für Biogas verbunden und davon getrennt zu werden.

## Claims

1. System for producing biogas, comprising:
• at least one central unit (25) for storing liquid digestate (27) and for additional digestion, consisting of one or more works, capable of containing mainly liquid digestate (27),
• at least one central unit (25) for storing biogas (29), consisting of one or more works, capable of containing mainly biogas (29),
• a plurality of anaerobic digesters, some of which are digesters of solid biomass, each of said digesters of solid biomass (1, 50, 51, 52, 53, 54, 55, 56, 60, 61, 62, 63, 64, 65, 70, 71, 72, 73, 74, 75, 100, 101, 102, 103, 100a) comprising:
- a tank (2) capable of containing solid biomass (24), including at least one zone (10) for intake of the solid biomass (24), located on the upper part of the tank (2), on which a roof (11, 10') is capable of opening and closing, and further including at least one zone (10, 16) for discharging the residual digested solid biomass, some of said zones for intake (10) and for discharge (10, 16) of solid biomass (23) being capable of being shared, and
- at least one opening (17, 170, 270, 370, 470) for supplying liquid digestate (27), located on the lower part of the anaerobic digester (1, 50, 60, 70, 100, 100a), connected to at least one circuit (31, 310, 410, 510, 610) for supplying liquid digestate (27), capable of allowing the introduction of liquid digestate (27) into said digester of solid biomass (1, 50, 60, 70, 100, 100a), and
- at least one opening (19) for discharging liquid digestate (27), located on the upper part of the anaerobic digester (1, 50, 60, 70, 100, 100a), connected to at least one circuit (32, 320, 420, 520, 620, 34a) for discharging liquid digestate (27), capable of allowing the discharge of liquid digestate (27) from said digester of solid biomass (1, 50, 60, 70, 100, 100a),
- at least one opening (19) for discharging biogas (29), located on the upper part of the anaerobic digester (1, 50, 60, 70, 100, 100a), connected to at least one circuit (32, 320, 420, 520, 620, 35a) for discharging biogas (29), capable of allowing the discharge of biogas (29) from said digester of solid biomass (1, 50, 60, 70, 100, 100a), and
- at least one opening (18) for draining liquid digestate (27), located on the lower part of the anaerobic digester (1, 50, 60, 70, 100, 100a), capable of allowing the draining of liquid digestate (27) from said digester of solid biomass (1, 50, 60, 70, 100, 100a), and
- at least one opening (20) for introducing and/or discharging air (38), located on the upper part of the anaerobic digester (1, 50, 60, 70, 100, 100a), capable of allowing the introduction and/or the discharge of air (38) in said digester of solid biomass (1, 50, 60, 70, 100, 100a),
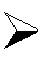 certain openings among said openings (17, 18, 19, 20) being capable of being shared, except for the openings for supplying and for discharging liquid digestate which are distinct, and
• at least one network (33) for supplying liquid digestate (27), including a plurality of circuits (31, 310, 410, 510, 610) for supplying liquid digestate (27), allowing to connect certain openings (17, 170, 270, 370, 470) for supplying liquid digestate (27) of certain digesters of solid biomass (1, 50, 60, 70, 100, 100a) to the central unit for storing liquid digestate and for additional digestion (25), capable of allowing the supply of liquid digestate (27), directly or indirectly, in said digesters (1, 50, 60, 70, 100, 100a) from the central unit (25) for storing liquid digestate and for additional digestion, and
• at least one network (34) for discharging liquid digestate (27), including a plurality of circuits (32, 320, 420, 520, 620, 34a) for discharging liquid digestate (27), allowing to connect certain openings (19) for discharging liquid digestate of certain digesters of solid biomass (1, 50, 60, 70, 100, 100a) to the central unit (25) for storing liquid digestate and for additional digestion, capable of allowing the discharge of liquid digestate (27), directly or indirectly, from said digesters (1, 50, 60, 70, 100, 100a) to the central unit (25) for storing liquid digestate and for additional digestion, and
• at least one network (35) for discharging biogas (29), including a plurality of circuits (32, 320, 420, 520, 620, 35a) for discharging biogas (29), allowing to connect certain openings (19) for discharging biogas of certain digesters of solid biomass (1, 50, 60, 70, 100, 100a) to the central unit (25) for storing biogas, capable of allowing the discharge of biogas (29), directly or indirectly, from said digesters (1, 50, 60, 70, 100, 100a) to the central unit (25) for storing biogas,
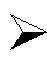 certain parts of said networks (33, 34, 35) being capable of being shared, and
• certain digesters among said digesters of solid biomass (1, 50, 51, 52, 53, 54, 55, 56, 60, 61, 62, 63, 64, 65, 70, 71, 72, 73, 74, 75, 100, 101, 102, 103, 100a) are removable digesters (1, 100, 101, 102, 103, 60, 61, 62, 63, 64, 65, 70, 71, 72, 73, 74, 75) and include means (17b, 18b, 19b) configured to allow, on the one hand, the connection of each of said removable digesters to at least one circuit (31) for supplying liquid digestate and to at least one circuit (32) for discharging liquid digestate (27) and to at least one circuit (32) for discharging biogas (29) and, on the other hand, the disconnection of each of said digesters from said circuits, and
• at least one means (31b, 310b, 410b, 510b, 610b) for circulating liquid digestate (27), preferably a pump, located on the network (31) for supplying liquid digestate (27) and/or on the network (34) for discharging liquid digestate (27), capable of allowing the circulation of the liquid digestate (27) through certain digesters of solid biomass, and
• at least one device (40) for heating liquid digestate (27) .

2. System for producing biogas for producing biogas according to claim 1, **characterised in that** there is a gripping means above some of said digesters of solid biomass (100a), capable of allowing the supply of the solid biomass (24).

3. System for producing biogas according to claim 2, **characterised in that** the gripping means is a grapple including a plurality of hooks or a grab including buckets.

4. System according to any one of the preceding claims, **characterised in that**, in some of said digesters of solid biomass (1, 50, 60, 70, 100, 100a), at least one of the openings (19) for discharging the liquid digestate (27) comprises at least one perforated separation element (21) for discharging the liquid digestate (27), located upstream of said opening (19) for discharging the liquid digestate (27), capable of separating, on the one hand, the liquid digestate (27) discharged and, on the other hand, the solid biomass (24).

5. System for producing biogas according to any one of the preceding claims, **characterised in that**, in some of said digesters of solid biomass (1, 50, 60, 70, 100, 100a), at least one of the openings (18) for draining the liquid digestate (27) comprises at least one perforated separation element (22) for draining the liquid digestate (27), located upstream of said opening (18) for draining the liquid digestate, capable of separating, on the one hand, the liquid digestate (5) drained and, on the other hand, the solid biomass (24).

6. System for producing biogas according to claim 4 or 5, **characterised in that** certain perforated separation elements (21, 22) are perforated plates.

7. System for producing biogas according to claim 5, **characterised in that** the perforated separation element (21) for draining the liquid digestate (27) is a perforated floor.

8. System for producing biogas according to any one of the preceding claims, **characterised in that** at least one of the removable digesters (1, 100, 101, 102, 103, 60, 61, 62, 63, 64, 65, 70, 71, 72, 73, 74, 75) includes, at at least one of its lateral parts (8), at least one hatch (16) for unloading the residual digested solid biomass, which hatch (16) is capable of being opened and closed in a sealed manner.

9. System for producing biogas according to any one of the preceding claims, **characterised in that** on some of said digesters of solid biomass (1, 50, 60, 70, 100, 100a):
- the opening(s) (17, 170, 270, 370, 470) for supplying liquid digestate (27) is/are provided with an obstruction means (17a), preferably a valve, capable of sealing or allowing the passage of the liquid digestate between the outside and the inside of the digester, and/or
- the opening(s) (19) for discharging liquid digestate (27) is/are provided with an obstruction means (19a), preferably a valve, capable of sealing or allowing the passage of the liquid digestate (27) between the inside and the outside of the digester (1, 50, 60, 70, 100, 100a), and/or
- the opening(s) (19) for discharging biogas (29) is/are provided with an obstruction means (19a), preferably a valve, capable of sealing or allowing the passage of the biogas between the inside and the outside of the digester, and/or
- the opening(s) (18) for discharging liquid digestate is/are provided with an obstruction means (18a), preferably a valve, capable of sealing or allowing the passage of the liquid digestate (27) between the inside and the outside of the digester (1, 50, 60, 70, 100, 100a), and/or
- the opening(s) (20) for introducing and/or discharging air is/are provided with an obstruction means (20a), preferably a valve, capable of sealing or allowing the passage of the air (38) between the inside and the outside of the digester (1, 50, 60, 70, 100, 100a).

10. System for producing biogas according to any one of the preceding claims, **characterised in that** some of said anaerobic digesters of solid biomass (1, 50, 60, 70, 100, 100a) include a liquid joint positioned around the roof of the tank, capable of preventing the outlet of the biogas (29) contained in said digester (1, 50, 60, 70, 100, 100a) when the roof is closed, and capable of preventing the inlets of air (38) into said digester when the roof (11, 10') is closed.

11. System for producing biogas according to any one of the preceding claims, **characterised in that** certain works forming the central unit (25) for storing liquid digestate and for additional digestion, on the one hand, and certain works forming the central unit (25) for storing biogas, on the other hand, are shared.

12. System for producing biogas according to any one of the preceding claims, **characterised in that** the central unit (25) for storing liquid digestate and for additional digestion, on the one hand, and the central unit (25) for storing biogas, on the other hand, are shared and form the same single central unit for storing liquid digestate and biogas and for additional digestion.

13. System for producing biogas according to any one of the preceding claims, **characterised in that**, on some of the digesters of solid biomass (1, 50, 60, 70, 100, 100a), at least an opening (19) for discharging liquid digestate (27) and an opening (19) for discharging biogas (29) are shared and are connected to at least one circuit (32, 320, 420, 520, 620) for discharging a mixture of liquid digestate (27) and of biogas (29), which circuit (32, 320, 420, 520, 620) is connected to a separation means (32') capable of separating the biogas (29) and the liquid digestate (27) .

14. System for producing biogas according to any one of the preceding claims, **characterised in that** at least one removable digester (1, 100, 101, 102, 103, 60, 61, 62, 63, 64, 65, 70, 71, 72, 73, 74, 75) is thermally insulated.

15. Method for producing biogas comprising at least the following successive steps:
- a step of loading at least one removable digester (1, 100, 101, 102, 103, 60, 61, 62, 63, 64, 65, 70, 71, 72, 73, 74, 75), as defined according to any one of the preceding claims, with solid biomass (24), implemented in a loading location other than the location of installation of the system for producing biogas, as defined according to any one of the preceding claims, manually or by at least one loading means, through the intake zone (10),
- a step of transporting said removable digester (1, 100, 101, 102, 103, 60, 61, 62, 63, 64, 65, 70, 71, 72, 73, 74, 75) loaded with solid biomass (24), from the location of loading of the solid biomass (24) to the location of installation of said system for producing biogas,
- a step of connecting said removable digester (1, 100, 101, 102, 103, 60, 61, 62, 63, 64, 65, 70, 71, 72, 73, 74, 75) loaded with solid biomass (24) to at least one circuit (31, 310, 410, 510, 610) for supplying liquid digestate (27) and to at least one circuit (32, 320, 420, 520, 620, 34a) for discharging liquid digestate (27) and to at least one circuit (32, 320, 420, 520, 620, 35a) for discharging biogas (29),
- a step of supplying said removable digester (1, 100, 101, 102, 103, 60, 61, 62, 63, 64, 65, 70, 71, 72, 73, 74, 75) with liquid digestate (27) to submerge the solid biomass (24) contained in said removable digester (1, 100, 101, 102, 103, 60, 61, 62, 63, 64, 65, 70, 71, 72, 73, 74, 75),
- a step of digesting the solid biomass (24) in said removable digester (1, 100, 101, 102, 103, 60, 61, 62, 63, 64, 65, 70, 71, 72, 73, 74, 75), comprising the percolation of the liquid digestate (27) through the solid biomass (24) and the diffusion of the liquid digestate (27) in the solid biomass (24) in anaerobic conditions to generate then recover biogas (29), and
- a step of aerobic draining of the liquid digestate (27) from the removable digester (1, 100, 101, 102, 103, 60, 61, 62, 63, 64, 65, 70, 71, 72, 73, 74, 75).

16. Method for producing biogas according to claim 15, **characterised in that** it further comprises, after the step of aerobic draining of the liquid digestate (27) from the removable digester (1, 100, 101, 102, 103, 60, 61, 62, 63, 64, 65, 70, 71, 72, 73, 74, 75), the following steps:
- a step of disconnecting said removable digester (1, 100, 101, 102, 103, 60, 61, 62, 63, 64, 65, 70, 71, 72, 73, 74, 75), loaded with residual digested solid biomass, from the circuit (31, 310, 410, 510, 610) for supplying liquid digestate (27) and from the circuit (32, 320, 420, 520, 620, 34a) for discharging liquid digestate (27) and from the circuit (32, 320, 420, 520, 620, 35a) for discharging biogas (29),
- a step of transporting said removable digester (1, 100, 101, 102, 103, 60, 61, 62, 63, 64, 65, 70, 71, 72, 73, 74, 75) loaded with residual digested solid biomass, from the location of installation of the system for producing biogas to a location for unloading the digested solid biomass,
- a step of discharging the residual digested solid biomass, implemented, preferably, by turning over the removable digester (1, 100, 101, 102, 103, 60, 61, 62, 63, 64, 65, 70, 71, 72, 73, 74, 75) causing the discharge of the residual digested solid biomass through a lateral unloading zone (16), or by at least one gripping means, through a zone for discharging the residual digested solid biomass located on the upper part of the tank (2), on which a roof (11) is capable of opening and closing.

17. Method for producing biogas according to claim 15, **characterised in that** it further comprises between the step of digestion and the step of aerobic draining of the liquid digestate at least the following steps:
- a step of anaerobic draining of the liquid digestate (27) from the removable digester (1, 100, 101, 102, 103, 60, 61, 62, 63, 64, 65, 70, 71, 72, 73, 74, 75), and
- a step of refilling the removable digester (1, 100, 101, 102, 103, 60, 61, 62, 63, 64, 65, 70, 71, 72, 73, 74, 75) with a liquid digestate (27) after anaerobic draining.

18. Method for producing biogas according to claim 17, **characterised in that** it further comprises, after the step of refilling the removable digester (1, 100, 101, 102, 103, 60, 61, 62, 63, 64, 65, 70, 71, 72, 73, 74, 75) with a liquid digestate (27), at least one step of digestion, consisting of a percolation of the liquid digestate (27) through the solid biomass (24) and of a diffusion of the liquid digestate in the solid biomass, to generate and then recover biogas.

19. Removable anaerobic digester of solid biomass (1, 100, 101, 102, 103, 60, 61, 62, 63, 64, 65, 70, 71, 72, 73, 74, 75), including:
- a tank (2) capable of containing solid biomass (24), including at least one zone (10) for intake of the solid biomass (24), located on the upper part of the tank (2), on which a roof (11) is capable of opening and closing, and further including at least one zone (10, 16) for discharging the residual digested solid biomass, some of said zones for intake (10) and for discharge (10, 16) of solid biomass being capable of being shared, and
- at least one opening (17, 170, 270, 370, 470) for supplying liquid digestate (27), located on the lower part of the anaerobic digester (1, 50, 60, 70, 100, 100a) including at least one means configured (17b) to allow to be connected to at least one circuit (31, 310, 410, 510, 610) for supplying liquid digestate (27), capable of allowing the introduction of liquid digestate (27) into the digester,
- at least one opening (19) for discharging liquid digestate (27), located on the upper part of the anaerobic digester, including at least one means (19b) configured to allow to be connected to at least one circuit (32, 320, 420, 520, 620) for discharging liquid digestate, capable of allowing the discharge of liquid digestate (27) from said digester,
- at least one opening (19) for discharging biogas (29), located on the upper part of the anaerobic digester, including at least one means configured (19b) to allow to be connected to at least one circuit (32, 320, 420, 520, 620) for discharging biogas (29), capable of allowing the discharge of biogas (29) from said digester, and
- at least one opening (18) for draining liquid digestate (27), located on the lower part of the anaerobic digester, capable of allowing the draining of liquid digestate from said digester of solid biomass, and
- at least one opening (20) for introducing and/or discharging air (38), located on the upper part of the anaerobic digester, capable of allowing the introduction and/or the discharge of air (38) in said digester of solid biomass,
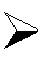 certain openings (17, 18, 19, 20) among said openings being capable of being shared, except for the openings for supplying (17) and discharging (19) liquid digestate which are distinct;
said digester (1, 100, 101, 102, 103, 60, 61, 62, 63, 64, 65, 70, 71, 72, 73, 74, 75) being capable:
- of being connected and disconnected from a network (33) for supplying liquid digestate, from a network (34) for discharging liquid digestate (27) and from a network (35) for discharging biogas.
